# EUROPEAN PATENT SPECIFICATION

(11) **EP 0 443 568 B1**
(45) Date of publication and mention of the grant of the patent: **12.06.1996**
(21) Application number: 91102513.8
(22) Date of filing: 21.02.1991
(51) Int. Cl.: C07D 495/04, A61K 31/435

(54) **Fused thiophene derivatives, their production and use**
Annelierte Thiophen-Derivate, ihre Herstellung und Verwendung
Dérivés fusés de thiophène, leur préparation et leur application

(30) Priority: 22.02.1990 JP 42125/90; 17.01.1991 JP 3958/91
(43) Date of publication of application: 28.08.1991
(73) Proprietor: Takeda Chemical Industries, Ltd., Chuo-ku, Osaka (JP)
(72) Inventor: Morimoto, Akira, Ikeda, Osaka 563 (JP); Nishikawa, Kohei, Nishikyo-ku, Kyoto 610-11 (JP); Naka, Takehiko, Higashinada-ku, Kobe, Hyogo 658 (JP)
(74) Representative: Keller, Günter, Dr.

(56) References cited:
- EP-A- 0 323 841
- EP-A- 0 400 835
- US-A- 4 670 560
- US-A- 4 835 157
- US-A- 4 880 804
- JOURNAL OF MEDICINAL CHEMISTRY, vol. 31, 1988, pages 1786 - 1793; R.K. RUSSELL et al.: "Thiophene Systems. 9. Thienopyrimidinedione Derivatives as Potential Antihypertensive Agents."
- CHEMICAL ABSTRACTS, vol. 99, no. 3, 18 July 1983 Columbus, Ohio, USA R. BOEHM et al.: "Thieno compounds. Part 1. Phase transfer-catalyzed alkylation of thieno[2,3-d]pyrimidin-4[3H]-ones or -2,4-diones" page 616;
- HYPERTENSION, vol. 13, 1989, Dallas, Tex., US, pages 489 - 497; P.C. WONG et al.: "Nonpeptide Angiotensin II Receptor Antagonists. IV. EXP6155 and EXP6803"
- JOURNAL OF PHARMACOLOGY AND EXPERIMENTAL THERAPEUTICS, vol. 247, no. 1, October 1988, pages 1 - 7; P.C. WONG et al.: "Nonpeptide Angiotensin II Receptor Antagonists. I. Pharmacological Characterization of 2-n-Butyl-4-chloro-1-[2- chlorobenzyl]..."

## Description

### FIELD OF THE INVENTION

The present invention relates to novel fused thiophene derivatives having potent pharmacological activity and intermediates for the preparation thereof. More particularly, the present invention relates to compounds having potent angiotensin II antagonist activity and antihypertensive activity, which are useful as therapeutic agents for treating circulatory diseases such as hypertensive diseases, heart diseases, strokes, etc.

### BACKGROUND OF THE INVENTION

The renin-angiotensin system is involved in the homeostatic function to control systemic blood pressure, the volume of body fluid, balance among the electrolytes, etc., associated with the aldosterone system. Development of angiotensin II converting enzyme inhibitors (ACE inhibitor) (this converting enzyme produces angiotensin II which possesses strong vasoconstrictive activity) has clarified the relation between the renin-angiotensin system and hypertension. Since angiotensin II elevates blood pressure via the angiotensin II receptors on cell membranes, angiotensin II antagonists as well as the ACE inhibitor would be useful in treating hypertension.

It has been reported that various angiotensin II analogues such as saralasin, [Sar¹,Ile⁸]A II, and the like, possess potent angiotensin II antagonist activity.

It has, however, been reported that, when peptide antagonists are administered parenterally, their actions are not prolonged and, when administered orally, they are ineffective (M. A. Ondetti and D. W. Cushman, Annual Reports in Medicinal Chemistry, 13, 82-91 (1978)).

Non-peptide angiotensin II antagonists are disclosed in Japanese Patent Laid Open No. 71073/1981; No. 71074/1981; No. 92270/1982; No. 157768/1983; No. 240683/1987; No. 23868/1988; and No. 117876/1989, and European Patent Laid Open No. 0323841, etc.

Imidazole derivatives having angiotensin II antagonist activity are disclosed in A. T. Chiu et al., Eur. J. Pharm., 157, 13 (1981), P. C. Wong et al., J. Pharmcol. Exp. Ther., 247, 1 (1988), P. C. Wong et al. ,Hypertension, 13, 489 (1989), etc.

US 4,670,560 discloses thienopyrimidine-2,4-dione derivatives as vasodilating agents and anti-hypertensive agents. US 4,835,157 discloses thienopyrimidine-2,4-dione piperidine derivatives as selective serotonin antagonists and alpha adrenergic blocking agents. Substituted benzimidazoles as angiotensin II antagonists are disclosed in EP-A-0 400 835.

It has not yet been known that fused thiophene derivatives possess potent angiotensin II antagonist activity.

### SUMMARY OF THE INVENTION

The present inventors made extensive investigations to prepare useful compounds which have angiotensin II antagonist activity. As a result of these researches, the present inventors have succeeded in synthesizing fused thiophene derivatives possessing excellently potent angiotensin II antagonist activity and developed their work to accomplish the present invention.

The present invention provides fused thiophene derivatives having the formula I: 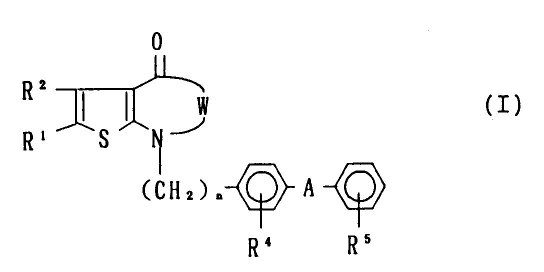
wherein W is 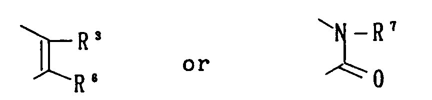
R¹ and R² which may be the same or different, are each independently
(1) hydrogen,
(2) halogen,
(3) cyano,
(4) nitro,
(5) a group having the formula: R⁸CONH- wherein R⁸ is hydrogen or (C₁₋₈)alkyl group which may be substituted with hydroxyl, (C₁₋₄)alkoxy, (C₁₋₄)alkyl, halogen, nitro, amino, methylamino, dimethylamino, phenylamino, benzylamino, morpholino, piperidino, piperazino, N-phenylpiperazino, (C₁₋₄)alkanoyloxy, benzoyloxy, phenyl which may be substituted with halogen, nitro, (C₁₋₄)alkoxy, (C₁₋₄)alkyl at an optional position on the phenyl ring, or naphthyl, or
(6) a hydrocarbon residue selected from the group consisting of (i) (C₁₋₈)alkyl, (ii) (C₂₋₈)alkenyl, (iii) (C₂₋₈)alkynyl, (iv) (C₃₋₈)cycloalkyl, (v) an aromatic hydrocarbon selected from the group consisting of phenyl and naphthyl, wherein said hydrocarbon residue may be substituted with hydroxyl, (C₁₋₄)alkoxy, (C₁₋₄)alkyl, halogen, nitro, amino, N-lower (C₁₋₄)alkylamino, N,N-dilower (C₁₋₄)alkylamino, phenylamino, naphthylamino, benzylamino, naphthylmethylamino, morpholino, piperidino, piperazino, N-phenylpiperazino, N-(m-methoxy)phenylpiperazino, (C₁₋₄)alkanoyloxy, benzoyloxy, phenyl which may be substituted with halogen, nitro, (C₁₋₄)alkoxy, (C₁₋₄)alkyl at an optional position on the phenyl ring, or a group having the formula: -COD' wherein D' is hydroxy, (C₁₋₄)alkoxy, amino, N-lower (C₁₋₄)alkylamino, N,N-dilower (C₁₋₄)alkylamino, phenylamino, naphthylamino, benzylamino, naphthylmethylamino, morpholino, piperidino, piperazino, or N-phenylpiperazino;
R³ is
(1) hydrogen,
(2) (C₁₋₈)alkyl or (C₂₋₈)alkenyl, which may be straight or branched and may be optionally substituted with hydroxyl, amino, N-lower (C₁₋₄)alkyl amino, N,N-dilower (C₁₋₄)alkyl amino, halogen, lower (C₁₋₄)alkoxy, or -COD'' wherein D'' is lower (C₁₋₄)alkoxy, hydroxy, halogen, amino, N-lower (C₁₋₄)alkyl amino, N,N-dilower (C₁₋₄)alkyl amino, phenylamino, naphthylamino, benzylamino, naphthylmethylamino, morpholino, piperidino, piperazino, or N-phenylpiperazino, or
(3) -COD wherein D is hydrogen, C₁₋₄-alkoxy, hydroxy, halogen, amino, N-lower (C₁₋₄)alkyl amino, N,N-dilower (C₁₋₄)alkyl amino, phenylamino, benzylamino, naphthylmethylamino, pyridylamino, pyridylmethylamino, morpholino, piperidino, piperazino, piperidylmethyl, N-(p-fluorophenyl)piperazino, or N-phenylpiperazino, wherein said alkyl, aryl and heteroaryl groups may be optionally substituted with methyl, ethyl, propyl, isopropyl, butyl, isobutyl, sec-butyl, hydroxyl, amino, N-lower (C₁₋₄)alkyl amino, N,N-dilower (C₁₋₄)alkylamino, morpholino, piperidino, piperazino, N-phenylpiperazino, halogen, nitro or lower (C₁₋₄) alkoxy;
R⁴ is hydrogen, halogen or nitro;
R⁵ is carboxyl, lower (C₁₋₄)alkoxycarbonyl, cyano, tetrazolyl, trifluoromethanesulfonic amide, phosphoric acid or sulfonic acid;
R⁶ is hydrogen or (C₁₋₈)alkyl or (C₂₋₈)alkenyl, which may be straight or branched and may be optionally substituted with hydroxyl, amino, N-lower (C₁₋₄)alkyl amino, N,N-dilower (C₁₋₄)alkyl amino, halogen, lower (C₁₋₄)alkoxy, or -COD'' wherein D'' is lower (C₁₋₄)alkoxy, hydroxy, halogen, N-lower (C₁₋₄)alkyl amino, N,N-dilower (C₁₋₄)alkyl amino, phenylamino, naphthylamino, benzylamino, naphthylmethylamino, morpholino, piperidino, piperazino, or N-phenylpiperazino;
R⁷ is a hydrocarbon residue which may be substituted like R¹ and R²; A is a direct bond or a spacer having atomic length of two or less between the phenylene group and the phenyl group selected from the group consisting of (C₁₋₄)alkylene, -C(=O)-, -O-, -S-, -NH-, -C(=O)-NH-, -O-CH₂-, -S-CH₂-, or -CH=CH-; and n is an integer of 1 or 2;
and a pharmaceutically acceptable salt thereof.

These compounds are potent angiotensin II antagonists which are of value in the treatment of circulatory system diseases such as hypertensive diseases, heart diseases, strokes, etc.

Another aspect of the present invention relates to pharmaceutical compositions comprising an effective amount of the fused thiophene derivative having the formula I and a pharmaceutically acceptable carrier useful in treating circulatory system diseases such as hypertensive diseases, heart diseases, strokes, etc., and processes for preparing such compounds and compositions.

Still another aspect of the present invention relates to a use of the fused thiophene derivative having the formula I or a pharmaceutically acceptable salt thereof for the manufacture of a medicament for antagonizing angiotensin II.

### DETAILED DESCRIPTION OF THE INVENTION

The present invention provides fused thiophene derivatives having the formula I and the pharmaceutically acceptable salts thereof, which possess potent angiotensin II antagonist activity and are of value in the treatment of circulatory diseases such as hypertensive diseases, heart diseases, strokes, etc., pharmaceutical compositions comprising an effective amount of the fused thiophene derivative having the formula I and a pharmaceutically acceptable carrier useful in treating said circulatory diseases and processes for preparing such compounds and compositions.

The present invention further provides a method for treating said circulatory system diseases of hosts, which comprises administering an effective amount of the fused thiophene derivative having the formula I or the pharmaceutical composition thereof to said host.

An important group of compounds according to the present invention are the compounds of the formula Ia: 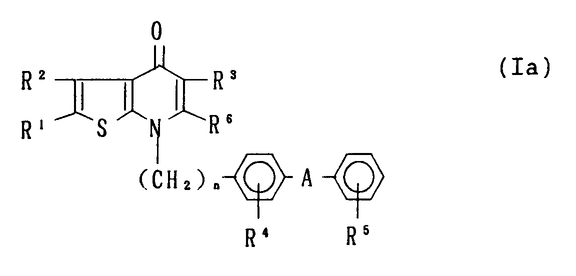
wherein R¹ and R² are as defined above;
R³ is hydrogen, formyl, optionally substituted alkyl or alkenyl as defined in claim 1, or -COD wherein D is alkoxy, hydroxy, halogen, or optionally substituted amino as defined above;
R⁴ is hydrogen, halogen or nitro;
R⁵ is as defined above;
R⁶, A and n are as defined above
and a pharmaceutically acceptable salt thereof.

Another important group of compounds according to the present invention are the compounds of the formula Ib: 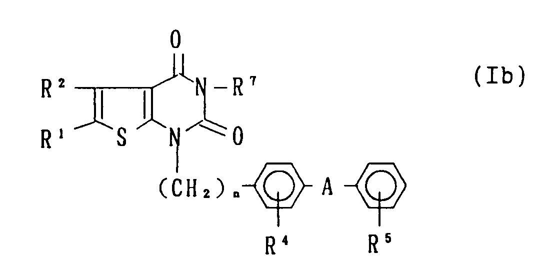
wherein R¹ and R² are as defined above;
R⁴ is hydrogen, halogen or nitro;
R⁵ is as defined above;
R⁷, A and n are as defined above;
and a pharmaceutically acceptable salt thereof.

With regard to the foregoing formula (I), halogen for R¹ and R² include fluorine, chlorine, bromine, and iodine.

R¹ and R² include a group having the formula: R⁸CONH- wherein R⁸ is hydrogen or lower alkyl of 1 to 8 carbon atoms (e.g. methyl, ethyl, propyl, isopropyl, butyl, isobutyl, sec-butyl, t-butyl, pentyl, i-pentyl, hexyl, heptyl, octyl, and the like), which may be substituted with hydroxyl, (C₁₋₄)alkoxy, (C₁₋₄)alkyl, halogen, nitro, amino, methylamino, dimethylamino, phenylamino, benzylamino, morpholino, piperidino, piperazino, N-phenylpiperazino, (C₁₋₄)alkanoyloxy, benzoyloxy, phenyl which may be substituted with halogen, nitro, (C₁₋₄)alkoxy, (C₁₋₄)alkyl at an optional position on the phenyl ring, or naphthyl.

Examples of hydrocarbon residues for R¹, R² and R⁷ include acyclic hydrocarbon residues such as lower alkyl of 1 to 8 carbon atoms (e.g. methyl, ethyl, propyl, isopropyl, butyl, isobutyl, sec-butyl, t-butyl, pentyl, i-pentyl, hexyl, heptyl, octyl, and the like), lower alkenyl of 2 to 8 carbon atoms (e.g. vinyl, allyl, isopropenyl, 2-butenyl, 2-pentenyl, 2-hexenyl, 2-octenyl, and the like), and lower alkynyl of 2 to 8 carbon atoms (e.g. ethynyl, 2-butynyl, 2-pentynyl, 2-octynyl, and the like); cyclic hydrocarbon residues such as alicyclic hydrocarbon residues of 3 to 8 carbon atoms (e.g. cyclopropyl, cyclopentyl, cyclohexyl, 2-cyclohexen-1-yl, cyclooctyl and the like), and aromatic hydrocarbon residues selected from the group consisting of phenyl and naphthyl.

Said hydrocarbon residues for R¹, R² and R⁷ may be optionally substituted with hydroxyl, lower (C₁₋₄) alkoxy (e.g. methoxy, ethoxy, and the like), lower (C₁₋₄) alkyl (e.g. methyl, ethyl, and the like), halogen (e.g. F, Cl, Br and the like), nitro, amino, N-lower (C₁₋₄) alkyl amino (e.g. methylamino, ethylamino, etc.), N,N-dilower (C₁₋₄) alkyl amino (e.g. dimethylamino, diethylamino, etc.), phenylamino, naphthylamino, benzylamino, naphthylmethylamino, morpholino, piperidino, piperazino, N-phenylpiperazino, N-(m-methoxyphenyl)piperazino, lower (C₁₋₄) alkanoyloxy, benzoyloxy, phenyl which may be optionally substituted with halogen, nitro, lower (C₁₋₄) alkoxy or lower (C₁₋₄) alkyl at an optional position on the phenyl ring , or a group having the formula: -COD' wherein D' is hydroxy, lower (C₁₋₄) alkoxy (e.g. methoxy, ethoxy, and the like), amino, N-lower (C₁₋₄) alkyl amino (e.g. methylamino, ethylamino, etc.), N,N-dilower (C₁₋₄) alkyl amino (e.g. dimethylamino, diethylamino, etc.), phenylamino, naphthylamino, benzylamino, naphthylmethylamino, morpholino, piperidino, piperazino or N-phenylpiperazino.

Alkyl or alkenyl groups for R³ and R⁶ are lower alkyl of 1 to 8 carbon atoms and lower alkenyl of 2 to 8 carbon atoms which may be straight or branched. Examples of such alkyl and alkenyl groups for R³ and R⁶ include methyl, ethyl, propyl, isopropyl, butyl, isobutyl, sec-butyl, vinyl, allyl, isopropenyl, 2-butenyl, 2-pentenyl, 2-hexenyl, 2-octenyl, and the like. Said alkyl or alkenyl groups for R³ may be optionally substituted with hydroxyl, amino, N-lower (C₁₋₄) alkyl amino (e.g. methylamino, ethylamino, etc.), N,N-dilower (C₁₋₄) alkyl amino (e.g. dimethylamino, diethylamino, etc.), halogen, lower (C₁₋₄) alkoxy (e.g. methoxyl, ethoxyl, and the like) and -COD'' wherein D'' is lower (C₁₋₄) alkoxy (e.g. methoxy, ethoxy, and the like), hydroxy, halogen, amino, N-lower (C₁₋₄) alkyl amino such as methylamino and ethylamino, N,N-dilower (C₁₋₄) alkyl amino such as dimethylamino and diethylamino, phenylamino, naphthylamino, benzylamino, naphthylmethylamino, morpholino, piperidino, piperazino and N-phenylpiperazino.

Said alkyl or alkenyl groups for R⁶ may be optionally substituted with hydroxyl, amino, N-lower (C₁₋₄) alkyl amino (e.g. methylamino, ethylamino, etc.), N,N-dilower (C₁₋₄) alkyl amino (e.g. dimethylamino, diethylamino, etc.), halogen, lower (C₁₋₄) alkoxy (e.g. methoxyl, ethoxyl, and the like) and -COD'' wherein D'' is lower (C₁₋₄) alkoxy (e.g. methoxy, ethoxy, and the like), hydroxy, halogen, N-lower (C₁₋₄) alkyl amino such as methylamino and ethylamino, N,N-dilower (C₁₋₄) alkyl amino such as dimethylamino and diethylamino, phenylamino, naphthylamino, benzylamino, naphthylmethylamino, morpholino, piperidino, piperazino and N-phenylpiperazino. Where R³ is a group having the formula: -COD, alkoxy groups for D include lower (C₁₋₄) alkoxy (e.g. methoxy, ethoxy, and the like). For D, examples of halogen include Cl, Br and the like. D may further represent amino, N-lower (C₁₋₄) alkyl amino (e.g. methylamino, and the like), N,N-dilower (C₁₋₄) alkyl amino (e.g. dimethylamino, and the like), phenylamino, benzylamino, naphthylmethylamino, pyridylamino, pyridylmethylamino, morpholino, piperidino, piperazino, piperidylmethyl, N-phenylpiperazino or N-(p-fluorophenyl)piperazino, wherein said alkyl, aryl and heteroaryl groups may be optionally substituted with methyl, ethyl, propyl, isopropyl, butyl, isobutyl, sec-butyl, hydroxyl, amino, N-lower (C₁₋₄) alkyl amino (e.g. methylamino, ethylamino, and the like), N,N-dilower (C₁₋₄) alkyl amino (e.g. dimethylamino, and the like), morpholino, piperidino, piperazino, N-phenylpiperazino, halogen, nitro, or lower (C₁₋₄) alkoxy (e.g. methoxyl, ethoxyl).

The compounds wherein D is halogen are useful as synthetic intermediates for the preparation of those wherein D is alkoxy.

R⁴ represents hydrogen, halogen (e.g. chlorine, bromine, and the like) or nitro, which may be in the ortho or meta position to the -A- group.

The residues capable of forming an anion and residues convertible into the anion for R⁵ are carboxyl, lower (C₁₋₄) alkoxycarbonyl, cyano, tetrazolyl, trifluoromethanesulfonic amide (-NHSO₂CF₃), phosphoric acid or sulfonic acid. Such residues include those which are capable of forming anions either chemically or under biological and/or physiological conditions. R⁵ is preferable in the ortho position. The compounds wherein R⁵ is a residue capable of forming an anion or convertible thereinto chemically (e.g. by oxidation, reduction or hydrolysis) (e.g. cyano and the like), are useful as synthetic intermediates.

A shows that the adjacent phenylene group is bonded to the phenyl group directly or through a spacer whose atomic chain is 2 or less.

Such spacers are lower (C₁₋₄) alkylene, -C(=O)-, -O-, -S-, -NH-, -C(=O)-NH-, -O-CH₂-, -S-CH₂-, -CH=CH-.

A preferred embodiment of the invention is a compound of the formula (Ia'): 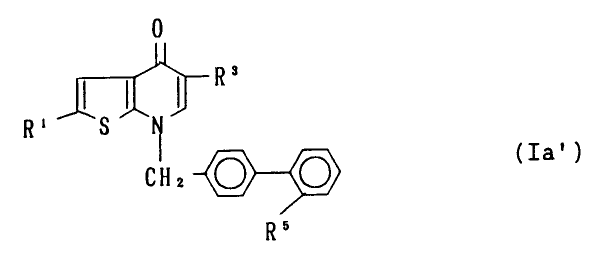
wherein R¹ is (C₁₋₈) alkyl; R³ is hydrogen, (C₁₋₈)alkyl which may be straight or branched and may be optionally substituted with hydroxyl, amino, N-lower (C₁₋₄)alkylamino, N,N-dilower (C₁₋₄)alkylamino, halogen, (C₁₋₄)alkoxy or -COD'' wherein D'' is (C₁₋₄)alkoxy, hydroxy, halogen, amino, N-lower (C₁₋₄)alkyl amino, N,N-dilower (C₁₋₄)alkylamino, phenylamino, naphthylamino, benzylamino, naphthylmethylamino, morpholino, piperidino, piperazino, or N-phenylpiperazino (e.g. lower (C₁₋₄) alkoxylmethyl, and the like) or -COD wherein D is hydrogen, (C₁₋₄)alkoxy, hydroxy, amino, N-lower (C₁₋₄)alkyl amino, N,N-dilower (C₁₋₄)alkylamino, phenylamino, benzylamino, naphthylmethylamino, pyridylamino, pyridylmethylamino, morpholino, piperidino, piperazino, piperidylmethyl, N-(p-fluorophenyl)piperazino, or N-phenylpiperazino; and R⁵ is carboxyl or tetrazolyl (inter alia tetrazolyl); and the pharmaceutically acceptable salts thereof.

A further preferred embodiment of the invention is a compound of the formula (Ib'). 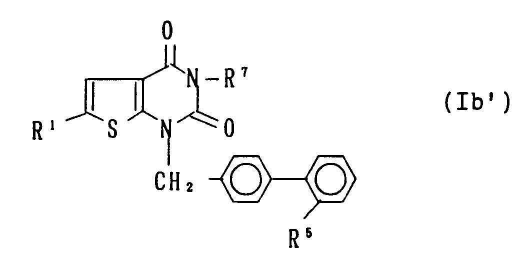
wherein R¹ is (C₁₋₈)alkyl; R⁷ is (C₁₋₈)alkyl which may be optionally substituted with phenyl which may be substituted with halogen, nitro, (C₁₋₄)alkoxy, (C₁₋₄)alkyl at an optional position on the phenyl ring, amino, N-lower (C₁₋₄)alkylamino, N,N-dilower (C₁₋₄)alkylamino, phenylamino, naphthylamino, benzylamino, naphthylmethylamino, morpholino, piperidino, piperazino, N-phenylpiperazino, N-(m-methoxy)phenylpiperazino, or -COD' wherein D' is (C₁₋₄)alkoxy, hydroxy, amino, N-lower (C₁₋₄)alkylamino, N,N-dilower (C₁₋₄)alkylamino, phenylamino, naphthylamino, benzylamino, naphthylmethylamino, morpholino, piperidino, piperazino or N-phenylpiperazino, (C₃₋₈)cycloalkyl, or phenyl or naphthyl which may be substituted with hydroxyl, (C₁₋₄)alkoxy, (C₁₋₄)alkyl, halogen, nitro, amino, N-lower (C₁₋₄)alkylamino, N,N-dilower (C₁₋₄)alkylamino, phenylamino, naphthylamino, benzylamino, naphthylmethylamino, morpholino, piperidino, piperazino, N-phenylpiperazino, N-(m-methoxy)phenylpiperazino, (C₁₋₄)alkanoyloxy, benzoyloxy, phenyl which may be substituted with halogen, nitro, (C₁₋₄)alkoxy, (C₁₋₄)alkyl at an optional position on the phenyl ring, or a group having the formula: -COD' wherein D' is hydroxy, (C₁₋₄)alkoxy, amino, N-lower (C₁₋₄)alkylamino, N,N-dilower (C₁₋₄)alkylamino, phenylamino, naphthylamino, benzylamino, naphthylmethylamino, morpholino, piperidino, piperazino, or N-phenylpiperazino; and R⁵ is carboxyl or tetrazolyl (inter alia tetrazolyl); and the pharmaceutically acceptable salts thereof.

The compounds (I) of the present invention may be prepared by several reaction schemes, as illustrated below for a preferred compound. 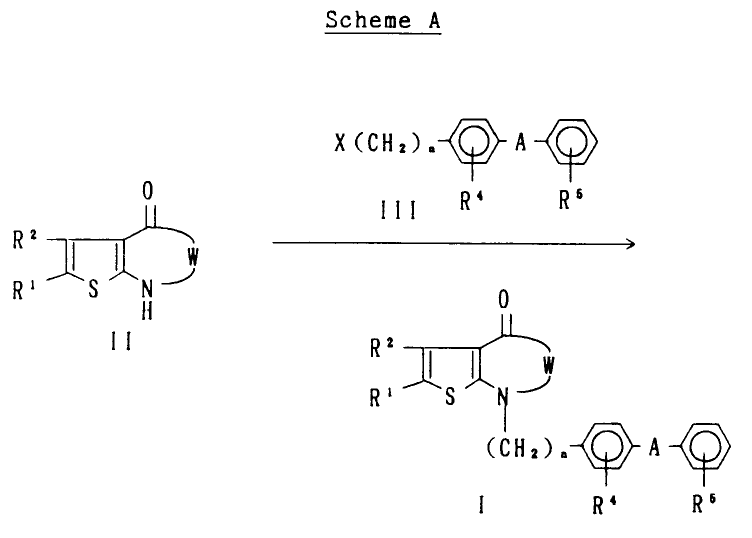
wherein R¹, R², R⁴, R⁵, A, W and n have the above-defined meanings, and X is halogen. 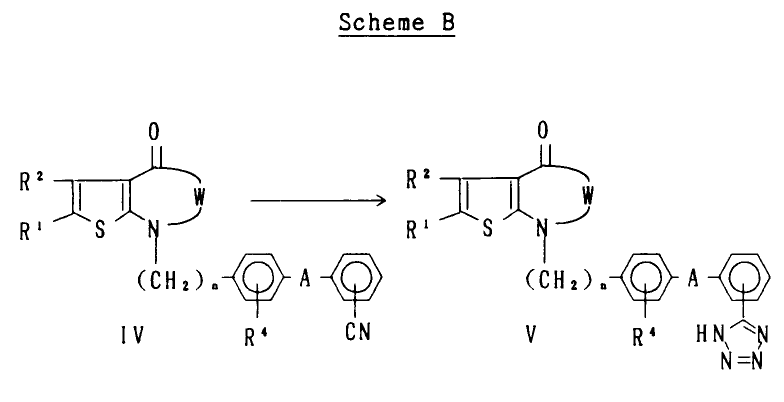
wherein each group is of the same meaning as defined above. 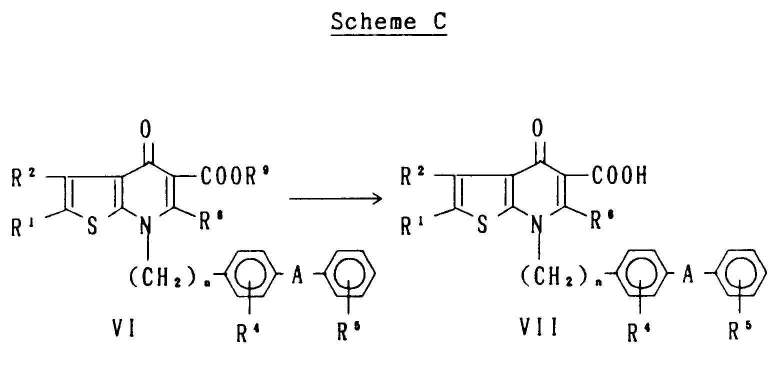
wherein R¹, R², R⁴, R⁵, R⁶, A and n have the above-defined meanings, and R⁹ is lower (C₁₋₄) alkyl. 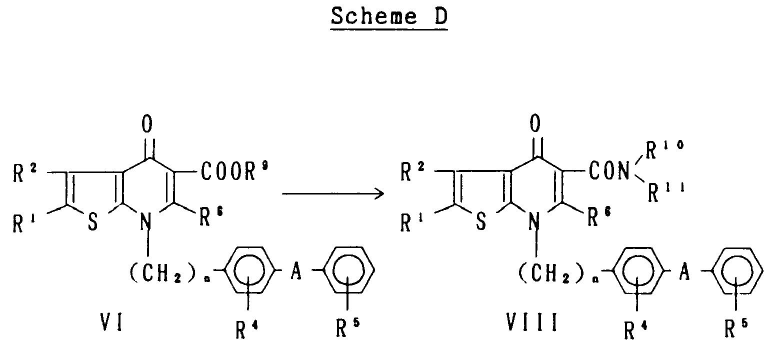
wherein R¹, R², R⁴, R⁵, R⁶, R⁹, A and n have the above-defined meanings, and R¹⁰ and R¹¹ are each independently hydrogen or a hydrocarbon residue. 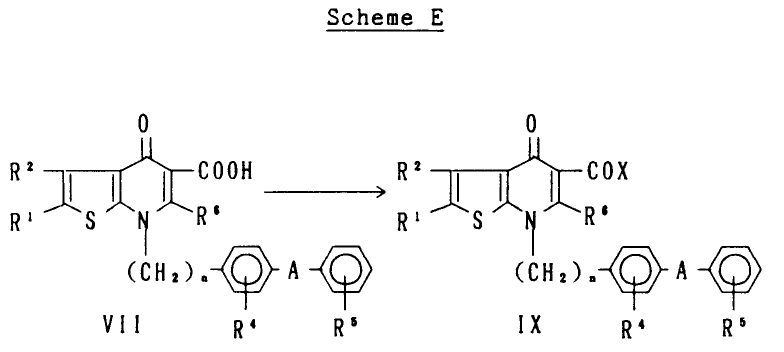
wherein R¹, R², R⁴, R⁵, R⁶, A and n have the above-defined meanings, and X is halogen. 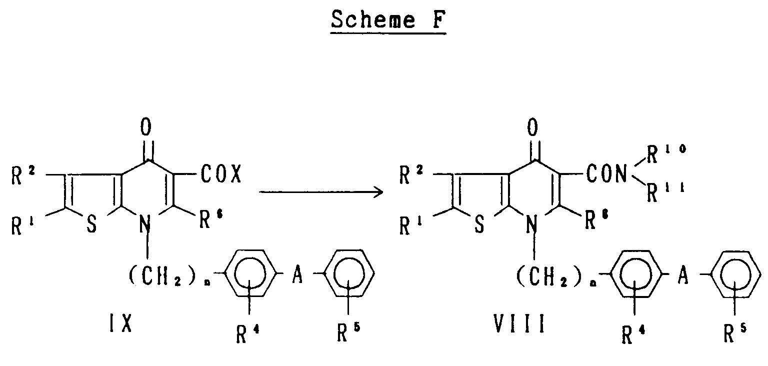
wherein R¹, R², R⁴, R⁵, R⁶, R¹⁰, R¹¹, X, A and n have the above-defined meanings. 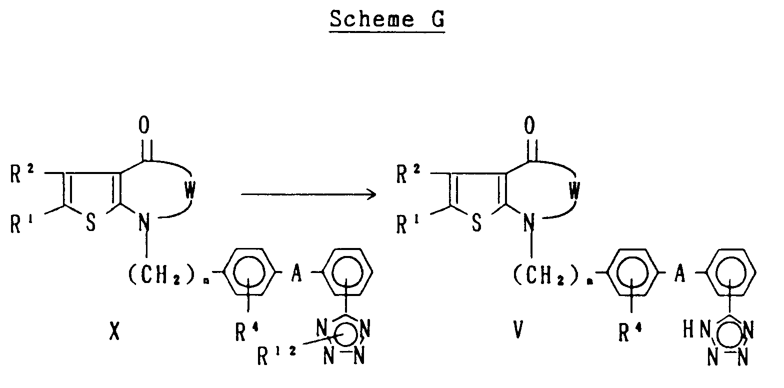
wherein R¹, R², R⁴, A, W and n have the above-defined meanings and R¹² is a protective group.

The reaction as illustrated in Scheme A is an alkylation using an alkylating agent in the presence of a base. One molar portion of the compound (II) is employed with 1 to 3 moles of the base and about 1 to about 3 moles of the alkylating agent. The reaction is conventionally conducted in solvents such as dimethylformamide, dimethylacetamide, dimethylsulfoxide, acetonitrile, acetone, ethylmethylketone, and the like. Examples of such bases include sodium hydride, potassium t-butoxide, cesium carbonate, potassium carbonate, sodium carbonate, and the like. Examples of such alkylating agents include substituted halides (e.g. chlorides, bromides, iodides, and the like), substituted sulfonate esters (e.g. methyl p-toluenesulfonate esters, and the like), etc.

The reaction conditions may vary depending on the combination of the base and the alkylating agent. A temperature in the range of ice-cooling - 100°C is preferred and a reaction period of 1-about 10 hours is preferably employed.

The cyano substituent on the benzene of the compounds (IV) is reacted with various azides to form the tetrazole compounds (V) as illustrated in Scheme B. One molar portion of the compound (IV) is employed with 1-10 moles of the azide. The reaction is conventionally conducted in solvents such as dimethylformamide, dimethylacetamide, toluene, benzene, and the like.

Examples of such azides include trialkyl-tin azide, triphenyl-tin azide, hydrogen azide, and the like. In the case where the organo-tin azide compound is employed, the reaction is carried out in toluene or benzene by heating under reflux for a period of 10 - 30 hours. When the hydrogen azide is used, 5 moles of sodium azide and ammonium chloride per compound (IV) are employed and the reaction is conducted in dimethylformamide at a temperature ranging from 100°C - 130°C for 1-10 days. During this reaction, it is preferable to facilitate working by adding an appropriate amount of sodium azide and ammonium chloride.

The reaction as illustrated in Scheme C is hydrolysis of the ester (VI) into the carboxylic acid (VII) in the presence of an alkali. One molar portion of the compound (VI) is employed with 1 to 3 moles of the alkali. The reaction is conventionally conducted in solvents such as alcohols containing water (e.g. methanol, ethanol, methylcellosolve, and the like). Examples of such alkalis include sodium hydroxide, potassium hydroxide, and the like. The reaction is preferably conducted at a temperature in the range of room temperature - 100°C for 1-10 hours.

The compounds (VI) are reacted with various amines to form the amide compounds (VIII) as illustrated in Scheme D. One molar portion of the compound (VI) is employed with about 2 to 50 moles of the amine. The reaction is conventionally conducted in solvents such as alcohols (e.g. methanol, ethanol, and the like) or without a solvent. The reaction is preferably conducted at a temperature in the range of room temperature - 200°C. Examples of such amines include ammonia, alkylamines (e.g. methylamine, ethylamine, propylamine, dimethylamine, diethylamine, butylamine, hydroxyethylamine, etc.), aralkylamines (e.g. benzylamine, o-methoxybenzylamine, etc.), arylamines (e.g. aniline, etc.), heteroaralkylamines (e.g. 2-, 3- or 4-pyridylmethylamine, etc.), alicyclic amines (e.g. morpholine, piperidine, piperazine, N-phenylpiperazine, 2-piperidylmethylamine, etc.), and the like.

The compounds (VII) are treated with various halogenating agents to form the acid halides (IX) as illustrated in Scheme E. One molar portion of the compound (VII) is employed with about 1 to 5 moles of the halogenating agent. The reaction is conventionally conducted in solvents such as halogenated hydrocarbons (e.g. CHCl₃, CH₂Cl₂, ClCH₂CH₂Cl, and the like), ethers (e.g. tetrahydrofuran, dioxane, and the like) and aromatic hydrocarbons (e.g. benzene, toluene, and the like). Examples of such halogenating agents include oxalyl chloride, thionyl chloride, phosphorous oxychloride, phosphorous trichloride, phosphorous pentachloride, etc. The reaction is preferably conducted at room temperature - 100°C for 1-10 hours.

The acid halides (IX) are reacted with various amines to form the amide compounds (VIII) as illustrated in Scheme F. One molar portion of the compound (IX) is employed with about 2 to 50 moles of the amine. The reaction is conventionally conducted in solvents such as alcohols (e.g. methanol, ethanol, and the like) and ethers (e.g. ethyl ether, tetrahydrofuran, dioxane, and the like). Examples of such amines include ammonia, alkylamines (e.g. methylamine, ethylamine, propylamine, dimethylamine, diethylamine, butylamine, hydroxyethylamine, etc.), aralkylamines (e.g. benzylamine, o-methoxybenzylamine, etc.), arylamines (e.g. aniline, etc.), heteroaralkylamines (e.g. 2-, 3- or 4-pyridylmethylamine, etc.), alicyclic amines (e.g. morpholine, piperidine, piperazine, N-phenylpiperazine, 2-piperidylmethylamine, etc.), and the like.

The protective group (R¹²) on the tetrazole compound (X) leaves to form the tetrazole compound (V) as illustrated in Scheme G. Reaction conditions may vary depending on the protective group (R¹²) used. When R¹² is triphenylmethyl (trityl), 2-tetrahydropyranyl, methoxymethyl, ethoxymethyl, or the like, the leaving of the protective group is conveniently conducted in aqueous alcohols (e.g. methanol, ethanol, etc) containing from about 0.5N to about 2N hydrochloric acid or acetic acid, or in a mixture of trifluoroacetic acid and water (1:2 ∼5) at room temperature for 1-10 hours.

The compounds (I) thus produced via the reaction processes as depicted in Schemes A, B, C, D, E, F and G can be isolated and purified from the reaction mixture according to conventional methods such as, for example, evaporation of solvents, extraction by water or organic solvents, concentration, neutralization, recrystallization, distillation, column chromatography and the like, to obtain a crystalline or oily product.

The compounds (I) of the present invention can be used in the form of salts derived from pharmaceutically or physiologically acceptable acids or bases. These salts include but are not limited to the following: salts with inorganic acids such as hydrochloric acid, sulphuric acid, nitric acid, phosphoric acid and, as the case may be, such organic acids as acetic acid, oxalic acid, succinic acid, and maleic acid. Other salts include salts with alkali metals or alkaline earth metals, such as sodium, potassium, calcium or magnesium or with organic bases.

The starting materials (II) can be easily prepared by or according to the known techniques, for example, as disclosed in:
(1) P. Blaszkiewicz, H. Vorbruggen and H. J. Kesler (Schering AG): DOS 2, 447,477 (15. 4. 76); Chem. Abst., 85, 46627 (1976),
(2) Y. Kuwada, K. Meguro, Y. Sato and T. Fugono (Takeda Chem.): DOS 2, 435,025 (6. 25. 75); Chem. Abst., 82, 156252 (1975), etc.

Among the starting materials (III), the compounds wherein n is 1 (the compounds (IIIa)) is prepared by the known techniques as disclosed in Japanese Patent Laid Open No. 23868/1988; and No. 117876/1989, and European Patent Laid Open No. 0323841.

As illustrated in Scheme H, the compounds (IIIa) can also be easily prepared by halogenomethylation of the compounds (X) commercially available or easily prepared according to methods described in known literatures such as, for example, A. A. Vansheidt et al., Khim. Nauka i Prom., 2, 799 (1957), 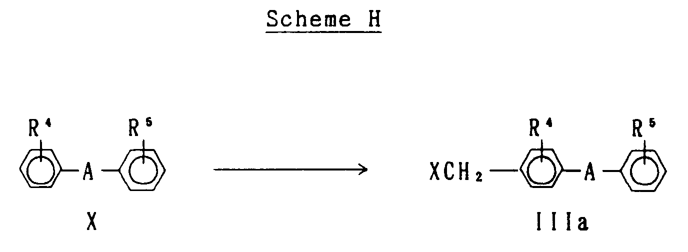
wherein each group has the above-defined meaning.

The compound (III) wherein n is 2 (the compounds (IIIb)) can be prepared from the compounds (IIIa) according to the methods as illustrated in Scheme I. 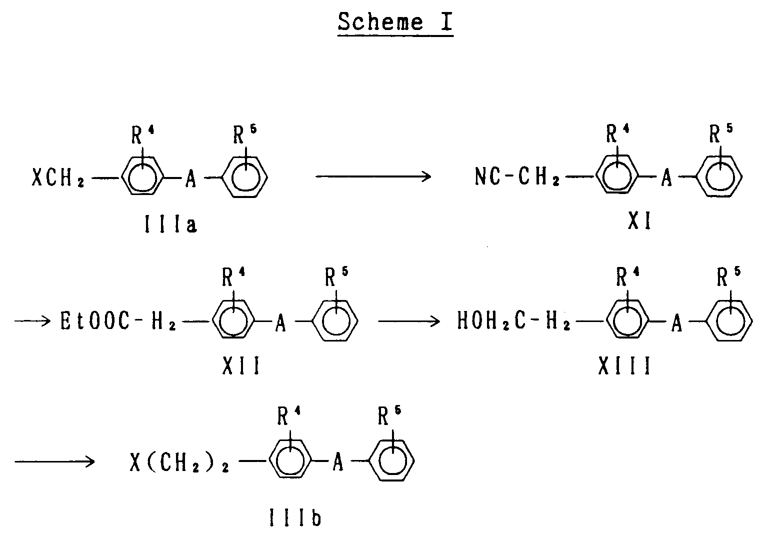
wherein each group has the above-defined meaning.

The compounds (I) and salts thereof according to the present invention strongly inhibit vasoconstriction and hypertension derived by angiotensin II and therefore possess potent anti-hypertensive activity in animals, more specifically mammal animals (e.g. humans, dogs, rabbits, rats, etc.). Further, the compounds (I) and salts thereof according to the present invention are of quite low toxicity and useful in treating not only hypertension but also circulatory system diseases such as heart diseases, strokes and the like.

For therapeutic use, the compounds (I) and salts thereof can be administered as pharmaceutical compositions (e,g, powders, granules, tablets, pills, capsules, injections, solutions and the like) comprising at least one such compound alone or in admixture with pharmaceutically acceptable carriers, excipients and/or diluents. The pharmaceutical compositions can be formulated in accordance with conventional methods.

Specific dose levels for any particular patient will be employed depending upon a variety of factors including the activity of specific compounds employed, the age, body weight, general health, sex, diet, time of administration, route of administration, rate of excretion, drug combination, and the severity of the particular disease undergoing therapy. When used for treating adult essential hypertension, the active ingredient will preferably be administered in an appropriate amount, for example, selected from the range of about 10 mg to 100 mg a day orally and from the range of about 5 mg to 50 mg a day intravenously. The active ingredient will preferably be administered in equal doses two or three times a day.

The foregoing is merely illustrative of the invention and is not intended to limit the invention to the disclosed compounds. Variations and changes which are obvious to one skilled in the art are intended to be within the scope and nature of the invention.

### Example

The invention is further illustrated but in no way limited by the following reference examples, working examples, pharmaceutical examples and experimental examples.

In the specification of the present application, examples of the abbreviations used are given below. Me: Methyl, Et: Ethyl, Pr: Propyl, Bu: Butyl, iBu: Isobutyl, tBu: Tert-butyl, Ph: Phenyl, DMF: Dimethylformamide.

### Reference Example 1

### Isobutyl 2-ethyl-4-hydroxythieno[2,3-b]-pyridine-5-carboxylate

A mixture of 2-ethyl-4-hydroxythieno[2,3-b]pyridine-5-carboxylic acid (200 mg, 0.9 mmol) and boron trifluoride-ethyl ether (47 %, 0.5 ml) in isobutyl alcohol (10ml) was heated under reflux for 5 hours. The reaction mixture was allowed to cool and concentrated to dryness. The resulting residue was purified by flash column chromatography on silica gel. The column was eluted with chloroform/ethyl acetate (3:1) to give 80 mg (31 %) of the title compound as colorless crystals. m.p. 156-158 °C.
IR (KBr)cm⁻¹: 1700, 1595, 1520.
- NMR (CDCl₃) δ :: 1.05(6H, d, J=6.6Hz), 1.40(3H, t, J=7.4Hz), 2.0-2.2(1H, m), 2.94(2H, q, J=7.4, 15.0Hz), 4.20(2H, d, J=6.6Hz), 7.16(1H, s), 8.84(1H, s).

| Elemental Analysis for C₁₄H₁₇NO₃ · H₂O | | | |
|---|---|---|---|
| | C (%) | H (%) | N (%) |
| Calcd: | C, 63.38; | H, 5.70; | N, 15.84 |
| Found: | C, 63.70; | H, 5.44; | N, 15.50. |

### Reference Example 2

### 2-Methoxyethyl 2-ethyl-4-hydroxythieno[2,3-b]pyridine-5-carboxylate

A mixture of 2-ethyl-4-hydroxythieno[2,3-b)pyridine-5-carboxylic acid (380 mg, 1.7 mmol) and DMF (0.13 ml, 1.7 mmol)in benzene (5 ml) was heated to 60 °C and to the reaction mixture was added thionyl chloride (0.15 ml, 2.1 mmol). The mixture was stirred at 60 °C for 3 hours and then allowed to cool with an ice bath. The precipitated product was collected by filtration and washed with benzene. The resulting precipitate (223 mg ) was dissolved in a mixture of 2-methoxyethanol (0.3 ml) and CH₂Cl₂ (5 ml) and then triethyamine (0.7 ml) were added to the solution. The mixture was stirred for 30 minutes and poured into chloroform. The resulting mixture was washed with 1N hydrochloric acid, dried (MgSO₄) and evaporated in vacuo. The resulting residue was purified by flash column chromatography on silica gel. The column was eluted with ethyl acetate/hexane/chloroform (1:1:1) and then chloroform/methanol (9:1) to give 200 mg (41 %) of the title compound as colorless powders.
IR (KBr)cm⁻¹: 1700, 1590, 1530, 1480.
- NMR (CDCl₃) δ :: 1.39(3H, t, J=7.4Hz), 2.94(2H, q, J=7.4, 15.0Hz), 3.45(3H, s), 3.7-3.8(2H, m), 4.5-4.6(2H, m), 7.17(1H, s), 8.87(1H, s).

| Elemental Analysis for C₁₃H₁₅NO₄S | | | |
|---|---|---|---|
| | C (%) | H (%) | N (%) |
| Calcd: | C, 55.50; | H, 5.37; | N, 4.98 |
| Found: | C, 55.47; | H, 5.38; | N, 4.95 |

### Reference Example 3

### Ethyl 2-ethyl-4-hydroxy-3-nitrothieno[2,3-b]pyridine-5-carboxylate

A solution of ethyl 2-ethyl-4-hydroxythieno-[2,3-b]pyridine-5-carboxylate (1.0 g, 4.0 mmol) in conc. sulfuric acid (10 ml) was cooled to -5 °C and a solution of sodium nitrate (370 mg, 4.3 mmol) in conc. sulfuric acid (5 ml) was added dropwise to the chilled solution. The reaction mixture was stirred at -3 °C - -5 °C for 1 hour and poured into ice-water. The precipitated product was collected by filtration and washed with cold water and then ethanol. The resulting precipitate was dissolved in chloroform, washed with a saturated aqueous sodium chloride, dried (MgSO₄) and evaporated in vacuo. The resulting yellow solid was washed with a mixture of ether/hexane, and dried to give 960 mg (81 %) of the title compound. m.p. 194-201 °C (dec.).
IR (KBr)cm⁻¹: 1700, 1600, 1590, 1530.
- NMR (CDCl₃) δ :: 1.42(3H, t, J=7.4Hz), 1.47(3H, t, J=7.2Hz), 3.05(2H, q, J=7.4, 15.0Hz), 4.50(2H, q, J=7.2, 14.4Hz), 8.93(1H, s).

| Elemental Analysis for C₁₂H₁₂N₂O₅S | | | |
|---|---|---|---|
| | C (%) | H (%) | N (%) |
| Calcd: | C, 48.64; | H, 4.08; | N, 9.45 |
| Found: | C, 48.48; | H, 4.01; | N, 9.28 |

### Reference Example 4

### Ethyl 2-ethyl-7-[[2'-(N-trityltetrazol-5-yl)biphenyl-4-yl]methyl]-4-oxo-4,7-dihydrothieno[2,3-b]pyridine-5-carboxylate

To a solution of ethyl 2-ethyl-4-hydroxythieno[2,3-b]pyridine-5-carboxylate (1.00 g, 4 mmol) in 20 ml of DMF) was added sodium hydride (60 % dispersion in oil, 160 mg) and the mixture was stirred for 10 minutes. To the reaction mixture was added 4-[2'-(N-trityltetrazol-5-yl)phenyl]benzyl bromide (2.23 g, 4 mmol) and the mixture was heated at 90 °C for 1 hour with stirring. The reaction mixture was poured into water followed by extraction with ethyl acetate. The organic layer was washed with water, dried (MgSO₄), and evaporated to dryness. The resulting residue was purified by flash column chromatography on silica gel. The column was eluted with ethyl acetate/dichloromethane (1:1) to give 2.24 g (77 %) of the title compound as white crystals. m.p. 195-198 °C.
IR (KBr)cm⁻¹: 1730, 1705, 1620.
- NMR (CDCl₃) δ :: 1.27(3H, t, J=9.6Hz), 1.39(3H, t, J=6.8Hz), 2.72(2H, q, J=8.9, 15.0Hz), 4.38(2H, q, J=7.2, 13.8Hz), 5.02(2H, s), 6.80-8.0(9H, m), 8.29(1H, s).

| Elemental Analysis for C₄₅H₃₇N₅O₃S | | | |
|---|---|---|---|
| | C (%) | H (%) | N (%) |
| Calcd: | C, 74.26; | H, 5.12; | N, 9.62 |
| Found: | C, 74.09; | H, 5.18; | N, 9.50 |

The following compounds (II) can be easily prepared by or according to the known techniques, for example, as disclosed in:
(1) P. Blaszkiewicz, H. Vorbruggen and H. J. Kesler (Schering AG): DOS 2, 447,477 (15. 4. 76); Chem. Abst., 85, 46627 (1976),
(2) Y. Kuwada, K. Meguro, Y. Sato and T. Fugono (Takeda Chem.): DOS 2, 435,025 (6. 25. 75); Chem. Abst., 82, 156252 (1975),
(3) R. K. Russell, J. B. Plress, R. A. Rampulla, J. J. McNally, R. Falotico, J. A. Keiser, D. A. Bright and A. Tobia, J. Med. Chem., 31, 1786 (1988),
(4) M. Suwada, T. Sakamoto, K. Tabata, K. Endo, K. Ito, M. Kobayashi and H. Fuumi, Chem. Pharm. Bull., 37, 2091 (1989),
(5) M. Sugiyama, T. Sakamoto, K. Tabata and H. Fuumi, Chem. Pharm. Bull., 37, 2717 (1989),
(6) G. D. Madding and M. D. Thompson, J. Heterocyclic Chem., 24, 581 (1987),
(7) J. Barker, P. R. Huddleston and D. Holmes, J. Chem. Research (S), 1985, 214,
(8) J. Barker, P. R. Huddleston and D. Holmes, J. Chem. Research (S), 1986, 122,
(9) M. A. Khan and A. E. Guarconi, J. Heterocyclic Chem., 14, 807 (1977),
(10) K. Ogawa, I. Yamawaki, Y. Matsusita, N. Nomura and I. Okazaki, WO 89/02432.
The compounds (III) can alternatively be prepared in the same manner as described in Reference Example 1, 2, or 3.

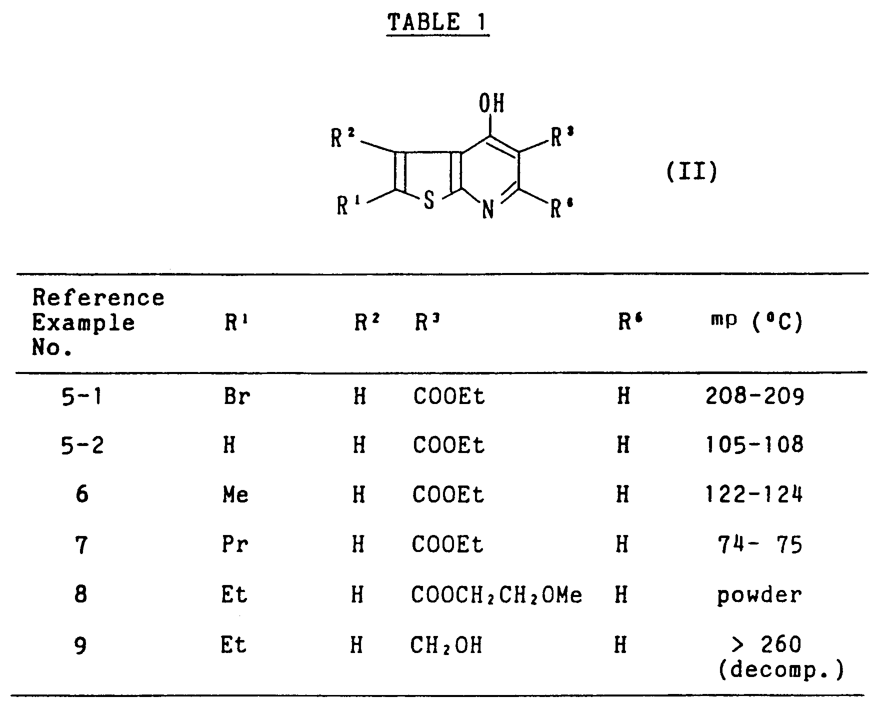

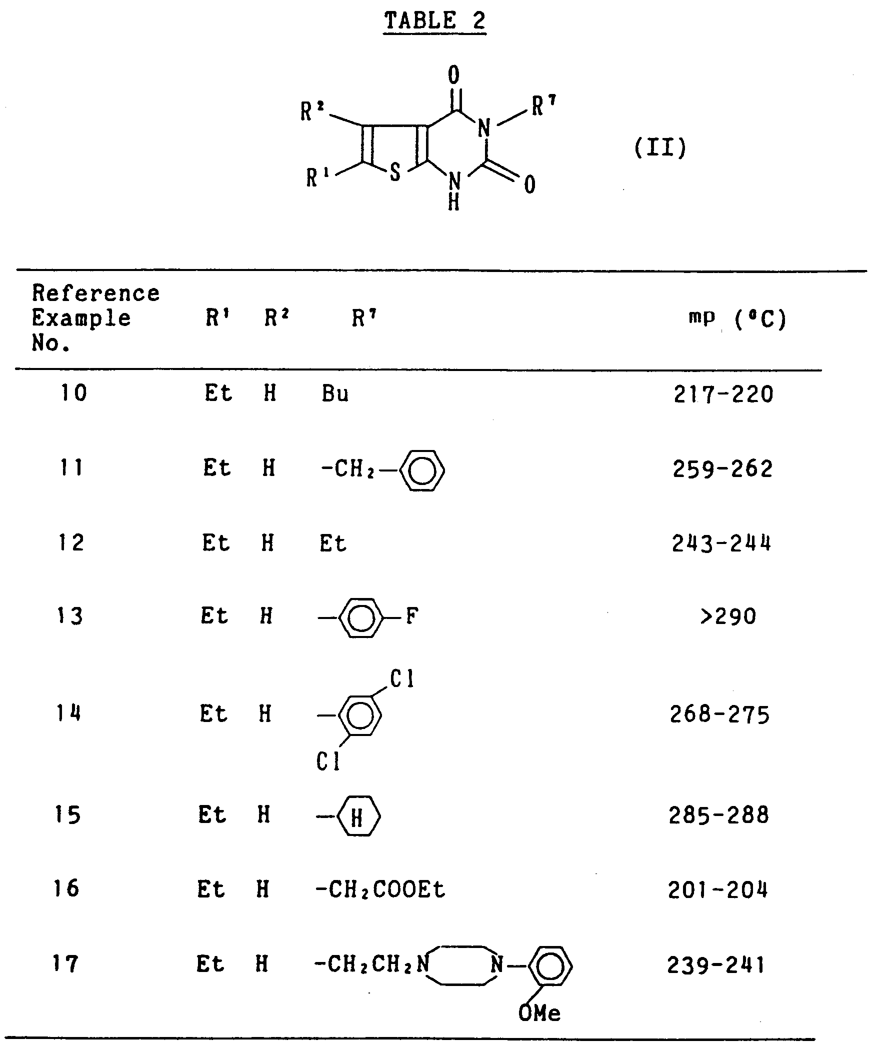

The following compounds (Reference Examples 18-32) were prepared in the same manner as in Reference Example 4.

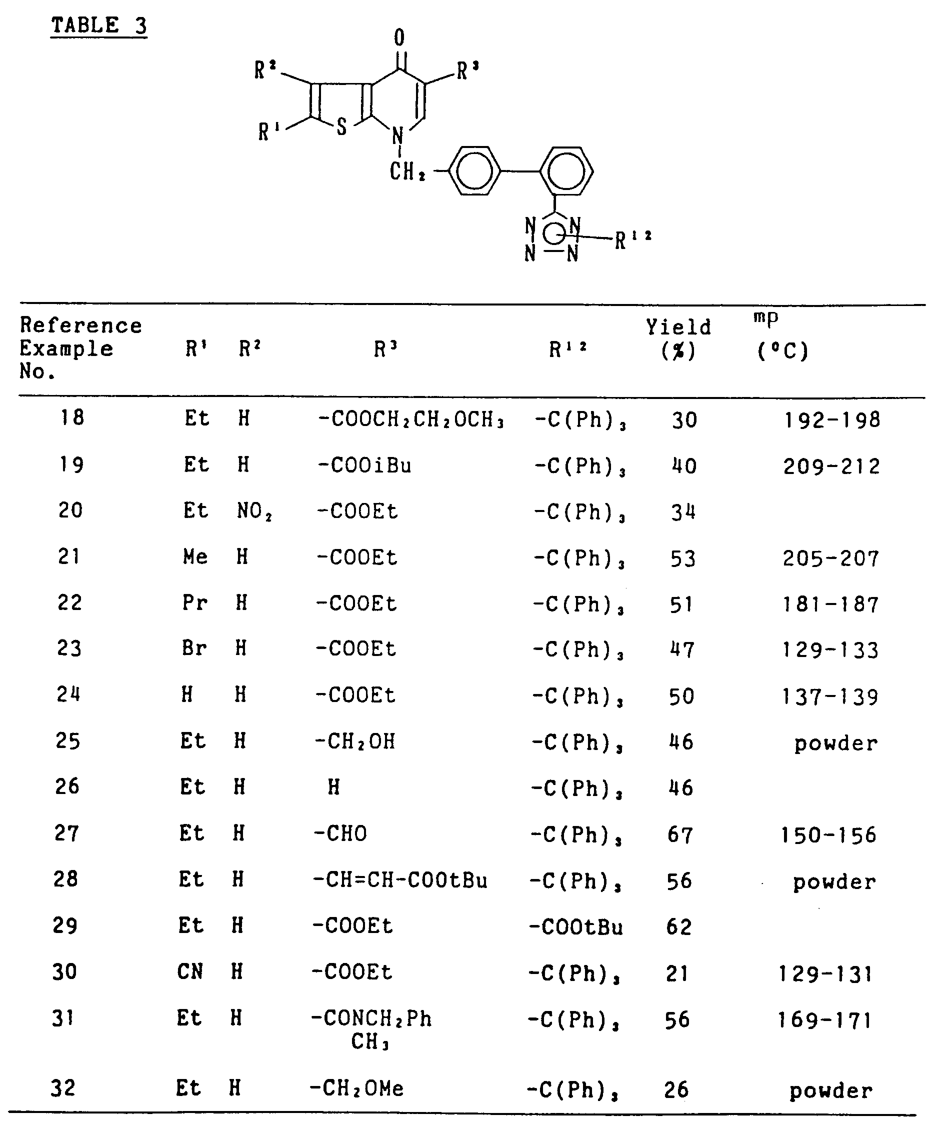

The following compounds (Reference Examples 33-40) were prepared in the same manner as in Reference Example 4.

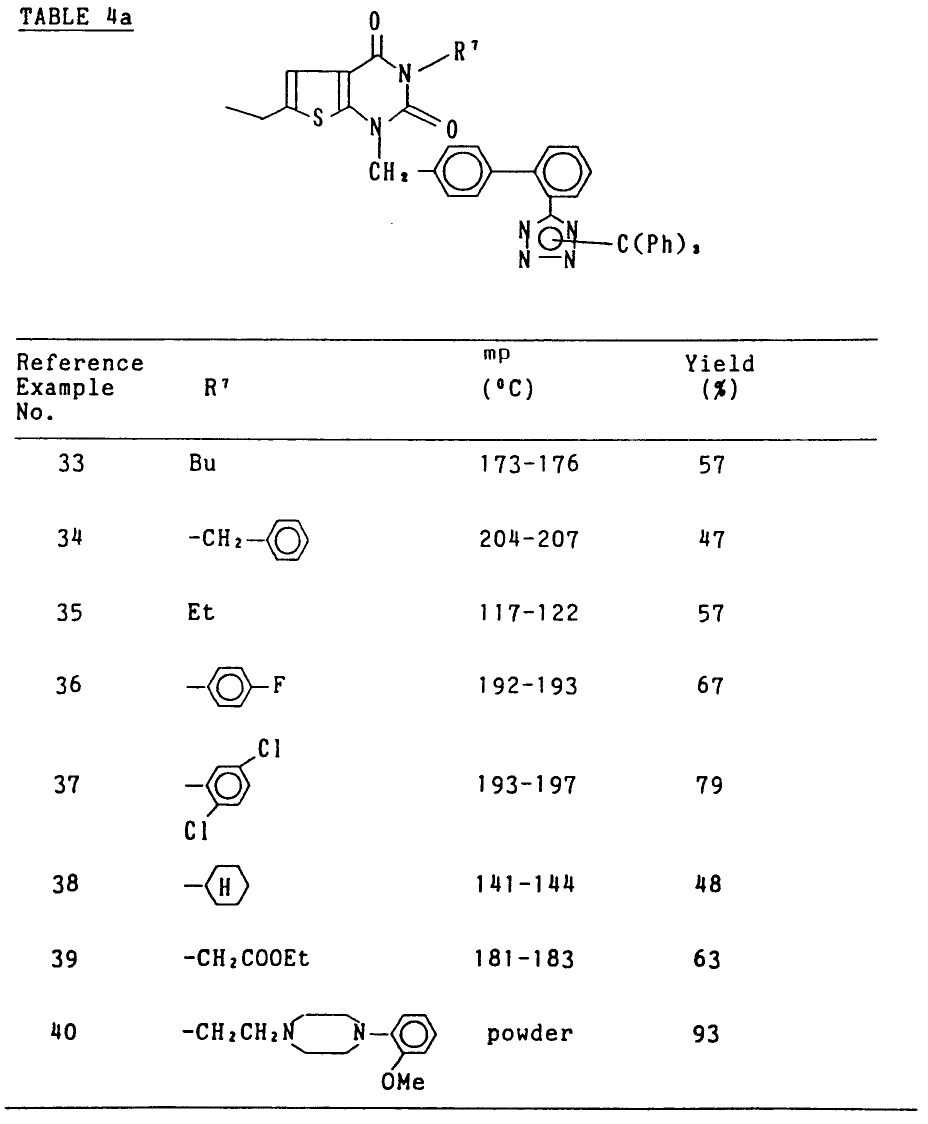

**TABLE 4b**

| Reference Example No. | ¹H-NMR (200MH₂ , CDCl₃)δ |
|---|---|
| 33 | 0.96(3H, t), 1.24(3H, t), 1.3-1.5(2H, m), 1.5-1.7(2H, m), 2.66(2H, q), 4.04(2H, t), 5.01(2H, s), 7.00(1H, s), 6.8-7.5(22H, m), 7.9-8.0(1H, m) |
| 34 | 1.22(3H, t), 2.65(2H, q), 5.00(2H, s), 5.23(2H, s), 7.00(1H, s), 6.8-8.0(28H, m) |
| 35 | 1.24(3H, t), 1.27(3H, t), 2.66(2H, q), 4.11(2H, q), 5.01(2H, s), 6.8-7.5(23H, m), 7.9-8.0(1H, m), |
| 36 | 1.28(3H, t), 2.72(2H, q), 5.03(2H, s), 6.8-8.0(28H, m) |
| 37 | 1.26(3H, t), 2.68(2H, q), 5.06(2H, q), 6.8-8.0(27H, m) |
| 38 | 1.23(3H, t), 1.2-2.6(10H, m), 2.65(2H, q), 4.8-5.0(1H, m), 4.97(2H, s), 6.8-7.9(24H, m) |
| 39 | 1.23(3H, t), 1.29(3H, t), 2.65(2H, q), 4.24(2H, q), 4.79(2H, s), 5.02(2H, s), 6.8-8.0(24H, m) |
| 40 | 1.23(3H, t), 2.64(2H, q), 2.7-3.2(10H, m), 3.85(3H, s), 4.2-4.4(2H, m), 5.03(2H, s), 6.8-7.5(27H, m), 7.9-8.0(1H, m) |

### Working Example 1

### A: Ethyl 2-ethyl-7-[2'-cyanobiphenyl-4-yl)methyl]-4-oxo-4,7-dihydrothieno[2,3-b]pyridine-5-carboxylate

Ethyl 2-ethyl-4-hydroxythieno[2,3-b]-pyridine-5-carboxylate (250 mg, 1 mmol) and 4-(2'-cyanophenyl)benzyl chloride (250 mg, 1.1 mmol) were dissolved in 5 ml of N,N-dimethylformamide (DMF). To the solution was added potassium carbonate (150 mg) and the mixture was stirred at 90 °C for 2 hours. The reaction mixture was poured into water followed by extraction with ethyl acetate. The organic layer was washed with water, dried (MgSO₄), and evaporated to dryness. The resulting residue was purified by flash column chromatography on silica gel. The column was eluted with ethyl acetate/dichloromethane (1:1) to give 254 mg (60 %) of the title compound as crystals.
- NMR (CDCl₃) δ :: 1.30(3H, t, J=6Hz), 1.39(3H, t, J=6Hz), 2.81(2H, q, J=6, 15Hz), 4.40(2H, q, J=6, 13.5Hz), 5.27(2H, s), 7.25-8.0(9H, m), 8.37(1H, s).

### B: Ethyl 2-ethyl-7-[[2'-(1H-tetrazol-5-yl)biphenyl-4-yl]methyl]-4-oxo-4,7-dihydrothieno[2,3-b]pyridine-5-carboxylate and 2-ethyl-7-[[2'-(1H-tetrazol-5-yl)biphenyl-4-yl]methyl]-4-oxo-4,7-dihydrothieno[2,3-b]pyridine-5-carboxylic acid

A solution of the compound (250 mg, 0.59 mmol) prepared in Working Example 1A, sodium azide (390 mg, 5.9 mmol) and ammonium chloride (300 mg, 5.9 mmol) in DMF (15 ml) was stirred at 110 °C for 10 days. After cooling, the reaction mixture was poured into water followed by extraction with ethyl acetate. The organic layer was washed with water, dried (MgSO₄), and evaporated to dryness. The resulting residue was purified by flash column chromatography on silica gel. The column was eluted with chloroform/methanol (9:1) to give 37 mg (13 %) of ethyl 2-ethyl-7-[[2'-(1H-tetrazol-5-yl)biphenyl-4-yl]methyl]-4-oxo-4,7-dihydrothieno[2,3-b]pyridine-5-carboxylate as pale yellow powders.
IR (KBr)cm⁻¹: 1720, 1600, 1550, 1505.
- NMR (CD₃OD) δ :: 1.31(3H, t, J=7.6Hz), 1.36(3H, t, J=7.0Hz), 2.85(2H, q, J=7.6, 15.0Hz), 4.33(2H, q, J=7.0, 14.2Hz), 5.45(2H, s), 7.15(2H, d, J=8.6Hz), 7.21(1H, s), 7.28(2H, d, J=8.6Hz), 7.5-7.7(4H, m), 8.75(1H, s).

The column was further eluted with chloroform/methanol (9:1) to give 7 mg (2.7 %) of 2-ethyl-7-[[2'-(1H-tetrazol-5-yl)biphenyl-4-yl]-methyl]-4-oxo-4,7-dihydrothieno[2,3-b]pyridine-5-carboxylic acid as white solids.
IR (KBr)cm⁻¹: 1650, 1605, 1580, 1540, 1505.
- NMR (CD₃OD) δ :: 1.34(3H, t, J=7.6Hz), 2.89(2H, q, J=8.6, 15.0Hz), 5.47(2H, s), 7.15(2H, d, J=8.2Hz), 7.24(1H, s), 7.28(2H, d, J=8.2Hz), 7.5-7.7(4H, m).

### Working Example 2

### Ethyl 2-ethyl-7-[[2'-(1H-tetrazol-5-yl)biphenyl-4-yl]methyl]-4-oxo-4,7-dihydrothieno[2,3-b]pyridine-5-carboxylate

A solution of the compound (727 mg, 1 mmol) prepared in Reference Example 4 in 20 ml of trifluoroacetic acid/water (1:3) was stirred at room temperature for 1 hour. The reaction mixture was poured into water followed by extraction with chloroform. The organic layer was washed with water, dried (MgSO₄), and evaporated to dryness. The resulting residue was purified by flash column chromatography on silica gel. The column was eluted with chloroform/methanol (9:1) to give 1.28 g (86 %) of the title compound as colorless crystals. This product was identified by comparing with NMR and IR spectra of the compound obtained in Working Example 1B.
M.p. 161-164 °C.

| Elemental Analysis for C₂₆H₂₃N₅O₃S · H₂O | | | |
|---|---|---|---|
| | C (%) | H (%) | N (%) |
| Calcd: | C, 62.01; | H, 5.00; | N, 13.91 |
| Found: | C, 62.05; | H, 4.59; | N, 13.78 |

The following compounds as listed in Table 5 were prepared in the same manner as in Working Examples 2.

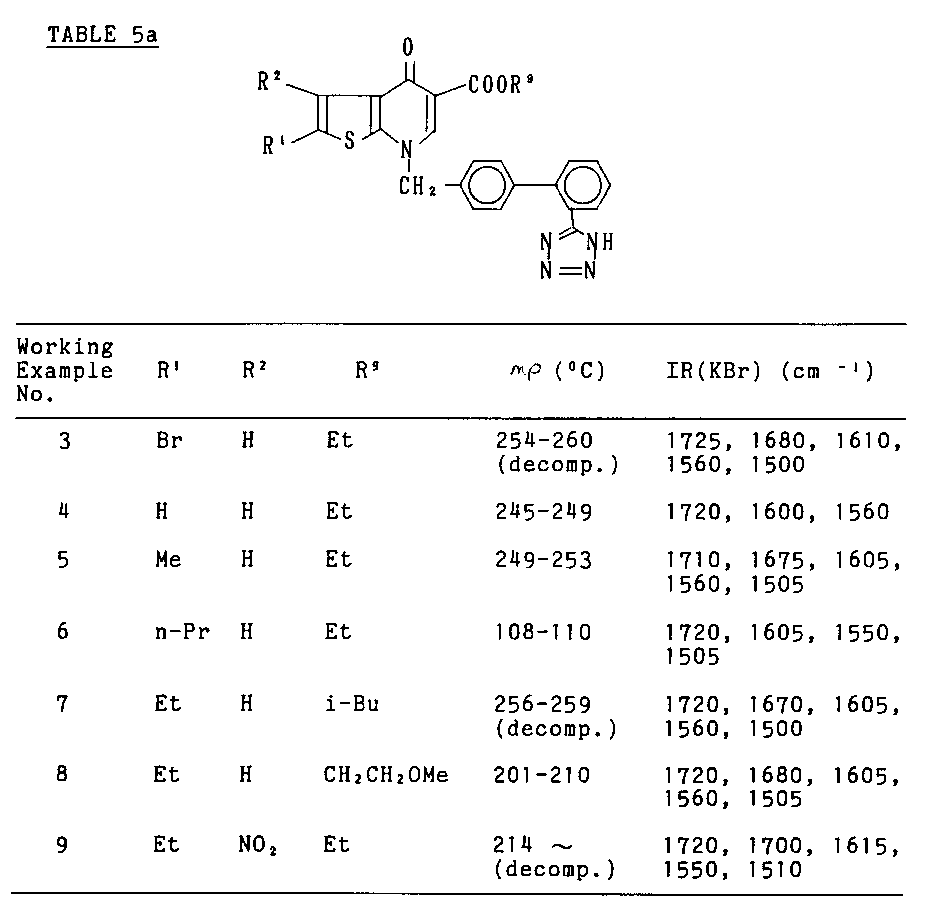

**TABLE 5b**

| Working Example No. | NMR (DMSO-d₆) δ | E. Anal. (Calcd/Found) C(%), H(%),N(%) |
|---|---|---|
| 3 | 1.29(3H,t,J=7.2Hz),4.24(2H,q, J=7.2,14.2Hz),5.46(2H,s),7.13(2H, d,J=8.2Hz),7.29(2H,d,J=8.2Hz), 7.5-7.7(5H,m),8.79(1H,s) | C₂₄H₁₈BrN₅O₃S 53.74;3.38;13.06 53.57;3.34;12.80 |
| 4 | 1.29(3H,t,J=7.2Hz),4.24(2H,q, J=7.2,14.2Hz),5.51(2H,s),7.13(2H, d,J=8.0Hz),7.27(2H,d,J=8.0Hz), 7.3-7.8(8H,m),8.80(1H,s) | C₂₄H₁₉N₅O₃S · 0.5H₂O 61.79;4.32;15.01 61.94;4.17;14.80 |
| 5 | 1.29(3H,t,J=7.2Hz),2.44(3H,d, J=1.0Hz),4.23(2H,q,J=7.2,14.2Hz), 5.45(2H,s),7.08(1H,d,J=1.0Hz), 7.13(2H,d,J=8.0Hz),7.25(2H,d, J=8.0Hz),7.5-7.7(4H,m),8.74(1H,s) | C₂₅H₂₁N₅O₃S 63.68;4.49;14.85 63.39;4.47;14.67 |
| 6 | 0.91(3H,t,J=7.2Hz),1.28(3H,t, J=7.0Hz),1.61(2H,q,J=7.2,15.0Hz), 2.76(2H,t,J=7.6Hz),4.23(2H,q, J=7.0,14.2Hz),5.45(2H,s),7.10(1H, s),7.12(2H,d,J=8.4Hz),7.26(2H,d, J=8.4Hz),7.5-7.7(4H,m), 8.74(1H,s) | C₂₇H₂₅N₅O₃S · H₂O 62.65;5.26;13.53 62.55;4.84;13.38 |
| 7 | 0.96(6H,d,J=6.8Hz),1.23(3H,t, J=7.4Hz),1.9-2.1(1H,m),2.80(2H, q,J=7.4,15.0Hz),3.97(2H,d, J=6.6Hz),5.46(2H,s),7.11(1H, s),7.13(2H,d,J=8.0Hz),7.27(2H,d, J=8.0Hz),7.5-7.7(4H,m), 8.70(1H,s) | C₂₈H₂₇N₅O₃S · 0.5H₂O 64.35;5.40;13.40 64.22;5.24;13.47 |
| 8 | 1.23(3H,t,J=7.4Hz),2.81(2H,q, J=7.4,15.0Hz),3.30(3H,s),3.62 2H,t,J=4.8Hz),4.31(2H,t,J=4.8Hz) 5.46(2H,s),7.11(1H,s),7.12(2H,d, J=8.0Hz),7.25(2H,d,J=8.0Hz), 7.5-7.7(4H,m),8.73(1H,s) | |
| 9 | 1.20(3H,t,J=7.4Hz),1.29(3H,t, J=7.0Hz),2.83(2H,q,J=7.4,15.4Hz), 4.24(2H,q,J=7.0,14.0Hz),5.53(2H, s),7.14(2H,d,J=8.2Hz),7.30(2H,d, J=8.2Hz),7.5-7.7(4H,m),8.86(1H,s) | C₂₆H₂₂N₆O₅S · H₂O 56.93;4.41;15.32 57.12;4.05;15.26 |

### Working Example 10

### 2-Ethyl-7-[[2'-(1H-tetrazol-5-yl)biphenyl-4-yl]methyl]-4-oxo-4,7-dihydrothieno[2,3-b]pyridine-5-carboxylic acid

Ethyl 2-ethyl-7-[[2'-(1H-tetrazol-5-yl) biphenyl-4-yl]methyl]-4-oxo-4,7-dihydrothieno[2,3-b]pyridine-5-carboxylate (600 mg, 1.235 mmol) was dissolved in 6 ml of 1N sodium hydroxide and the mixture was heated at 100 °C for 20 minutes with stirring. After cooling, 7 ml of 1N hydrochloric acid was added to the reaction mixture and the mixture was extracted with chloroform. The organic layer was washed with water, dried (MgSO₄), and evaporated to dryness. The resulting crystal was washed with dichloromethane and dried 550 mg of the title compound as colorless crystals. This product was identified by comparing with NMR and IR spectra of the compound obtained in Working Example 1B. M.p. 153-157 °C.

| Elemental Analysis for C₂₄H₁₉N₅O₃S · 3.5H₂O | | | |
|---|---|---|---|
| | C (%) | H (%) | N (%) |
| Calcd: | C, 55.38; | H, 5.03; | N, 13.48 |
| Found: | C, 54.75; | H, 3.93; | N, 13.15 |

### Working Example 11

### A: Ethyl 2-ethyl-7-[(2'-t-butoxycarbonylbiphenyl-4-yl)methyl]-4-oxo-4,7-dihydrothieno[2,3-b]pyridine-5-carboxylate

A mixture of ethyl 2-ethyl-4-hydroxythieno[2,3-b]pyridine-5-carboxylate (200 mg, 0.8 mmol), 4-(2-t-butoxycarbonylphenyl)benzyl bromide (300 mg, 0.9 mmol) and cesium carbonate (650 mg, 2.0 mmol) in DMF (10 ml) was stirred at 60 °C for 3 hours and further at 100 °C for an additional hour. After cooling, the reaction mixture was poured into water followed by extraction with ethyl acetate. The organic layer was washed with water, dried (MgSO₄), and evaporated to dryness. The resulting residue was purified by flash column chromatography on silica gel to give 258 mg (62 %) of the title compound as crystals.
IR (KBr)cm⁻¹: 1720, 1705, 1690, 1510.
- NMR (CDCl₃) δ :: 1.20(9H, s), 1.32(3H, t, J=7.6Hz), 1.41(3H, t, J=7.2Hz), 2.81(2H, q, J=7.6, 14.6Hz), 4.40(2H, q, J=7.2, 14.2Hz), 5.23(2H, s), 7.2-7.5(8H, m), 7.81(1H, dd, J=1.6, 7.6Hz), 8.42(1H, s).

| Elemental Analysis for C₃₀H₃₁NO₅S | | | |
|---|---|---|---|
| | C (%) | H (%) | N (%) |
| Calcd: | C, 69.61; | H, 6.04; | N, 2.71 |
| Found: | C, 69.85; | H, 6.15; | N, 2.37 |

### B: Ethyl 2-ethyl-7-[(2'-carboxybiphenyl-4-yl)methyl]-4-oxo-4,7-dihydrothieno[2,3-b]pyridine-5-carboxylate

To 5 ml of trifluoroacetic acid under ice-cooling were added the compound (200 mg, 0.39 mmol) prepared in Working Example 11A and anisole (0.1 ml) and the mixture was stirred for 2.5 hours. The reaction mixture was concentrated to dryness in vacuo. To the resulting residue was added dichloromethane followed by evaporation to dryness in vacuo. These treatments were repeated twice and ether was added to the resulting residue to precipitate solids which were filtered and dried to give 167 mg (93 %) of the title compound as pale yellow powders.
IR (KBr)cm⁻¹: 1715, 1680, 1605.
- NMR (d₆-DMSO) δ :: 1.23(3H, t, J=7.4Hz), 1.29(3H, t, J=7.2Hz), 2.80(2H, q, J=7.4, 15.0Hz), 4.23(2H, q, J=7.2, 13.8Hz), 5.49(2H, s), 7.11(1H, s), 7.3-7.6(7H, m), 7.73(1H, d, J=7.4Hz), 8.77(1H, s).

| Elemental Analysis for C₂₆H₂₃NO₅S · 0.3H₂O | | | |
|---|---|---|---|
| | C (%) | H (%) | N (%) |
| Calcd: | C, 66.88; | H, 5.09; | N, 3.00 |
| Found: | C, 67.03; | H, 5.14; | N, 2.95 |

### Working Example 12

### 2-Ethyl-5-(N-benzylcarbamoyl)-7-[[2'-(1H-tetrazol-5-yl)biphenyl-4-yl]methyl]-4-oxo-4,7-dihydrothieno[2,3-B]pyridin-4(7H)-one

A mixture of the compound (123 mg, 0.25 mmol) prepared in Working Example 2 and benzylamine (2 ml) was stirred at 60 °C for 3 days. After cooling, the reaction mixture was poured into chloroform, washed twice with 1N hydrochloric acid, dried (MgSO₄), and concentrated. The resulting residue was purified by flash column chromatography on silica gel. The column was eluted with chloroform/methanol (19:1 to 9:1) to give 63 mg (45 %) of the title compound as crystals. m p 177-180 °C.
IR (KBr)cm⁻¹: 1650, 1590, 1550, 1500.
- NMR (DMSO-d₆)δ :: 1.25(3H, t, J=7.4Hz), 2.83(2H, q, J=7.4Hz, 14.2Hz), 4.56(2H, d, J=5.8Hz), 5.57(2H, s), 7.0-7.7(14H, m), 8.95(1H, s).

| Elemental Analysis for C₃₁H₂₆N₆O₂S | | | |
|---|---|---|---|
| | C (%) | H (%) | N (%) |
| Calcd: | C, 68.11; | H, 4.79; | N, 15.37 |
| Found: | C, 68.06; | H, 4.79; | N, 15.17 |

The following compounds as listed in Table 6 were prepared in the same manner as in Working Examples 12.

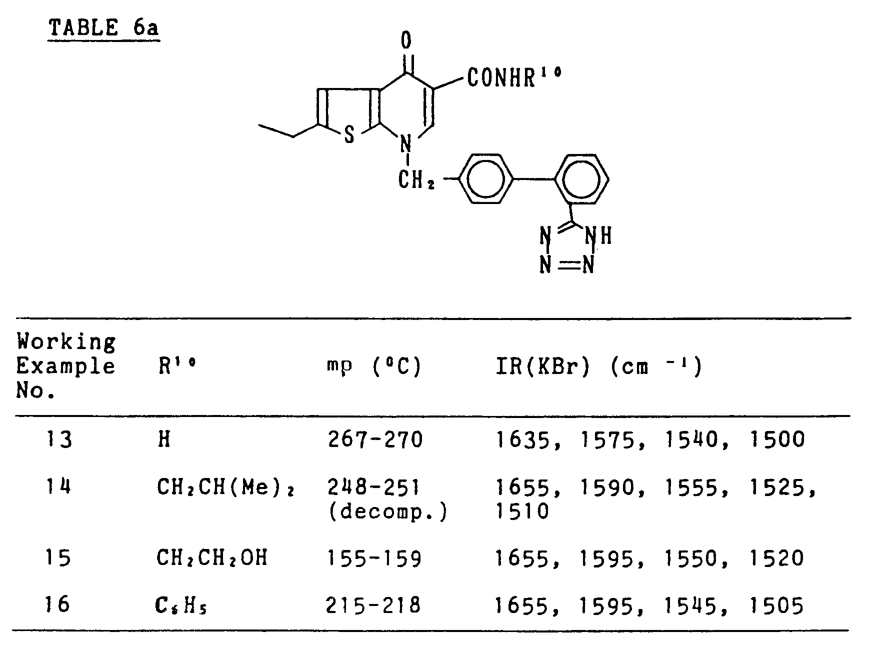

**TABLE 6b**

| Working Example No. | NMR (DMSO-d₆) δ | E. Anal. (Calcd/Found) C(%), H(%), N(%) |
|---|---|---|
| 13 | 1.25(3H,t,J=7.6Hz),2.83(2H,q, J=7.6,14.8Hz),5.54(2H,s),7.12(2H, d,J=8.2Hz),7.22(1H,s),7.26(2H,d, J=8.2Hz),7.5-7.7(4H,m),8.91(1H,s), 9.46(1H,bs) | C₂₄H₂₀N₆O₂S · H₂O 60.75;4.67;17.71 61.09;4.41;17.37 |
| 14 | 0.93(6H,d,J=6.6Hz),1.25(3H,t, J=7.4Hz),1.7-1.9(1H,m),2.84(2H,d, J=7.4,14.0Hz),3.18(2H,t,J=6.2Hz), 5.55(2H,s),7.11(2H,d,J=8.2Hz), 7.19(1H,s),7.25(2H,d,J=8.2Hz), 7.5-7.7(4H,m),8.90(1H,s) | C₂₈H₂₈N₆O₂S · H₂O 63.38;5.70;15.84 63.70;5.44;15.50 |
| 15 | 1.25(3H,t,J=7.4Hz),2.84(2H,q, J=7.4,16.0Hz),3.3-3.6(4H,m), 4.81(1H,bs),5.55(2H,s),7.11(2H, d,J=8.2Hz),7.18(1H,s),7.24(2H,d J=8.2Hz),7.5-7.7(4H,m),8.31(1H,s), 8.90(1H,s) | |
| 16 | 1.27(3H,t,J=7.4Hz),2.87(2H,q_{,} J=7.4,14.6Hz),5.59(2H,s),7.0-7.8 (14H,m),9.05(1H,s) | C₃₀H₂₄N₆O₂S · 0.5H₂O 66.53;4.65;15.52 66.28;4.51;15.26 |

### Working Example 17

### Ethyl 3-acetylamino-2-ethyl-7-[[2'-(1H-tetrazol-5-yl)biphenyl-4-yl]methyl]-4-oxo-4,7-dihydrothieno[2,3-b]pyridine-5-carboxylate

Ethyl 2-ethyl-3-nitro-7-[[2'-(N-trityltetrazol-5-yl)biphenyl-4-yl]methyl]-4-oxo-4,7-dihydrothieno[2,3-b]pyridine-5-carboxylate (200 mg, 0.26 mmol) was dissolved in a mixture of acetic anhydride (6 ml), acetic acid (6 ml), dioxane (4 ml) and tetrahydrofuran (5 ml) and the solution was heated to 70 °C. To the heated solution were added zinc powders (85 mg) and the reaction mixture was stirred for 2 hours. The insoluble material was removed from the reaction mixture by filtration and the filtrate was concentrated to dryness in vacuo. The resulting oil was dissolved in a mixture of trifluoroacetic acid (8 ml) and water (1 ml) and the solution was stirred at room temperature for 5 hours. The reaction mixture was poured into water and extracted with chloroform. The organic layer was washed with water, dried (MgSO₄), and evaporated to dryness. The resulting residue was purified by flash column chromatography on silica gel. The column was eluted with chloroform/methanol (20:1) to give 59 mg (42 %) of the title compound as white crystals.
m p 163-166 °C.
IR (KBr)cm⁻¹ : 1720, 1700, 1615, 1550, 1510.
- NMR (CDCl₃) δ :: 1.14(3H, t, J=7.4Hz), 1.29(3H, t, J=7.0Hz), 2.03(3H, s), 2.63(2H, q, J=7.4, 15.0Hz), 4.23(2H, q, J=7.0, 14.2Hz), 5.45(2H, s), 7.13(2H, d, J=8.2Hz), 7.28(2H, d, J=8.2Hz), 7.5-7.7(4H, m), 8.74(1H, s), 9.66(1H, Bs).

| Elemental Analysis for C₂₈H₂₆N₆O₄S · H₂O | | | |
|---|---|---|---|
| | C (%) | H (%) | N (%) |
| Calcd: | C, 59.99; | H, 5.03; | N, 14.99 |
| Found: | C, 59.50; | H, 4.56; | N, 14.76 |

The following compounds (Working Examples 18- 20) as listed in Table 7 were prepared in the same manner as in Working Examples 12.

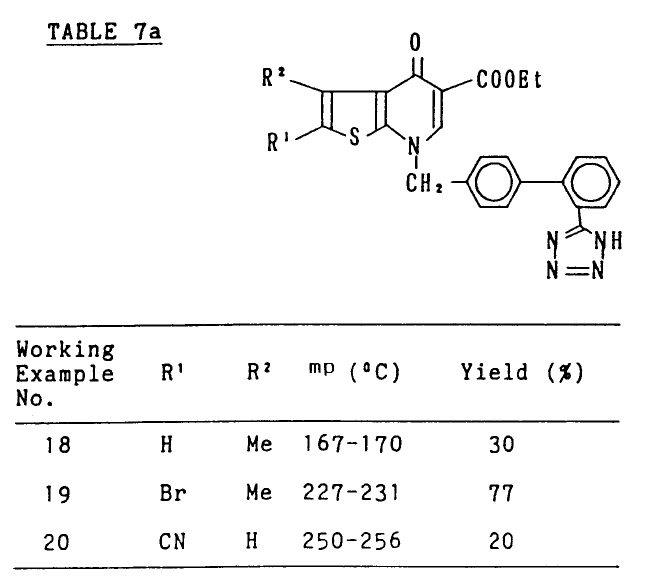

**TABLE 7b**

| Working Example No. | NMR (DMSO-d₆) δ | E. Anal. (Calcd/Found) C(%), H(%), N(%) |
|---|---|---|
| 18 | 1.28(3H,t),2.47(3H,s),4.22(2H,q), 5.44(2H,s),6.92(1H,s),7.10(2H,d), 7.23(2H,d),7.5-7.7(4H,m),8.70(1H, s) | C₂₅H₂₁N₅O₃S · 0.2H₂O 63.20;4.54;14.74 63.00;4.37;14.80 |
| 19 | 1.29(3H,t),2.45(3H,s),4.23(2H,q), 5.43(2H,s),7.13(1H,d),7.27(2H,d), 7.5-7.8(4H,m),8.73(1H,s) | C₂₅H₂₀BrN₅O₃S 54.55;3.66;12.72 54.82;3.63;12.83 |
| 20 | 1.29(3H,t),4.25(2H,q),5.52(2H,s), 7.13(2H,d),7.31(2H,d),7.5-7.8 (4H,m),8.25(1H,s),8.89(1H,s) | |

The following compounds (Working Examples 21-36) were prepared in the same manner as in Working Example 12.

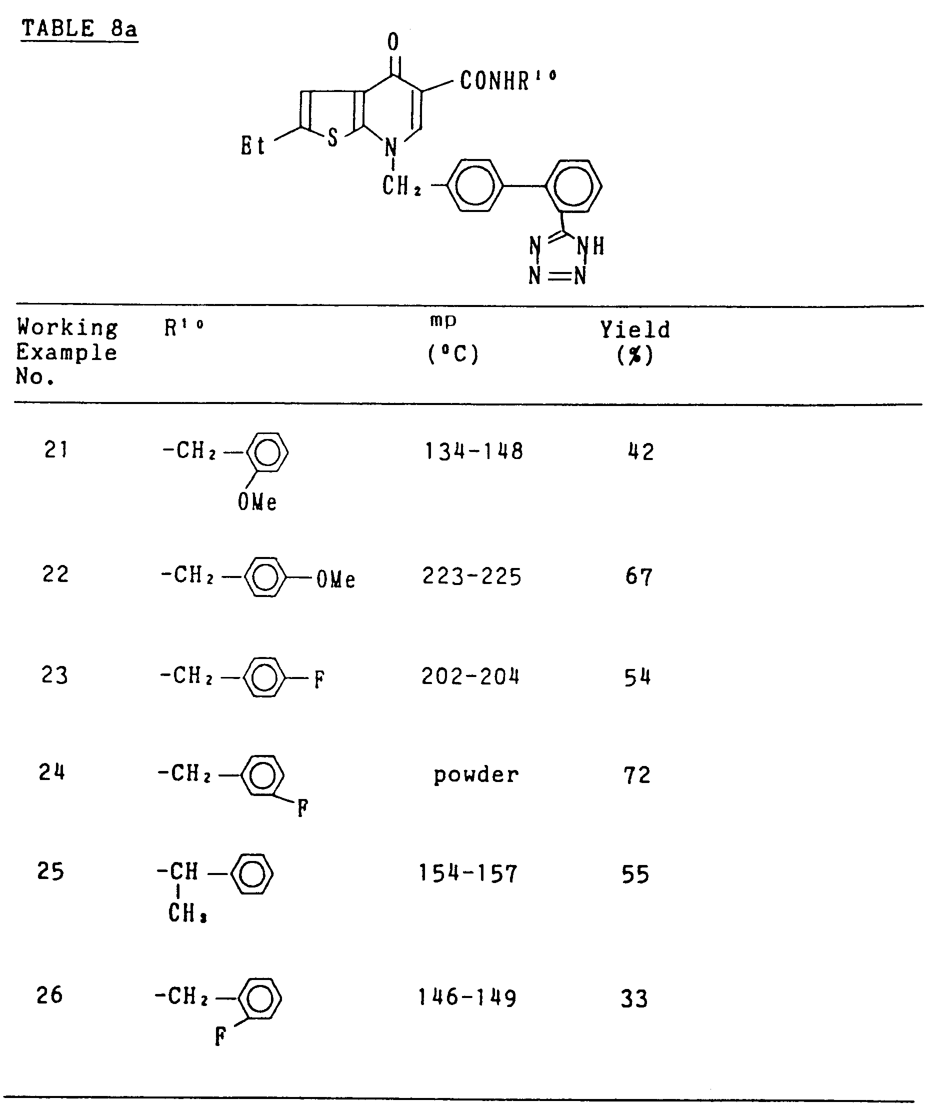

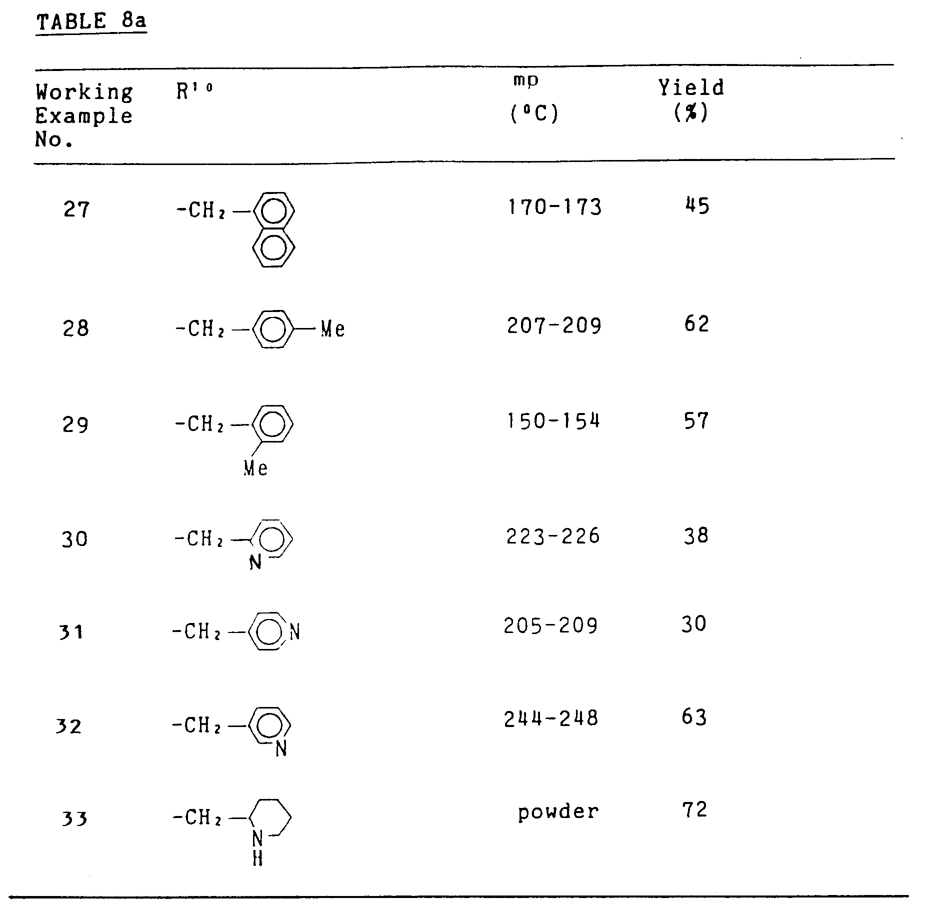

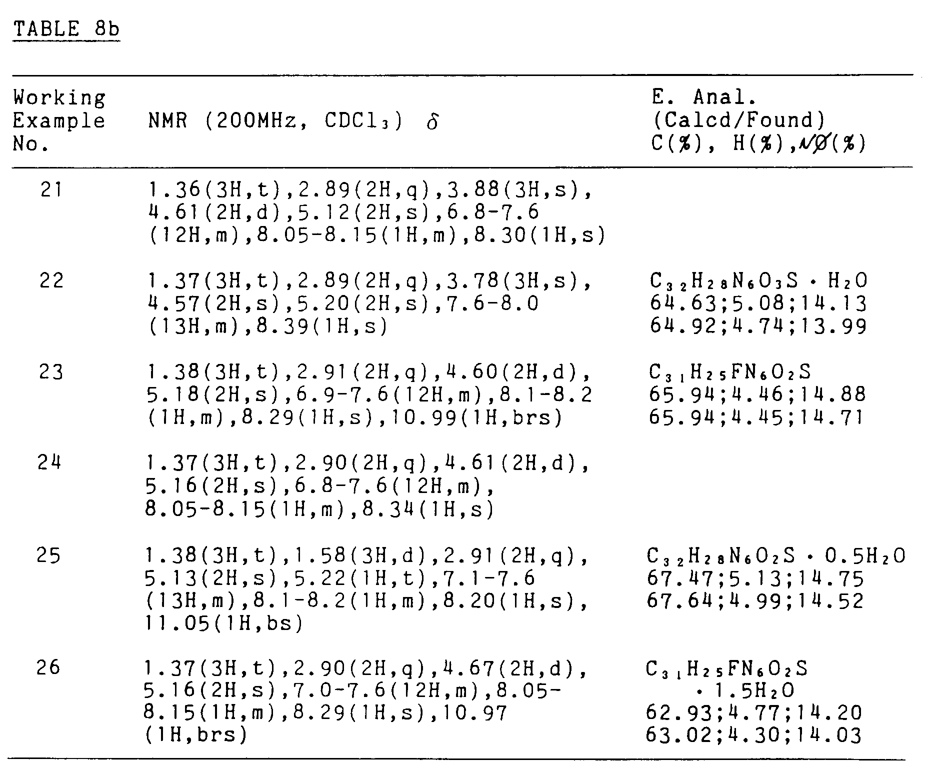

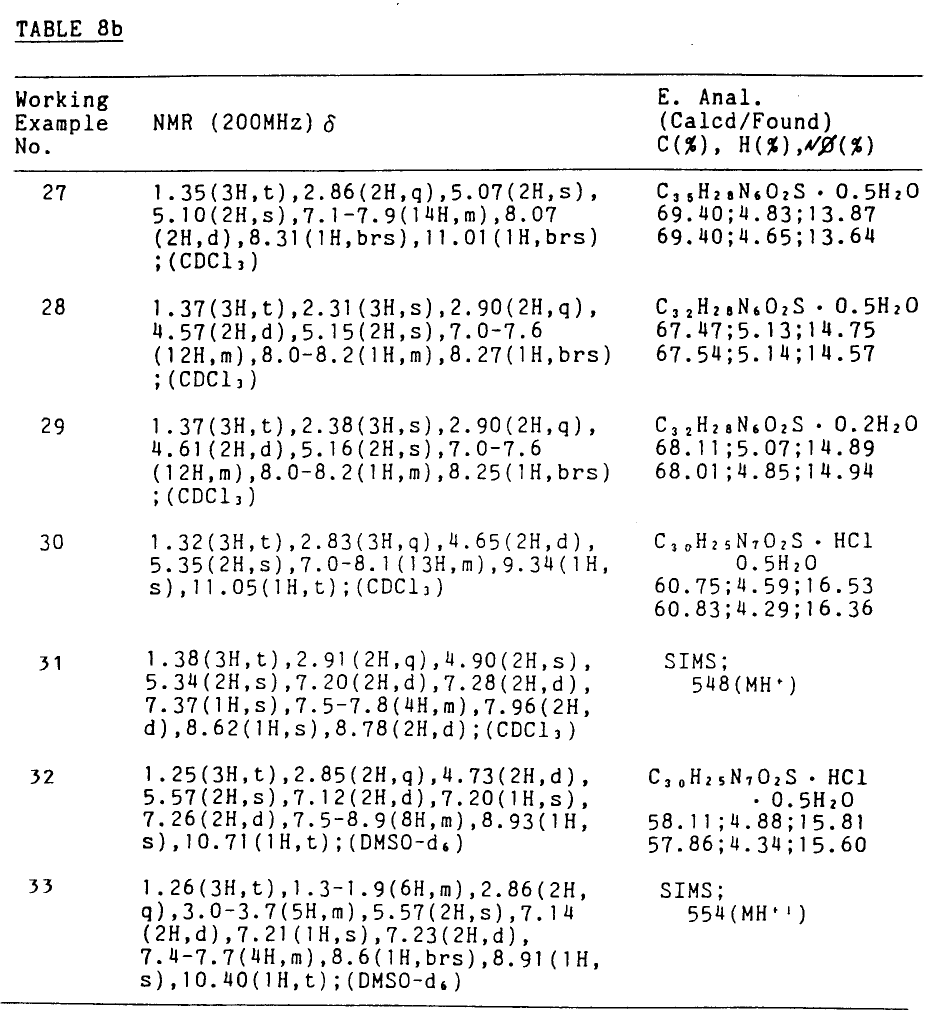

The following compounds (Working Examples 34-41) were prepared in the same manner as in Working Example 12.

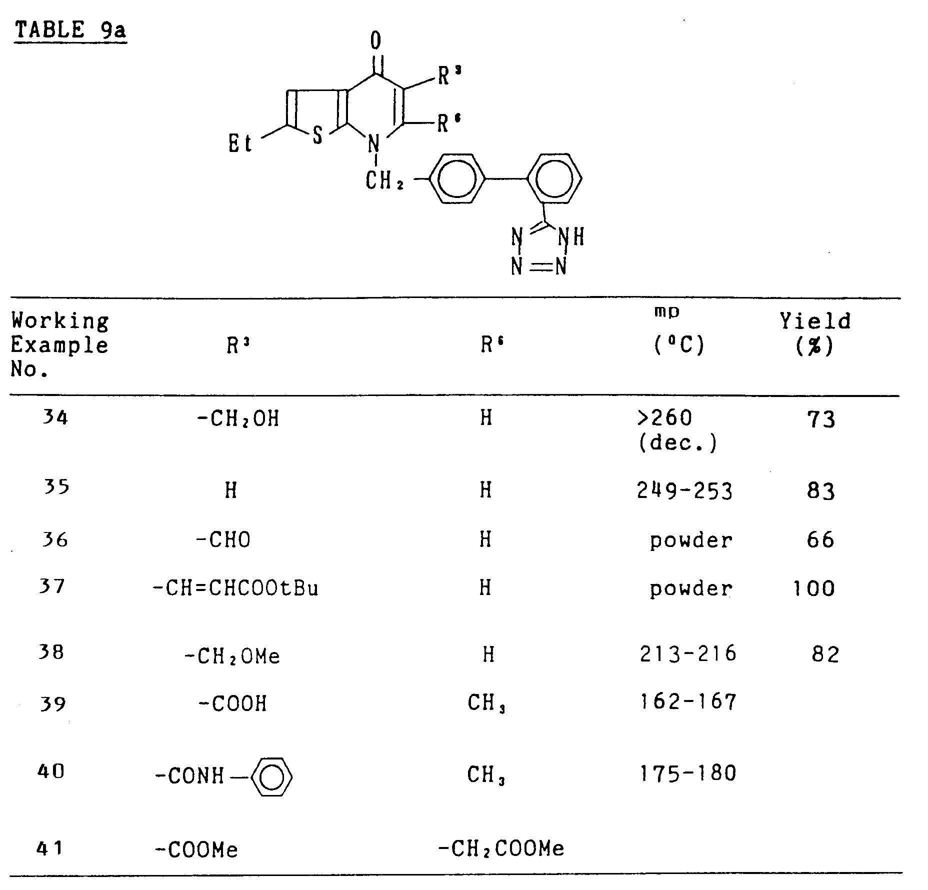

**TABLE 9b**

| Working Example No. | NMR (200MHz) δ | E. Anal. (Calcd/Found) C(%), H(%), N(%) |
|---|---|---|
| 34 | 1.24(3H,t),2.79(2H,q),4.40(2H,s), 7.05(1H,s),7.11(2H,d),7.22(2H,d), 7.5-7.8(4H,m),7.98(1H,s);(DMSO-d₆) | C₂₄H₂₁N₅O₂S · 0.8H₂O 62.95;4.97;15.29 63.18;4.88;14.86 |
| 35 | 1.24(3H,t),2.80(2H,q),5.35(2H,s), 6.21(1H,d),7.07(1H,s),7.11(2H,d), 7.24(2H,d),7.4-7.8(4H,m), 8.08(1H,d);(DMSO-d₆) | C₂₃H₁₉N₅OS · H₂O 64.02;4.91;16.23 63.87;4.44;15.90 |
| 36 | 1.24(3H,t),2.82(2H,q),5.50(2H,s), 7.12(2H,d),7.20(1H,s),7.28(2H,d), 7.5-7.7(4H,m),8.69(1H,s),10.28(1H, s);(DMSO-d₆) | |
| 37 | 1.25(3H,t),1.37(9H,s),2.80(2H,q), 5.35(2H,s),6.93(1H,d),7.1-7.9(10H, m),8.55(1H,s);(DMSO-d₆) | |
| 38 | 1.21(3H,t),2.69(2H,q),3.44(3H,s), 4.09(2H,s),5.21(2H,s), 6.8-7.9(10H,m);(CDCl₃) | C₂₅H₂₃N₅O₂S · 0.7H₂O 63.87;5.23;14.90 64.00;4.99;14.69 |
| 39 | 1.27(3H,t),2.88(2H,q),2.95(3H,s), 5.68(2H,s),7.11(4H,s),7.29(1H,s), 7.5-7.8(4H,m);(DMSO-d₆) | C₂₅H₂₁N₅O₃S · 0.5H₂O 62.49;4.61;14.57 62.28;4.36;14.39 |
| 40 | 1.39(3H,t),2.75(3H,s),2.99(2H,q), 5.85(2H,s),7.1-7.8(14H,m);(CD₃OD) | SIMS; 547(MH⁺) |
| 41 | 1.32(3H,t),2.86(2H,q),3.66(3H,s), 3.88(3H,s),3.94(2H,s),5.55(2H,s), 6.98(2H,d),7.13(2H,d),7.22(1H,s) 7.4-7.7(4H,m);(CD₃OD) | SIMS; 544(MH⁺) |

The following compounds (Working Examples 42-48) were prepared in the same manner as in Working Example 2.

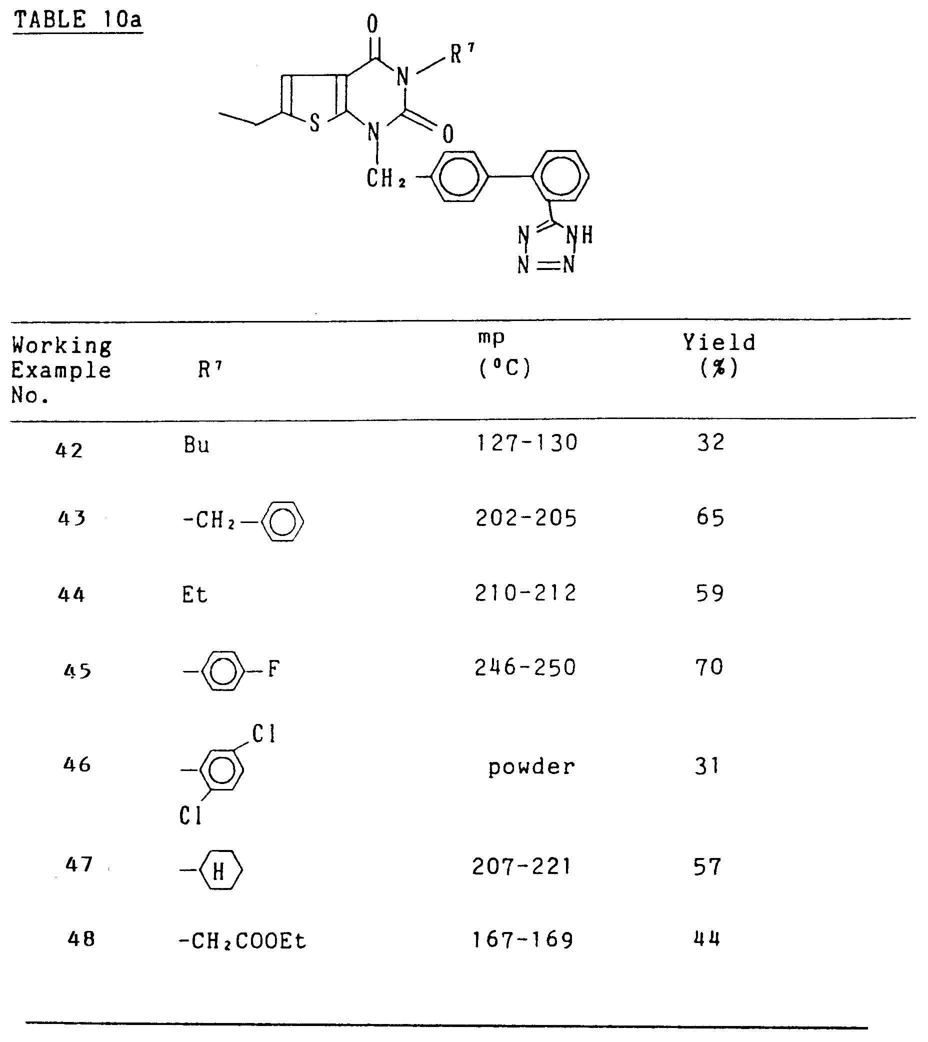

**TABLE 10b**

| Working Example No. | ¹H-NMR (CDCl₃) δ | E. Anal. (Calcd/Found) C(%), H(%), N(%) |
|---|---|---|
| 42 | 0.92(3H,t),1.30(3H,t),1.2-1.5(2H, m),1.5-1.8(2H,m),2.77(2H,q),4.01 (2H,t),5.15(2H,s),7.00(1H,s), 7.23(2H,d),7.42(2H,d),7.5-7.7(3H, m),8.1-8.2(1H,m) | C₂₆H₂₆N₆O₂S 64.18;5.39;17.27 64.00;5.51;17.18 |
| 43 | 1.21(3H,t),2.75(2H,q),5.12(2H,s), 5.15(2H,s),7.02(1H,s),7.09(2H,d), 7.2-7.8(11H,m) | C₂₉H₂₄N₆O₂S · 0.4H₂O 65.99;4.74;15.92 66.17;4.91;15.63 |
| 44 | 1.25(3H,t),1.30(3H,t),2.78(2H,q), 4.08(2H,q),5.16(2H,s),7.01(1H,s), 7.23(2H,d),7.43(2H,d),7.5-7.7(3H, m),8.1-8.2(1H,m) | C₂₄H₂₂N₆O₂S · 0.5H₂O 61.65;4.96;17.97 61.70;4.83;17.80 |
| 45 | 1.32(3H,t),2.81(2H,q),5.14(2H,s), 7.06(1H,s),7.1-7.8(12H,m) | C₂₈H₂₁,FN₆O₂S 64.11;4.04;16.02 63.82;3.97;15.77 |
| 46 | 1.33(3H,t),2.81(2H,q),5.20(2H,q), 7.07(1H,s),7.2-8.2(11H,m) | C₂₈H₂₀N₆O₂SCl₂ · 0.6H₂O 57.36;3.64;14.33 57.74;3.75;13.75 |
| 47 | 1.29(3H,t),1.2-2.6(10H,m),2.76 (2H,q),4.7-5.0(1H,m),5.13(2H,s), 6.99(1H,s),7.24(2H,d),7.42(2H,d), 7.5-7.7(3H,m),8.1-8.2(1H,m) | C₂₈H₂₈N₆O₂S · 0.4H₂O 64.69;5.58;16.17 64.81;5.50;15.95 |
| 48 | 1.29(3H,t),1.31(3H,t),2.79(2H,q), 4.23(2H,q),4.80(2H,s),5.17(2H,s), 7.03(1H,s),7.22(2H,d),7.4-7.7(5H, m),8.1-8.2(1H,m) | C₂₆H₂₄N₆O₄S 60.45;4.68;16.27 60.33;4.59;16.17 |

### Pharmaceutical Examples

The compounds (I) of the present invention are employed, for example, when used as agents for treating circulatory system diseases such as hypertension, heart diseases, strokes and the like, in the following formulations.

### 1. Capsule

| | |
|---|---|
| (1) Ethyl 2-ethyl-7-[[2'-(1H-tetrazol-5-yl)biphenyl-4-yl]methyl]-4-oxo-4,7-dihydrothieno-[2,3-b]pyridine-5-carboxylate | 10 mg |
| (2) Lactose | 90 mg |
| (3) Microcrystalline cellulose | 70 mg |
| (4) Magnesium stearate | 10 mg |
| One capsule | 180 mg |

The ingredients (1), (2), and (3) and a half of the ingredient (4) were blended together and granulated. To this mixture was added the remaining half of the ingredient (4) and distributed into gelatine capsules.

### 2. Tablet

| | |
|---|---|
| (1) Ethyl 2-ethyl-7-[[2'-(1H-tetrazol-5-yl)biphenyl-4-yl]methyl]-4-oxo-4,7-dihydrothieno-[2,3-b]pyridine-5-carboxylate | 10 mg |
| (2) Lactose | 35 mg |
| (3) Maize starch | 150 mg |
| (4) Microcrystalline cellulose | 30 mg |
| (5) Magnesium stearate | 5 mg |
| One tablet | 230 mg |

Two third each of the ingredients (1), (2), (3) and (4) and a half of the ingredient (5) were blended together and granulated. To these granules were added the remaining ingredients (4) and (5) and then compressed to form tablets.

### 3. Injection

| | |
|---|---|
| (1) 2-Ethyl-7-[[2'-(1H-tetrazol-5-yl)biphenyl-4-yl]methyl]-4-oxo-4,7-dihydrothieno[2,3-b]pyridine-5-carboxylic acid · sodium salt | 10 mg |
| (2) Inositol | 100 mg |
| (3) Benzyl alcohol | 20 mg |
| One ampule | 130 mg |

The ingredients (1), (2) and (3) were dissolved in distilled water for injection to a total volume of two ml and distributed into ampules. Total processes were carried out under sterile conditions.

### Experimental Example 1

### Inhibition of binding of angiotensin-II to angiotensin receptor

### [Method]

An experiment of inhibition on the binding of angiotensin-II (A-II) to A-II-receptor was conducted by modifying the method of Douglas et al. [Endocrinology, 102, 685-696 (1978)]. An A-II-receptor was prepared from the membrane fraction of bovine adrenal cortex.

The compound of the present invention (10⁻⁹M to 3 × 10⁻⁵M ) and ¹²⁵I-A-II (1.85 kBq/50 µ l) were added to the receptor membrane fraction, and the mixture was incubated at room temperature for one hour. The receptor-bound and free ¹²⁵I-A-II were separated through a filter (Whatman GF/B filter), and the radioactivity of ¹²⁵I-A-II bound to the receptor was measured.

### [Results]

The results relating to the compounds of the present invention are shown in Table 11.

### Experimental Example 2

### Inhibitory effect of the compound of the present invention on pressor action of A-II

### [Method]

Jcl : SD rats (9 week old, male) were used. On the day previous to the experiment, these animals were applied with cannulation into the femoral artery and vein under anesthesia with pentobarbital Na. The animals were fasted but allowed to access freely to drinking water until the experiment was started. Just on the day of conducting the experiment, the artery cannula was connected with a blood-pressure transducer, and the average blood pressure was recorded by means of polygraph. Before administration of the drug, the pressor action due to intravenous administration of A-II (100 ng/kg) as the control was measured. The drugs were orally administered, and then, at each point of the measurement, A-II was administered intravenously, and the pressor action was similarly measured. By comparing the pressor action before and after administration of the drug, the percent inhibition by the drug on A-II-induced pressor action was evaluated.

### [Results]

The results relating to the compounds of the present invention are shown in Table 11.

**TABLE 11**

| Working Example No. | Radio Receptor Assay IC₅₀(µM) | Pressor Response (30 mg/Kg, p.o.) |
|---|---|---|
| 2 | 0.05 | NT*^{a} |
| 3 | 0.07 | NT |
| 4 | 0.20 | NT |
| 5 | 0.05 | NT |
| 6 | 0.09 | NT |
| 7 | 0.17 | NT |
| 8 | 0.01 | NT |
| 10 | 0.04 | +*^{b} |
| 11 | 0.21 | NT |
| 12 | 0.06 | ++ |
| 13 | 0.02 | NT |
| 14 | 0.13 | NT |
| 15 | 0.05 | NT |
| 16 | 0.12 | ++ |
| 20 | 0.67 | NT |
| 21 | 0.25 | ++ |
| 23 | 0.17 | ++ |
| 24 | 0.19 | ++ |
| 25 | 0.78 | ++ |
| 26 | 0.21 | ++ |
| 28 | 0.22 | + |
| 29 | 0.12 | ++ |
| 30 | 0.02 | ++ |

| | | |
|---|---|---|
| *a : NT , not tested. | | |
| *b : (% Inhibition), ++ ≧ 70 % > + ≧ 50 %. | | |

It is understood that the preceding representative examples may be varied within the scope of the present invention by one skilled in the art to achieve essentially the same results.

As many widely different embodiments of this invention may be made without departing from the spirit and scope thereof, it is to be understood that this invention is not limited to the specific embodiments thereof except as defined in the appended claims.

## Claims (Claims for the following Contracting State(s): AT, BE, CH, DE, DK, FR, GB, IT, LI, LU, NL, SE)

1. A compound of the formula: 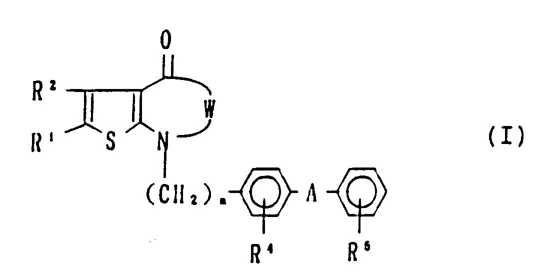 wherein W is 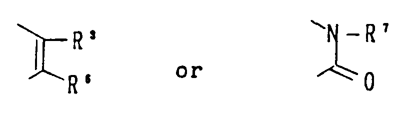 R¹ and R² which may be the same or different, are each independently
(1) hydrogen,
(2) halogen,
(3) cyano,
(4) nitro,
(5) a group having the formula: R⁸CONH- wherein R⁸ is hydrogen or (C₁₋₈)alkyl group which may be substituted with hydroxyl, (C₁₋₄)alkoxy, (C₁₋₄)alkyl, halogen, nitro, amino, methylamino, dimethylamino, phenylamino, benzylamino, morpholino, piperidino, piperazino, N-phenylpiperazino, (C₁₋₄)alkanoyloxy, benzoyloxy, phenyl which may be substituted with halogen, nitro, (C₁₋₄)alkoxy, (C₁₋₄)alkyl at an optional position on the phenyl ring, or naphthyl, or
(6) a hydrocarbon residue selected from the group consisting of (i) (C₁₋₈)alkyl, (ii) (C₂₋₈)alkenyl, (iii) (C₂₋₈)alkynyl, (iv) (C₃₋₈)cycloalkyl, (v) an aromatic hydrocarbon selected from the group consisting of phenyl and naphthyl, wherein said hydrocarbon residue may be substituted with hydroxyl, (C₁₋₄)alkoxy, (C₁₋₄)alkyl, halogen, nitro, amino, N-lower (C₁₋₄)alkylamino, N,N-dilower (C₁₋₄)alkylamino, phenylamino, naphthylamino, benzylamino, naphthylmethylamino, morpholino, piperidino, piperazino, N-phenylpiperazino, N-(m-methoxy)phenylpiperazino, (C₁₋₄)alkanoyloxy, benzoyloxy, phenyl which may be substituted with halogen, nitro, (C₁₋₄)alkoxy, (C₁₋₄)alkyl at an optional position on the phenyl ring, or a group having the formula: -COD' wherein D' is hydroxy, (C₁₋₄)alkoxy, amino, N-lower (C₁₋₄)alkylamino, N,N-dilower (C₁₋₄)alkylamino, phenylamino, naphthylamino, benzylamino, naphthylmethylamino, morpholino, piperidino, piperazino, or N-phenylpiperazino;
R³ is
(1) hydrogen,
(2) (C₁₋₈)alkyl or (C₂₋₈)alkenyl, which may be straight or branched and may be optionally substituted with hydroxyl, amino, N-lower (C₁₋₄)alkyl amino, N,N-dilower (C₁₋₄)alkyl amino, halogen, lower (C₁₋₄)alkoxy, or -COD'' wherein D'' is lower (C₁₋₄)alkoxy, hydroxy, halogen, amino, N-lower (C₁₋₄)alkyl amino, N,N-dilower (C₁₋₄)alkyl amino, phenylamino, naphthylamino, benzylamino, naphthylmethylamino, morpholino, piperidino, piperazino, or N-phenylpiperazino, or
(3) -COD wherein D is hydrogen, C₁₋₄-alkoxy, hydroxy, halogen, amino, N-lower (C₁₋₄)alkyl amino, N,N-dilower (C₁₋₄)alkyl amino, phenylamino, benzylamino, naphthylmethylamino, pyridylamino, pyridylmethylamino, morpholino, piperidino, piperazino, piperidylmethyl, N-(p-fluorophenyl)piperazino, or N-phenylpiperazino, wherein said alkyl, aryl and heteroaryl groups may be optionally substituted with methyl, ethyl, propyl, isopropyl, butyl, isobutyl, sec-butyl, hydroxyl, amino, N-lower (C₁₋₄)alkyl amino, N,N-dilower (C₁₋₄)alkylamino, morpholino, piperidino, piperazino, N-phenylpiperazino, halogen, nitro or lower (C₁₋₄) alkoxy;
R⁴ is hydrogen, halogen or nitro;
R⁵ is carboxyl, lower (C₁₋₄)alkoxycarbonyl, cyano, tetrazolyl, trifluoromethanesulfonic amide, phosphoric acid or sulfonic acid;
R⁶ is hydrogen or (C₁₋₈)alkyl or (C₂₋₈)alkenyl, which may be straight or branched and may be optionally substituted with hydroxyl, amino, N-lower (C₁₋₄)alkyl amino, N,N-dilower (C₁₋₄)alkyl amino, halogen, lower (C₁₋₄)alkoxy, or -COD'' wherein D'' is lower (C₁₋₄)alkoxy, hydroxy, halogen, N-lower (C₁₋₄)alkyl amino, N,N-dilower (C₁₋₄)alkyl amino, phenylamino, naphthylamino, benzylamino, naphthylmethylamino, morpholino, piperidino, piperazino, or N-phenylpiperazino;
R⁷ is a hydrocarbon residue which may be substituted like R¹ and R²; A is a direct bond or a spacer having atomic length of two or less between the phenylene group and the phenyl group selected from the group consisting of (C₁₋₄)alkylene, -C(=O)-, -O-, -S-, -NH-, -C(=O)-NH-, -O-CH₂-, -S-CH₂-, or -CH=CH-; and n is an integer of 1 or 2;
and a pharmaceutically acceptable salt thereof.

2. A compound according to claim 1, which is a compound of the formula (Ia): 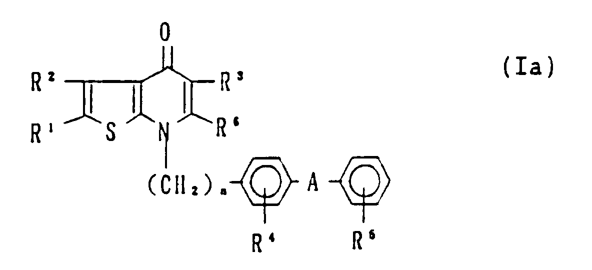 wherein R¹ and R² are as defined in claim 1;
R³ is hydrogen, formyl, optionally substituted alkyl or alkenyl as defined in claim 1, or -COD wherein D is alkoxy, hydroxy, halogen, or optionally substituted amino as defined in claim 1;
R⁴ is hydrogen, halogen or nitro;
R⁵ is as defined in claim 1;
R⁶, A and n are as defined in claim 1
and a pharmaceutically acceptable salt thereof.

3. A compound according to claim 1, which is a compound of the formula (Ib): 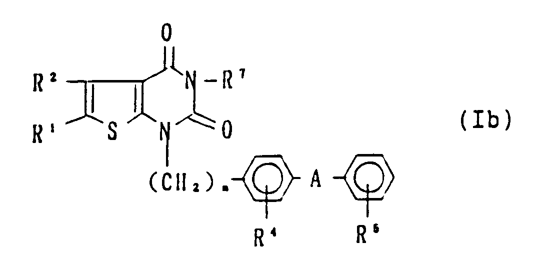 wherein R¹ and R² are as defined in claim 1;
R⁴ is hydrogen, halogen or nitro;
R⁵ is as defined in claim 1;
R⁷, A and n are as defined in claim 1;
and a pharmaceutically acceptable salt thereof.

4. A compound according to claim 1, wherein an optionally substituted amino as defined in claim 1 for the substituents of said hydrocarbon residue is amino, methylamino, dimethylamino, phenylamino, benzylamino, morpholino, piperidino, piperazino, N-phenylpiperazino, or N-(m-methoxy)phenylpiperazino.

5. A compound according to claim 1, wherein said D' is amino, methylamino, dimethylamino, phenylamino, benzylamino, morpholino, piperidino, piperazino, or N-phenylpiperazino.

6. A compound according to claim 1, wherein said D is amino, N- (C₁₋₄) alkyl amino, N,N-di (C₁₋₄)alkyl amino, phenylamino, benzylamino, naphthylmethylamino, pyridylamino, pyridylmethylamino, morpholino, piperidino, piperazino, piperidylmethyl, N-phenylpiperazino, or N-(p-fluorophenyl)piperazino wherein said alkyl, aryl and heteroaryl groups may be optionally substituted with methyl, ethyl, propyl, isopropyl, butyl, isobutyl, sec-butyl, hydroxyl, amino, N-lower (C₁₋₄)alkyl amino, N,N-dilower (C₁₋₄)alkylamino, morpholino, piperidino, piperazino, N-phenylpiperazino, halogen, nitro or lower (C₁₋₄)alkoxy.

7. A compound according to claim 1 , wherein said R⁵ is tetrazolyl.

8. A compound according to claim 1 , wherein said R⁵ is in the ortho position.

9. A compound according to claim 1, which is a compound of the formula (Ia'): 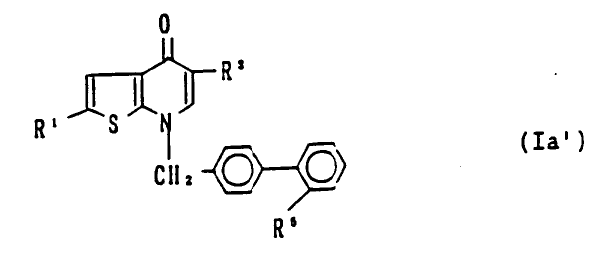 wherein R¹ is (C₁₋₈)alkyl; R³ is
(1) hydrogen,
(2) (C₁₋₈)alkyl which may be straight or branched and may be optionally substituted with hydroxyl, amino, N-lower (C₁₋₄)alkylamino, N,N-dilower (C₁₋₄)alkylamino, halogen, (C₁₋₄)alkoxy or -COD'' wherein D'' is (C₁₋₄)alkoxy, hydroxy, halogen, amino, N-lower (C₁₋₄)alkyl amino, N,N-dilower (C₁₋₄)alkylamino, phenylamino, naphthylamino, benzylamino, naphthylmethylamino, morpholino, piperidino, piperazino, or N-phenylpiperazino, or
(3) -COD wherein D is hydrogen, (C₁₋₄)alkoxy, hydroxy, amino, N-lower (C₁₋₄)alkyl amino, N,N-dilower (C₁₋₄)alkylamino, phenylamino, benzylamino, naphthylmethylamino, pyridylamino, pyridylmethylamino, morpholino, piperidino, piperazino, piperidylmethyl, N-(p-fluorophenyl)piperazino, or N-phenylpiperazino; and R⁵ is carboxyl or tetrazolyl;
or a pharmaceutically acceptable salt thereof.

10. A compound according to claim 1, which is a compound of the formula (Ib'): 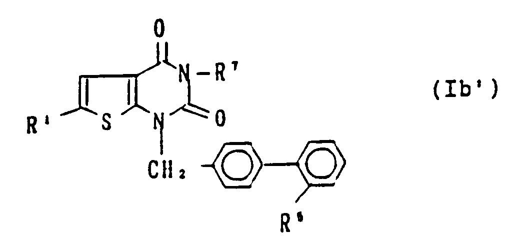 wherein R¹ is (C₁₋₈)alkyl; R⁷ is (C₁₋₈)alkyl which may be optionally substituted with phenyl which may be substituted with halogen, nitro, (C₁₋₄)alkoxy, (C₁₋₄)alkyl at an optional position on the phenyl ring, amino, N-lower (C₁₋₄)alkylamino, N,N-dilower (C₁₋₄)alkylamino, phenylamino, naphthylamino, benzylamino, naphthylmethylamino, morpholino, piperidino, piperazino, N-phenylpiperazino, N-(m-methoxy)phenylpiperazino, or -COD' wherein D' is (C₁₋₄)alkoxy, hydroxy, amino, N-lower (C₁₋₄)alkylamino, N,N-dilower (C₁₋₄)alkylamino, phenylamino, naphthylamino, benzylamino, naphthylmethylamino, morpholino, piperidino, piperazino or N-phenylpiperazino, (C₃₋₈)cycloalkyl, or phenyl or naphthyl which may be substituted with hydroxyl, (C₁₋₄)alkoxy, (C₁₋₄)alkyl, halogen, nitro, amino, N-lower (C₁₋₄)alkylamino, N,N-dilower (C₁₋₄)alkylamino, phenylamino, naphthylamino, benzylamino, naphthylmethylamino, morpholino, piperidino, piperazino, N-phenylpiperazino, N-(m-methoxy)phenylpiperazino, (C₁₋₄)alkanoyloxy, benzoyloxy, phenyl which may be substituted with halogen, nitro, (C₁₋₄)alkoxy, (C₁₋₄)alkyl at an optional position on the phenyl ring, or a group having the formula: -COD' wherein D' is hydroxy, (C₁₋₄)alkoxy, amino, N-lower (C₁₋₄)alkylamino, N,N-dilower (C₁₋₄)alkylamino, phenylamino, naphthylamino, benzylamino, naphthylmethylamino, morpholino, piperidino, piperazino, or N-phenylpiperazino;
and R⁵ is carboxyl or tetrazolyl;
or a pharmaceutically acceptable salt thereof.

11. A compound according to claim 1 or a pharmaceutically acceptable salt thereof, which is selected from the group consisting of ethyl 2-ethyl-7-[[2'-(1H-tetrazol-5-yl)biphenyl-4-yl]methyl]-4-oxo-4,7-dihydrothieno[2,3-b]pyridine-5-carboxylate, 2-ethyl-7-[[2'-(1H-tetrazol-5-yl)biphenyl-4-yl]methyl]-4-oxo-4,7-dihydrothieno[2,3-b]pyridine-5-carboxylic acid, methoxyethyl 2-ethyl-7-[[2'-(1H-tetrazol-5-yl)biphenyl-4-yl]methyl]-4-oxo-4,7-dihydrothieno[2,3-b]pyridine-5-carboxylate, 2-ethyl-5-(N-benzylcarbamoyl)-7-[[2'-(1H-tetrazol-5-yl)biphenyl-4-yl]methyl]-4-oxo-4,7-dihydrothieno[2,3-b]pyridin-4(7H)-one, 2-ethyl-5-(N-phenylcarbamoyl)-7-[[2'-(1H-tetrazol-5-yl)biphenyl-4-yl]methyl]-4-oxo-4,7-dihydrothieno[2,3-b]pyridin-4(7H)-one, and 2-ethyl-5-(N-2-pyridylmethylcarbamoyl)-7-[[2'-(1H-tetrazol-5-yl)biphenyl-4-yl]methyl]-4-oxo-4,7-dihydrothieno[2,3-b]pyridin-4(7H)-one.

12. A compound according to claim 10, wherein R¹ is ethyl, R⁵ is 1H-tetrazol-5-yl and R⁷ is butyl, benzyl, ethyl, p-fluorophenyl, 2,5-dichlorophenyl, cyclohexyl, ethoxycarbonylmethyl, or 2-[4-(o-methoxyphenyl)-piperazino]ethyl.

13. A pharmaceutical composition for antagonizing angiotensin II which comprises a therapeutically effective amount of a compound according to claim 1 or a pharmaceutically acceptable salt thereof in admixture with a pharmaceutical acceptable carrier, excipient or diluent.

14. A use of a compound according to claim 1 or a pharmaceutically acceptable salt thereof for the manufacture of a medicament for antagonizing angiotensin II.

15. A method for producing a compound of the formula (I): 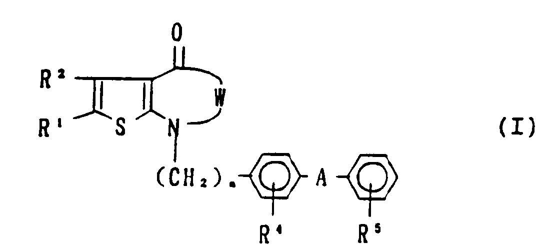 wherein W, R¹, R², R³, R⁴, R⁵, R⁶, R⁷, A and n Claim 1 have the meaning as defined in Claim 1 or a pharmaceutically acceptable salt thereof, which comprises reacting a compound of the formula (II): 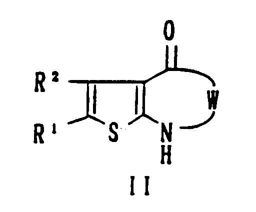 wherein R¹, R², and W have the above-defined meanings, with a compound of the formula (III): 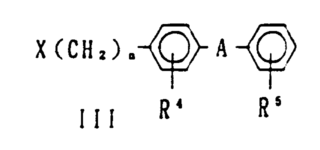 wherein R⁴, R⁵, A and n have the above-defined meanings, and X is halogen,
and, (i) if desired, converting a compound of the formula (I) wherein R⁵ is cyano or protected tetrazolyl, and R¹, R², R⁴, R⁵, A, W and n have the above-defined meanings, into a compound of the formula (I) wherein R⁵ is tetrazolyl and R¹, R², R⁴, R⁵, A, W and n have the above-defined meanings, (ii) if desired, converting a compound of the formula (I) wherein -R³ is lower (C₁₋₄) alkoxycarbonyl or halogenocarbonyl, and R¹, R², R⁴, R⁵, R⁶, A and n have the above-defined meanings, into a compound of the formula (I) wherein R³ is carboxyl, or optionally substituted carbamoyl and R¹, R², R⁴, R⁵, R⁶, A and n have the above-defined meanings, or (iii) if desired, converting a compound of the formula (I) wherein -R³ is carboxyl, and R¹, R², R⁴, R⁵, R⁶, A and n have the above-defined meanings, into a compound of the formula (I) wherein R³ is halogenocarbonyl and R¹, R², R⁴, R⁵, R⁶, A and n have the above-defined meanings, and, if desired, converting a compound of the formula (I) into a pharmaceutically acceptable salt thereof.

## Claims (Claims for the following Contracting State(s): ES, GR)

1. A method for producing a compound of the formula (I): 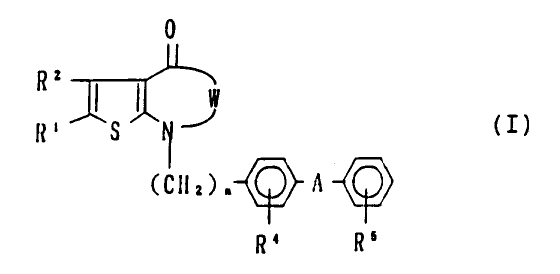 wherein W is 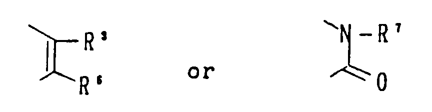 R¹ and R² which may be the same or different, are each independently
(1) hydrogen,
(2) halogen,
(3) cyano,
(4) nitro, (5) a group having the formula: R⁸CONH- wherein R⁸ is
hydrogen or (C₁₋₈)alkyl group which may be substituted with hydroxyl, (C₁₋₄)alkoxy, (C₁₋₄)alkyl, halogen, nitro, amino, methylamino, dimethylamino, phenylamino, benzylamino, morpholino, piperidino, piperazino, N-phenylpiperazino, (C₁₋₄)alkanoyloxy, benzoyloxy, phenyl which may be substituted with halogen, nitro, (C₁₋₄)alkoxy, (C₁₋₄)alkyl at an optional position on the phenyl ring, or naphthyl, or
(6) a hydrocarbon residue selected from the group consisting of (i) (C₁₋₈)alkyl, (ii) (C₂₋₈)alkenyl, (iii) (C₂₋₈)alkynyl, (iv) (C₃₋₈)cycloalkyl, (v) an aromatic hydrocarbon selected from the group consisting of phenyl and naphthyl, wherein said hydrocarbon residue may be substituted with hydroxyl, (C₁₋₄)alkoxy, (C₁₋₄)alkyl, halogen, nitro, amino, N-lower (C₁₋₄)alkylamino, N,N-dilower (C₁₋₄)alkylamino, phenylamino, naphthylamino, benzylamino, naphthylmethylamino, morpholino, piperidino, piperazino, N-phenylpiperazino, N-(m-methoxy)phenylpiperazino, (C₁₋₄)alkanoyloxy, benzoyloxy, phenyl which may be substituted with halogen, nitro, (C₁₋₄)alkoxy, (C₁₋₄)alkyl at an optional position on the phenyl ring, or a group having the formula: -COD' wherein D' is hydroxy, (C₁₋₄)alkoxy, amino, N-lower (C₁₋₄)alkylamino, N,N-dilower (C₁₋₄)alkylamino, phenylamino, naphthylamino, benzylamino, naphthylmethylamino, morpholino, piperidino, piperazino, or N-phenylpiperazino;
R³ is
(1) hydrogen,
(2) (C₁₋₈)alkyl or (C₂₋₈)alkenyl, which may be straight or branched and may be optionally substituted with hydroxyl, amino, N-lower (C₁₋₄)alkyl amino, N,N-dilower (C₁₋₄)alkyl amino, halogen, lower (C₁₋₄)alkoxy, or -COD'' wherein D'' is lower (C₁₋₄)alkoxy, hydroxy, halogen, amino, N-lower (C₁₋₄)alkyl amino, N,N-dilower (C₁₋₄)alkyl amino, phenylamino, naphthylamino, benzylamino, naphthylmethylamino, morpholino, piperidino, piperazino, or N-phenylpiperazino, or
(3) -COD wherein D is hydrogen, C₁₋₄-alkoxy, hydroxy, halogen, amino, N-lower (C₁₋₄)alkyl amino, N,N-dilower (C₁₋₄)alkyl amino, phenylamino, benzylamino, naphthylmethylamino, pyridylamino, pyridylmethylamino, morpholino, piperidino, piperazino, piperidylmethyl, N-(p-fluorophenyl)piperazino, or N-phenylpiperazino, wherein said alkyl, aryl and heteroaryl groups may be optionally substituted with methyl, ethyl, propyl, isopropyl, butyl, isobutyl, sec-butyl, hydroxyl, amino, N-lower (C₁₋₄)alkyl amino, N,N-dilower (C₁₋₄)alkylamino, morpholino, piperidino, piperazino, N-phenylpiperazino, halogen, nitro or lower (C₁₋₄) alkoxy;
R⁴ is hydrogen, halogen or nitro;
R⁵ is carboxyl, lower (C₁₋₄)alkoxycarbonyl, cyano, tetrazolyl, trifluoromethanesulfonic amide, phosphoric acid or sulfonic acid;
R⁶ is hydrogen or (C₁₋₈)alkyl or (C₂₋₈)alkenyl, which may be straight or branched and may be optionally substituted with hydroxyl, amino, N-lower (C₁₋₄)alkyl amino, N,N-dilower (C₁₋₄)alkyl amino, halogen, lower (C₁₋₄)alkoxy, or -COD'' wherein D'' is lower (C₁₋₄)alkoxy, hydroxy, halogen, N-lower (C₁₋₄)alkyl amino, N,N-dilower (C₁₋₄)alkyl amino, phenylamino, naphthylamino, benzylamino, naphthylmethylamino, morpholino, piperidino, piperazino, or N-phenylpiperazino;
R⁷ is a hydrocarbon residue which may be substituted like R¹ and R²; A is a direct bond or a spacer having atomic length of two or less between the phenylene group and the phenyl group selected from the group consisting of (C₁₋₄)alkylene, -C(=O)-, -O-, -S-, -NH-, -C(=O)-NH-, -O-CH₂-, -S-CH₂-, or -CH=CH-; and n is an integer of 1 or 2;
or a pharmaceutically acceptable salt thereof, which comprises
reacting a compound of the formula (II): 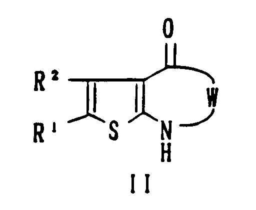 wherein R¹, R², and W have the above-defined meanings, with a compound of the formula (III): 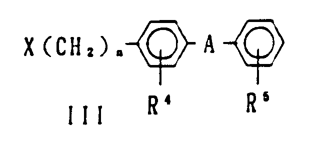 wherein R⁴, R⁵, A and n have the above-defined meanings, and X is halogen,
and, (i) if desired, converting a compound of the formula (I) wherein R⁵ is cyano or protected tetrazolyl, and R¹, R², R⁴, R⁵, A, W and n have the above-defined meanings, into a compound of the formula (I) wherein R⁵ is tetrazolyl and R¹, R², R⁴, R⁵, A, W and n have the above-defined meanings, (ii) if desired, converting a compound of the formula (I) wherein -R³ is lower (C₁₋₄) alkoxycarbonyl or halogenocarbonyl, and R¹, R², R⁴, R⁵, R⁶, A and n have the above-defined meanings, into a compound of the formula (I) wherein R³ is carboxyl, or optionally substituted carbamoyl and R¹, R², R⁴, R⁵, R⁶, A and n have the above-defined meanings, or (iii) if desired, converting a compound of the formula (I) wherein -R³ is carboxyl, and R¹, R², R⁴, R⁵, R⁶, A and n have the above-defined meanings, into a compound of the formula (I) wherein R³ is halogenocarbonyl and R¹, R², R⁴, R⁵, R⁶, A and n have the above-defined meanings, and, if desired, converting a compound of the formula (I) into a pharmaceutically acceptable salt thereof.

2. A method according to claim 1 for producing a compound of the formula (Ia): 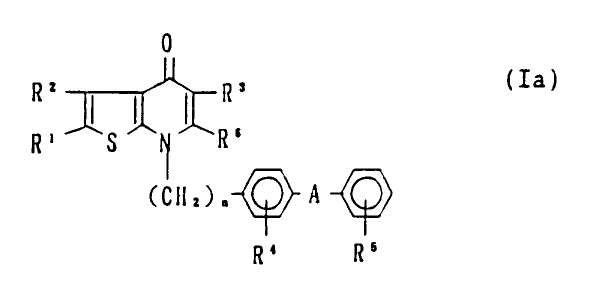 wherein R¹ and R² are as defined in claim 1;
R³ is hydrogen, formyl, optionally substituted alkyl or alkenyl as defined in claim 1, or -COD wherein D is alkoxy, hydroxy, halogen, or optionally substituted amino as defined in claim 1;
R⁴ is hydrogen, halogen or nitro;
R⁵ is as defined in claim 1;
R⁶, A and n are as defined in claim 1
and a pharmaceutically acceptable salt thereof.

3. A method according to claim 1 for producing a compound of the formula (Ib): 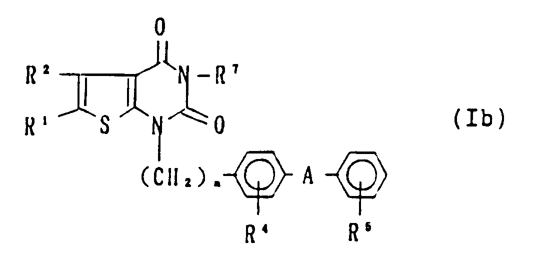 wherein R¹ and R² are as defined in claim 1;
R⁴ is hydrogen, halogen or nitro;
R⁵ is as defined in claim 1;
R⁷, A and n are as defined in claim 1;
and a pharmaceutically acceptable salt thereof.

4. A method according to claim 1, wherein an optionally substituted amino as defined in claim 1 for the substituents of said hydrocarbon residue is amino, methylamino, dimethylamino, phenylamino, benzylamino, morpholino, piperidino, piperazino, N-phenylpiperazino, or N-(m-methoxy)phenylpiperazino.

5. A method according to claim 1, wherein said D' is amino, methylamino, dimethylamino, phenylamino, benzylamino, morpholino, piperidino, piperazino, or N-phenylpiperazino.

6. A method according to claim 1, wherein said D is amino, N- (C₁₋₄) alkyl amino, N,N-di (C₁₋₄)alkyl amino, phenylamino, benzylamino, naphthylmethylamino, pyridylamino, pyridylmethylamino, morpholino, piperidino, piperazino, piperidylmethyl, N-phenylpiperazino, or N-(p-fluorophenyl)piperazino wherein said alkyl, aryl and heteroaryl groups may be optionally substituted with methyl, ethyl, propyl, isopropyl, butyl, isobutyl, sec-butyl, hydroxyl, amino, N-lower (C₁₋₄)alkyl amino, N,N-dilower (C₁₋₄)alkylamino, morpholino, piperidino, piperazino, N-phenylpiperazino, halogen, nitro or lower (C₁₋₄)alkoxy.

7. A method according to claim 1 , wherein said R⁵ is tetrazolyl.

8. A method according to claim 1 , wherein said R⁵ is in the ortho position.

9. A method according to claim 1 for producing a compound of the formula (Ia'): 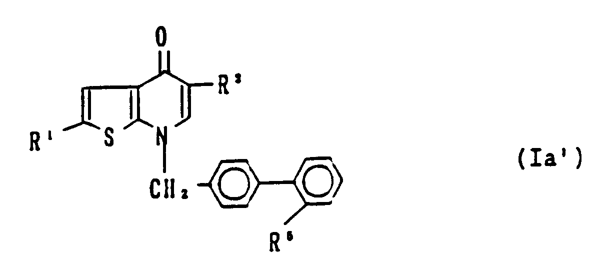 wherein R¹ is (C₁₋₈)alkyl; R³ is
(1) hydrogen,
(2) (C₁₋₈)alkyl which may be straight or branched and may be optionally substituted with hydroxyl, amino, N-lower (C₁₋₄)alkylamino, N,N-dilower (C₁₋₄)alkylamino, halogen, (C₁₋₄)alkoxy or -COD'' wherein D'' is (C₁₋₄)alkoxy, hydroxy, halogen, amino, N-lower (C₁₋₄)alkyl amino, N,N-dilower (C₁₋₄)alkylamino, phenylamino, naphthylamino, benzylamino, naphthylmethylamino, morpholino, piperidino, piperazino, or N-phenylpiperazino, or
(3) -COD wherein D is hydrogen, (C₁₋₄)alkoxy, hydroxy, amino, N-lower (C₁₋₄)alkyl amino, N,N-dilower (C₁₋₄)alkylamino, phenylamino, benzylamino, naphthylmethylamino, pyridylamino, pyridylmethylamino, morpholino, piperidino, piperazino, piperidylmethyl, N-(p-fluorophenyl)piperazino, or N-phenylpiperazino; and R⁵ is carboxyl or tetrazolyl; or a pharmaceutically acceptable salt thereof.

10. A method according to claim 1 for producing a compound of the formula (Ib'): 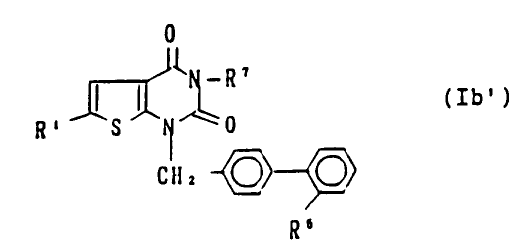 wherein R¹ is (C₁₋₈)alkyl; R⁷ is (C₁₋₈)alkyl which may be optionally substituted with phenyl which may be substituted with halogen, nitro, (C₁₋₄)alkoxy, (C₁₋₄)alkyl at an optional position on the phenyl ring, amino, N-lower (C₁₋₄)alkylamino, N,N-dilower (C₁₋₄)alkylamino, phenylamino, naphthylamino, benzylamino, naphthylmethylamino, morpholino, piperidino, piperazino, N-phenylpiperazino, N-(m-methoxy)phenylpiperazino, or -COD' wherein D' is (C₁₋₄)alkoxy, hydroxy, amino, N-lower (C₁₋₄)alkylamino, N,N-dilower (C₁₋₄)alkylamino, phenylamino, naphthylamino, benzylamino, naphthylmethylamino, morpholino, piperidino, piperazino or N-phenylpiperazino, (C₃₋₈)cycloalkyl, or phenyl or naphthyl which may be substituted with hydroxyl, (C₁₋₄)alkoxy, (C₁₋₄)alkyl, halogen, nitro, amino, N-lower (C₁₋₄)alkylamino, N,N-dilower (C₁₋₄)alkylamino, phenylamino, naphthylamino, benzylamino, naphthylmethylamino, morpholino, piperidino, piperazino, N-phenylpiperazino, N-(m-methoxy)phenylpiperazino, (C₁₋₄)alkanoyloxy, benzoyloxy, phenyl which may be substituted with halogen, nitro, (C₁₋₄)alkoxy, (C₁₋₄)alkyl at an optional position on the phenyl ring, or a group having the formula: -COD' wherein D' is hydroxy, (C₁₋₄)alkoxy, amino, N-lower (C₁₋₄)alkylamino, N,N-dilower (C₁₋₄)alkylamino, phenylamino, naphthylamino, benzylamino, naphthylmethylamino, morpholino, piperidino, piperazino, or N-phenylpiperazino; and R⁵ is carboxyl or tetrazolyl; or a pharmaceutically acceptable salt thereof.

11. A method according to claim 1 for producing a compound which is selected from the group consisting of ethyl 2-ethyl-7-[[2'-(1H-tetrazol-5-yl)biphenyl-4-yl]methyl]-4-oxo-4,7-dihydrothieno[2,3-b]pyridine-5-carboxylate, 2-ethyl-7-[[2'-(1H-tetrazol-5-yl)biphenyl-4-yl]methyl]-4-oxo-4,7-dihydrothieno[2,3-b]pyridine-5-carboxylic acid, methoxyethyl 2-ethyl-7-[[2'-(1H-tetrazol-5-yl)biphenyl-4-yl]methyl]-4-oxo-4,7-dihydrothieno[2,3-b]pyridine-5-carboxylate, 2-ethyl-5-(N-benzylcarbamoyl)-7-[[2'-(1H-tetrazol-5-yl)biphenyl-4-yl]methyl]-4-oxo-4,7-dihydrothieno[2,3-b]pyridin-4(7H)-one, 2-ethyl-5-(N-phenylcarbamoyl)-7-[[2'-(1H-tetrazol-5-yl)biphenyl-4-yl]methyl]-4-oxo-4,7-dihydrothieno[2,3-b]pyridin-4(7H)-one, and 2-ethyl-5-(N-2-pyridylmethylcarbamoyl)-7-[[2'-(1H-tetrazol-5-yl)biphenyl-4-yl]methyl]-4-oxo-4,7-dihydrothieno[2,3-b]pyridin-4(7H)-one or a pharmaceutically acceptable salt thereof.

12. A method according to claim 10, wherein R¹ is ethyl, R⁵ is 1H-tetrazol-5-yl and R⁷ is butyl, benzyl, ethyl, p-fluorophenyl, 2,5-dichlorophenyl, cyclohexyl, ethoxycarbonylmethyl, or 2-[4-(o-methoxyphenyl)-piperazino]ethyl.

13. A method for producing a pharmaceutical composition for antagonizing angiotensin II which comprises admixing a therapeutically effective amount of a compound according to claim 1 or a pharmaceutically acceptable salt thereof with a pharmaceutical acceptable carrier, excipient or diluent.

14. A use of a compound according to claim 1 or a pharmaceutically acceptable salt thereof for the manufacture of a medicament for antagonizing angiotensin II.

## Patentansprüche (Patentansprüche für folgende(n) Vertragsstaat(en): AT, BE, CH, DE, DK, FR, GB, IT, LI, LU, NL, SE)

1. Verbindung der Formel 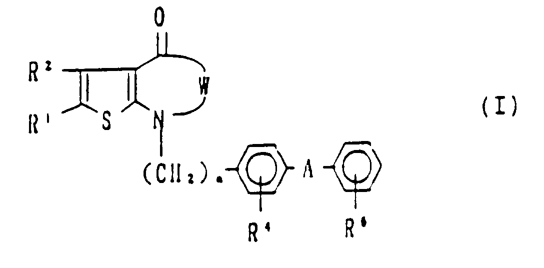 worin W 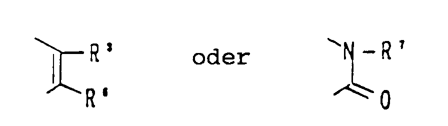 ist,
R¹ und R², die gleich oder verschieden sein können, jeweils unabhängig
(1) Wasserstoff,
(2) Halogen,
(3) Cyanoreste,
(4) Nitroreste,
(5) Gruppen mit der Formel R⁸CONH-, worin R⁸ Wasserstoff oder eine (C₁-C₈)-Alkylgruppe ist, die mit Hydroxyl-, (C₁-C₄)-Alkoxy-, (C₁-C₄)-Alkyl-, Halogen-, Nitro-, Amino-, Methylamino-, Dimethylamino-, Phenylamino-, Benzylamino-, Morpholino-, Piperidino-, Piperazino-, N-Phenylpiperazino-, (C₁-C₄)-Alkanoyloxy-, Benzoyloxy-, Phenylresten, die mit Halogen-, Nitro-, (C₁-C₄)-Alkoxy-, (C₁-C₄)-Alkylresten an freigewählter Position am Phenylring substituiert sein können, oder Naphthylresten substituiert sein kann, oder
(6) Kohlenwasserstoffreste sind, ausgewählt aus der Gruppe bestehend aus (i) (C₁-C₈)-Alkylresten, (ii) (C₂-C₈)-Alkenylresten, (iii) (C₂-C₈)-Alkinylresten, (iv) (C₃-C₈)-Cycloalkylresten, (v) einem aromatischen Kohlenwasserstoffrest ausgewählt aus der Gruppe bestehend aus Phenyl- und Naphthylresten, wobei der Kohlenwasserstoffrest mit Hydroxyl-, (C₁-C₄)-Alkoxy-, (C₁-C₄)-Alkyl-, Halogen-, Nitro-, Amino-, N-Niedrig(C₁-C₄)alkylamino-, N,N-Di-niedrig(C₁-C₄)alkylamino-, Phenylamino-, Naphthylamino-, Benzylamino-, Naphthylmethylamino-, Morpholino-, Piperidino-, Piperazino-, N-Phenylpiperazino-, N-(m-Methoxy)phenylpiperazino-, (C₁-C₄)-Alkanoyloxy-, Benzoyloxy-, Phenylresten, die mit Halogen-, Nitro-, (C₁-C₄)-Alkoxy-, (C₁-C₄)-Alkylresten an freigewählter Position am Phenylring substituiert sein können, oder einer Gruppe der Formel -COD' substituiert sein kann, worin D' ein Hydroxy-, (C₁-C₄)-Alkoxy-, Amino-, N-Niedrig(C₁-C₄)alkylamino-, N,N-Di-niedrig(C₁-C₄)alkylamino-, Phenylamino-, Naphthylamino-, Benzylamino-, Naphthylmethylamino-, Morpholino-, Piperidino-, Piperazino- oder N-Phenylpiperazinorest ist;
R³
(1) Wasserstoff,
(2) ein (C₁-C₈)-Alkyl- oder (C₂-C₈)-Alkenylrest, der geradkettig oder verzweigt sein kann und gegebenenfalls mit Hydroxyl-, Amino-, N-Niedrig(C₁-C₄)alkylamino-, N,N-Di-niedrig(C₁-C₄)alkylamino-, Halogen-, Niedrig(C₁-C₄)alkoxyresten oder -COD'' substituiert sein kann, worin D'' ein Niedrig(C₁-C₄)-alkoxy-, Hydroxy-, Halogen-, Amino-, N-Niedrig(C₁-C₄)alkylamino-, N,N-Di-niedrig(C₁-C₄)alkylamino-, Phenylamino-, Naphthylamino-, Benzylamino-, Naphthylmethylamino-, Morpholino-, Piperidino-, Piperazino- oder N-Phenylpiperazinorest ist oder
(3) -COD ist, worin D Wasserstoff, ein (C₁-C₄)-Alkoxy-` Hydroxy-, Halogen-, Amino-, N-Niedrig(C₁-C₄)alkylamino-, N,N-Di-niedrig(C₁-C₄)alkylamino-, Phenylamino-, Benzylamino-, Naphthylmethylamino-, Pyridylamino-, Pyridylmethylamino-, Morpholino-, Piperidino-, Piperazino-, Piperidylmethyl-, N-(p-Fluorphenyl)piperazino- oder N-Phenylpiperazinorest ist, worin die Alkyl-, Aryl- und Heteroarylgruppen gegebenenfalls mit Methyl-, Ethyl-, Propyl-, Isopropyl-, Butyl-, Isobutyl-, sek.-Butyl-, Hydroxyl-, Amino-, N-Niedrig(C₁-C₄)alkylamino-, N,N-Di-niedrig(C₁-C₄)-alkylamino-, Morpholino-, Piperidino-, Piperazino-, N-Phenylpiperazino-, Halogen-, Nitro- oder Niedrig(C₁-C₄)alkoxyresten substituiert sein können;
R⁴ Wasserstoff, Halogen oder ein Nitrorest ist;
R⁵ ein Carboxyl-, Niedrig(C₁-C₄)alkoxycarbonyl-, Cyano-, Tetrazolyl-, Trifluormethansulfonsäureamid-, Phosphorsäure- oder Sulfonsäurerest ist;
R⁶ Wasserstoff oder ein (C₁-C₈)-Alkyl- oder (C₂-C₈)-Alkenylrest ist, der geradkettig oder verzweigt sein kann und gegebenenfalls mit Hydroxyl-, Amino-, N-Niedrig(C₁-C₄)alkylamino-, N,N-Di-niedrig(C₁-C₄)alkylamino-, Halogen-, Niedrig(C₁-C₄)alkoxyresten oder -COD'' substituiert sein kann, worin D'' ein Niedrig(C₁-C₄)alkoxy-, Hydroxy-, Halogen-, N-Niedrig(C₁-C₄)alkylamino-, N,N-Di-niedrig(C₁-C₄)alkylamino-, Phenylamino-, Naphthylamino-, Benzylamino-, Naphthylmethylamino-, Morpholino-, Piperidino-, Piperazino- oder N-Phenylpiperazinorest ist;
R⁷ ein Kohlenwasserstoffrest ist, der wie R¹ und R² substituiert sein kann; A eine direkte Bindung oder ein Abstandshalter mit einer Atomlänge von 2 oder weniger zwischen der Phenylengruppe und der Phenylgruppe ist ausgewählt aus der Gruppe bestehend aus einem (C₁-C₄)-Alkylenrest, -C(=O)-, -O-, -S-, -NH-, -C(=O)-NH-, -O-CH₂-, -S-CH₂- oder -CH=CH- und n eine ganze Zahl von 1 oder 2 ist und
ein pharmazeutisch annehmbares salz davon.

2. Verbindung nach Anspruch 1, die eine Verbindung der Formel (Ia) ist: 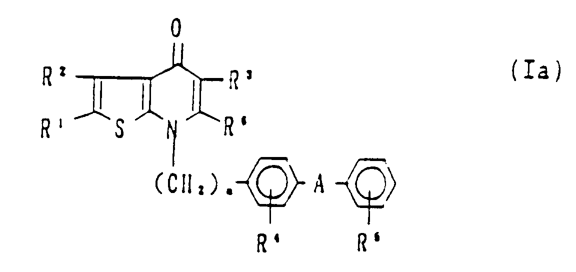 worin R¹ und R² wie in Anspruch 1 definiert sind;
R³ Wasserstoff, ein Formylrest, ein gegebenenfalls substituierter Alkyl- oder Alkenylrest, wie in Anspruch 1 definiert, ist oder -COD ist, worin D ein Alkoxy-, Hydroxy-, Halogen- oder gegebenenfalls substituierter Aminorest, wie in Anspruch 1 definiert, ist;
R⁴ Wasserstoff, Halogen oder ein Nitrorest ist;
R⁵ wie in Anspruch 1 definiert ist;
R⁶, A und n wie in Anspruch 1 definiert sind und ein pharmazeutisch annehmbares Salz davon.

3. Verbindung nach Anspruch 1, die eine Verbindung der Formel (Ib) ist: 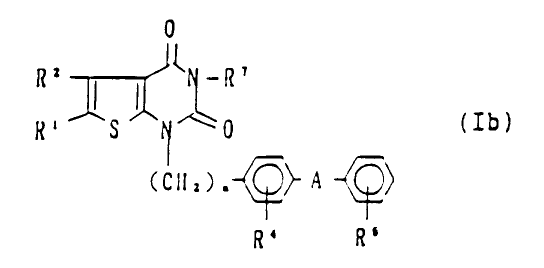 worin R¹ und R² wie in Anspruch 1 definiert sind;
R⁴ Wasserstoff, Halogen oder ein Nitrorest ist;
R⁵ wie in Anspruch 1 definiert ist;
R⁷, A und n wie in Anspruch 1 definiert sind und
ein pharmazeutisch annehmbares Salz davon.

4. Verbindung nach Anspruch 1, worin ein wie für die Substituenten des Kohlenwasserstoffrestes in Anspruch 1 definierter gegebenenfalls substuierter Aminorest ein Amino-, Methylamino-, Dimethylamino-, Phenylamino-, Benzylamino-, Morpholino-, Piperidino-, Piperazino-, N-Phenylpiperazino- oder N-(m-Methoxy)-phenylpiperazinorest ist.

5. Verbindung nach Anspruch 1, worin D' ein Amino-, Methylamino-, Dimethylamino-, Phenylamino-, Benzylamino-, Morpholino-, Piperidino-, Piperazino- oder N-Phenylpiperazinorest ist.

6. Verbindung nach Anspruch 1, worin D ein Amino-, N-(C₁-C₄)Alkylamino-, N,N-Di(C₁-C₄)alkylamino-, Phenylamino-, Benzylamino-, Naphthylmethylamino-, Pyridylamino-, Pyridylmethylamino-, Morpholino-, Piperidino-, Piperazino-, Piperidylmethyl-, N-Phenylpiperazino- oder N-(p-Fluorphenyl)piperazinorest ist, worin die Alkyl-, Aryl- und Heteroarylgruppen gegebenenfalls mit Methyl-, Ethyl-, Propyl-, Isopropyl-, Butyl-, Isobutyl-, sek.-Butyl-, Hydroxyl-, Amino-, N-Niedrig(C₁-C₄)alkylamino-, N,N-Di-niedrig(C₁-C₄)alkylamino-, Morpholino-, Piperidino-, Piperazino-, N-Phenylpiperazino-, Halogen-, Nitro- oder Niedrig(C₁-C₄)alkoxyresten substituiert sein können.

7. Verbindung nach Anspruch 1, worin R⁵ ein Tetrazolylrest ist.

8. Verbindung nach Anspruch 1, worin R⁵ in ortho-Position ist.

9. Verbindung nach Anspruch 1, die eine Verbindung der Formel (Ia') ist: 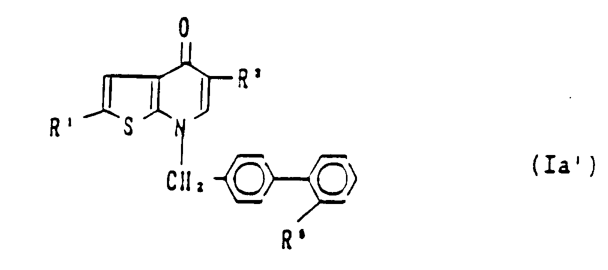 worin R¹ ein (C₁-C₈)-Alkylrest ist;
R³
(1) Wasserstoff,
(2) ein (C₁-C₈)-Alkylrest ist, der geradkettig oder verzweigt sein kann und gegebenenfalls mit Hydroxyl-, Amino-, N-Niedrig(C₁-C₄)alkylamino-, N,N-Di-niedrig-(C₁-C₄)alkylamino-, Halogen-, (C₁-C₄)-Alkoxyresten oder -COD'' substituiert sein kann, worin D'' ein (C₁-C₄)-Alkoxy-, Hydroxy-, Halogen-, Amino-, N-Niedrig(C₁-C₄)alkylamino-, N,N-Di-niedrig(C₁-C₄)alkylamino-, Phenylamino-, Naphthylamino-, Benzylamino-, Naphthylmethylamino-, Morpholino-, Piperidino-, Piperazino- oder N-Phenylpiperazinorest ist oder
(3) -COD ist, worin D Wasserstoff, ein (C₁-C₄)-Alkoxy-, Hydroxy-, Amino-, N-Niedrig(C₁-C₄)alkylamino-, N,N-Di-niedrig(C₁-C₄)alkylamino-, Phenylamino-, Benzylamino-, Naphthylmethylamino-, Pyridylamino-, Pyridylmethylamino-, Morpholino-, Piperidino-, Piperazino-, Piperidylmethyl-, N-(p-Fluorphenyl)piperazino- oder N-Phenylpiperazinorest ist und R⁵ ein Carboxyl- oder Tetrazolylrest ist oder
ein pharmazeutisch annehmbares Salz davon.

10. Verbindung nach Anspruch 1, die eine Verbindung der Formel (Ib') ist: 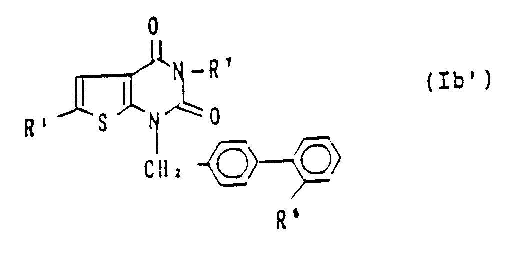 worin R¹ ein (C₁-C₈)-Alkylrest ist; R⁷ ein (C₁-C₈)-Alkylrest ist, der gegebenenfalls mit Phenylresten, die mit Halogen-, Nitro-, (C₁-C₄)-Alkoxy-, (C₁-C₄)-Alkylresten an freigewählter Position am Phenylring, Amino-, N-Niedrig(C₁-C₄)alkylamino-, N,N-Diniedrig(C₁-C₄)alkylamino-, Phenylamino-, Naphthylamino-, Benzylamino-, Naphthylmethylamino-, Morpholino-, Piperidino-, Piperazino-, N-Phenylpiperazino-, N-(m-Methoxy)phenylpiperazinoresten oder -COD' substituiert sein kann, worin D' ein (C₁-C₄)-Alkoxy-, Hydroxy-, Amino-, N-Niedrig(C₁-C₄)alkylamino-, N,N-Diniedrig(C₁-C₄)alkylamino-, Phenylamino-, Naphthylamino-, Benzylamino-, Naphthylmethylamino-, Morpholino-, Piperidino-, Piperazino- oder N-Phenylpiperazino-, (C₃-C₈)-Cycloalkyl- oder Phenyl- oder Naphthylrest ist, der mit Hydroxyl-, (C₁-C₄)-Alkoxy-, (C₁-C₄)-Alkyl-, Halogen-, Nitro-, Amino-, N-Niedrig(C₁-C4)alkylamino-, N,N-Di-niedrig(C₁-C₄)-alkylamino-, Phenylamino-, Naphthylamino-, Benzylamino-, Naphthylmethylamino-, Morpholino-, Piperidino-, Piperazino-, N-Phenylpiperazino-, N-(m-Methoxy)-phenylpiperazino-, (C₁-C₄)-Alkanoyloxy-, Benzoyloxy-, Phenylresten, die mit Halogen-, Nitro-, (C₁-C₄)-Alkoxy-, (C₁-C₄)-Alkylresten an freigewählter Position des Phenylrings substituiert sein können, oder einer Gruppe der Formel -COD' substituiert sein kann, worin D' ein Hydroxy-, (C₁-C₄)-Alkoxy-, Amino-, N-Niedrig(C₁-C₄)alkylamino-, N,N-Di-niedrig(C₁-C₄)alkylamino-, Phenylamino-, Naphthylamino-, Benzylamino-, Naphthylmethylamino-, Morpholino-, Piperidino-, Piperazino- oder N-Phenylpiperazinorest ist; und R⁵ ein Carboxyl- oder Tetrazolylrest ist oder ein pharmazeutisch annehmbares Salz davon.

11. Verbindung nach Anspruch 1 oder pharmazeutisch annehmbares Salz davon, die ausgewählt ist aus der Gruppe bestehend aus Ethyl-2-ethyl-7-[[2'-(1H-tetrazol-5-yl)biphenyl-4-yl]methyl]-4-oxo-4,7-Dihydrothieno[2,3-b]pyridin-5-carboxylat, 2-Ethyl-7-[[2'-(1H-tetrazol-5-yl)biphenyl-4-yl]methyl]-4-oxo-4,7-Dihydrothieno[2,3-b]pyridin-5-carbonsäure, Methoxyethyl-2-ethyl-7-[[2'-(1H-tetrazol-5-yl)biphenyl-4-yl]methyl]-4-oxo-4,7-Dihydrothieno[2,3-b]pyridin-5-carboxylat, 2-Ethyl-5-(N-benzylcarbamoyl)-7-[[2'-(1H-tetrazol-5-yl)biphenyl-4-yl]methyl]-4-oxo-4,7-Dihydrothieno[2,3-b]pyridin-4(7H)-on, 2-Ethyl-5-(N-phenylcarbamoyl)-7-[[2'-(1H-tetrazol-5-yl)biphenyl-4-yl]methyl]-4-oxo-4,7-Dihydrothieno[2,3-b]pyridin-4(7H)-on und 2-Ethyl-5-(N-2-pyridylmethylcarbamoyl)-7-[[2'-(1H-tetrazol-5-yl)biphenyl-4-yl]methyl]-4-oxo-4,7-Dihydrothieno[2,3-b]pyridin-4(7H)-on.

12. Verbindung nach Anspruch 10, worin R¹ ein Ethylrest ist, R⁵ ein 1H-Tetrazol-5-yl-rest ist und R⁷ ein Butyl-, Benzyl-, Ethyl-, p-Fluorphenyl-, 2,5-Dichlorphenyl-, Cyclohexyl-, Ethoxycarbonylmethyl- oder 2-[4-(o-Methoxyphenyl)-piperazino]ethylrest ist.

13. Pharmazeutische Zusammensetzung zur Antagonisierung von Angiotensin-II umfassend eine therapeutisch wirksame Menge einer Verbindung nach Anspruch 1 oder ein pharmazeutisch annehmbares Salz davon in Mischung mit einem pharmazeutisch annehmbaren Träger, Hilfsstoff oder Verdünnungsmittel.

14. Verwendung einer Verbindung nach Anspruch 1 oder eines pharmazeutisch annehmbaren Salzes davon zur Herstellung eines Arzneimittels, um Angiotensin-II zu antagonisieren.

15. Verfahren zur Herstellung einer Verbindung der Formel (I): 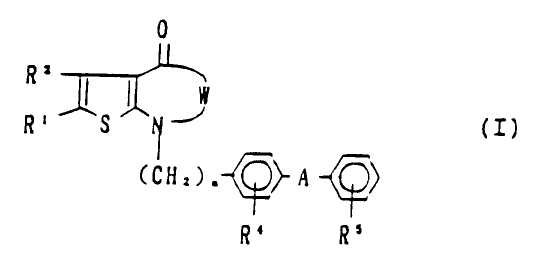 worin W, R¹, R², R³, R⁴, R⁵, R⁶, R⁷, A und n die in Anspruch 1 definierten Bedeutungen haben oder eines pharmazeutisch annehmbaren Salzes davon, umfassend, daß man eine Verbindung der Formel (II): 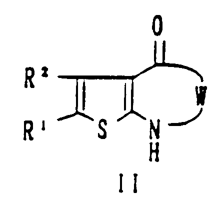 worin R¹, R² und W die oben angegebenen Bedeutungen haben mit einer Verbindung der Formel (III): 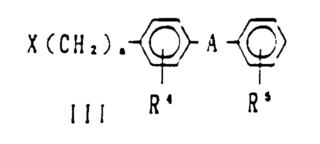 worin R⁴, R⁵, A und n die oben angegebenen Bedeutungen haben und X Halogen ist, umsetzt und (i) falls erwünscht, eine Verbindung der Formel (I) worin R⁵ ein Cyanorest oder ein geschützter Tetrazolylrest ist und R¹, R², R⁴, R⁵, A, W und n die oben angegebenen Bedeutungen haben, in eine Verbindung der Formel (I) umwandelt, worin R⁵ ein Tetrazolylrest ist und R¹, R², R⁴, R⁵, A, W und n die oben angegebenen Bedeutungen haben, (ii) falls erwünscht, eine Verbindung der Formel (I), worin -R³ ein Niedrig(C₁-C₄)alkylcarbonyl- oder Halogencarbonylrest ist und R¹, R², R⁴, R⁵, R⁶, A und n die oben angegebenen Bedeutungen haben, in eine Verbindung der Formel (I) umwandelt, worin R³ ein Carboxylrest oder ein gegebenenfalls substituierter Carbamoylrest ist und R¹, R², R⁴, R⁵, R⁶, A und n die oben angegebenen Bedeutungen haben oder (iii) falls erwünscht, eine Verbindung der Formel (I), worin -R³ ein Carboxylrest ist und R¹, R², R⁴, R⁵, R⁶, A und n die oben angegebenen Bedeutungen haben, in eine Verbindung der Formel (I) umwandelt, worin R³ ein Halogencarbonylrest ist und R¹, R², R⁴, R⁵, R⁶, A und n die oben angegebenen Bedeutungen haben und, falls erwünscht, eine Verbindung der Formel (I) in ein pharmazeutisch annehmbares Salz davon umwandelt.

## Patentansprüche (Patentansprüche für folgende(n) Vertragsstaat(en): ES, GR)

1. Verfahren zur Herstellung einer Verbindung der Formel (I): 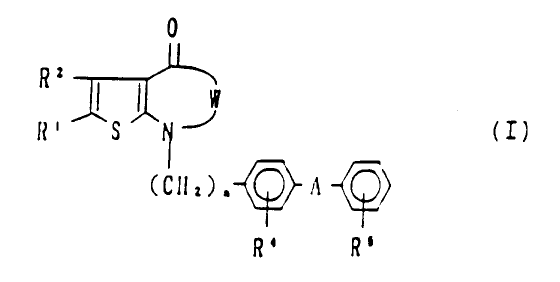 worin W 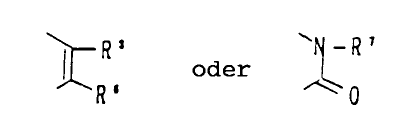 ist,
R¹ und R², die gleich oder verschieden sein können, jeweils unabhängig
(1) Wasserstoff,
(2) Halogen,
(3) Cyanoreste,
(4) Nitroreste,
(5) Gruppen der Formel R⁸CONH-, worin R⁸ Wasserstoff oder eine (C₁-C₈)-Alkylgruppe ist, die mit Hydroxyl-, (C₁-C₄)-Alkoxy-, (C₁-C₄)-Alkyl-, Halogen-, Nitro-, Amino-, Methylamino-, Dimethylamino-, Phenylamino-, Benzylamino-, Morpholino-, Piperidino-, Piperazino-, N-Phenylpiperazino-, (C₁-C₄)-Alkanoyloxy-, Benzoyloxy-, Phenylresten, die mit Halogen-, Nitro-, (C₁-C₄)-Alkoxy-, (C₁-C₄)-Alkylresten an freigewählter Position an dem Phenylring substituiert sein können, oder Naphthylresten substituiert sein kann oder
(6) Kohlenwasserstoffreste sind, ausgewählt aus der Gruppe bestehend aus (i) (C₁-C₈)-Alkylresten, (ii) (C₂-C₈)-Alkenylresten, (iii) (C₂-C₈)Alkinylresten, (iv) (C₃-C₈)-Cycloalkylresten, (v) einem aromatischen Kohlenwasserstoff ausgewählt aus der Gruppe bestehend aus Phenyl- und Naphthylresten, wobei der Kohlenwasserstoffrest mit Hydroxyl-, (C₁-C₄)-Alkoxy-, (C₁-C₄)-Alkyl-, Halogen-, Nitro-, Amino-, N-Niedrig(C₁-C₄)alkylamino-, N,N-Di-niedrig(C₁-C₄)-alkylamino-, Phenylamino-, Naphthylamino-, Benzylamino-, Naphthylmethylamino-, Morpholino-, Piperidino-, Piperazino-, N-Phenylpiperazino-, N-(m-Methoxy)phenylpiperazino-, (C₁-C₄)-Alkanoyloxy-, Benzoyloxy-, Phenylresten, die mit Halogen-, Nitro-, (C₁-C₄)-Alkoxy-, (C₁-C₄)-Alkylresten an freigewählter Position am Phenylring substituiert sein können, oder einer Gruppe der Formel -COD' substituiert sein kann, worin D' ein Hydroxy-, (C₁-C₄)-Alkoxy-, Amino-, N-Niedrig(C₁-C₄)alkylamino-, N,N-Di-niedrig(C₁-C₄)alkylamino-, Phenylamino-, Naphthylamino-, Benzylamino-, Naphthylmethylamino-, Morpholino-, Piperidino-, Piperazino- oder N-Phenylpiperazinorest ist;
R³
(1) Wasserstoff,
(2) ein (C₁-C₈)-Alkyl- oder (C₂-C₈)-Alkenylrest, der geradkettig oder verzweigt sein kann und gegebenenfalls mit Hydroxyl-, Amino-, N-Niedrig(C₁-C₄)alkylamino-, N,N-Di-niedrig(C₁-C₄)alkylamino-, Halogen-, Niedrig(C₁-C₄)alkoxyresten oder -COD'' substituiert sein kann, worin D'' ein Niedrig(C₁-C₄)alkoxy-, Hydroxy-, Halogen-, Amino-, N-Niedrig(C₁-C₄)alkylamino-, N,N-Di-niedrig(C₁-C₄)alkylamino-, Phenylamino-, Naphthylamino-, Benzylamino-, Naphthylmethylamino-, Morpholino-, Piperidino-, Piperazino- oder N-Phenylpiperazinorest ist oder
(3) -COD ist, worin D Wasserstoff, ein (C₁-C₄)-Alkoxy-, Hydroxy-, Halogen-, Amino-, N-Niedrig(C₁-C₄)alkylamino-, N,N-Di-niedrig(C₁-C₄)alkylamino-, Phenylamino-, Benzylamino-, Naphthylmethylamino-, Pyridylamino-, Pyridylmethylamino-, Morpholino-, Piperidino-, Piperazino-, Piperidylmethyl-, N-(p-Fluorphenyl)piperazino- oder N-Phenylpiperazinorest ist, worin die Alkyl-, Aryl- und Heteroarylgruppen gegebenenfalls mit Methyl-, Ethyl-, Propyl-, Isopropyl-, Butyl-, Isobutyl-, sek.-Butyl-, Hydroxyl-, Amino-, N-Niedrig(C₁-C₄)alkylamino-, N,N-Di-niedrig(C₁-C₄)-alkylamino-, Morpholino-, Piperidino-, Piperazino-, n-Phenylpiperazino-, Halogen-, Nitro- oder Niedrig-(C₁-C₄)alkoxyresten substituiert sein können;
R⁴ Wasserstoff, Halogen oder ein Nitrorest ist;
R⁵ ein Carboxyl-, Niedrig(C₁-C₄)alkoxycarbonyl-, Cyano-, Tetrazolyl-, Trifluormethansulfonsäureamid-, Phosphorsäure- oder Sulfonsäurerest ist;
R⁶ Wasserstoff oder ein (C₁-C₈)-Alkyl- oder (C₂-C₈)-Alkenylrest ist, der geradkettig oder verzweigt sein kann und gegebenenfalls mit Hydroxyl-, Amino-, N-Niedrig(C₁-C₄)alkylamino-, N,N-Di-niedrig(C₁-C₄)alkylamino-, Halogen-, Niedrig(C₁-C₄)alkoxyresten oder -COD'' substituiert sein kann, worin D'' ein Niedrig(C₁-C₄)alkoxy-, Hydroxy-, Halogen-, N-Niedrig(C₁-C₄)alkylamino-, N,N-Di-niedrig(C₁-C₄)alkylamino-, Phenylamino-, Naphthylamino-, Benzylamino-, Naphthylmethylamino-, Morpholino-, Piperidino-, Piperazino- oder N-Phenylpiperazinorest ist;
R⁷ ein Kohlenwasserstoffrest ist, der wie R¹ und R² substituiert sein kann; A eine direkte Bindung oder ein Abstandshalter mit einer Atomlänge von 2 oder weniger zwischen der Phenylengruppe und der Phenylgruppe ist ausgewählt aus der Gruppe bestehend aus einem (C₁-C₄)-Alkylenrest, -C(=O)-, -O-, -S-, -NH-, -C(=O)-NH-, -O-CH₂-, -S-CH₂- oder -CH=CH- und n eine ganze Zahl von 1 oder 2 ist
und eines pharmazeutisch annehmbaren Salzes davon umfassend, daß man eine Verbindung der Formel (II): 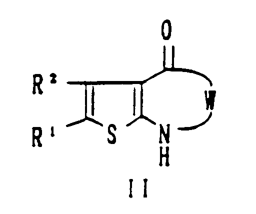 worin R¹, R² und W die oben angegebenen Bedeutungen haben mit einer Verbindung der Formel (III): 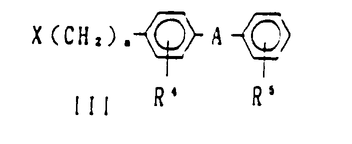 worin R⁴, R⁵, A und n die oben angegebenen Bedeutungen haben und X Halogen ist, umsetzt und (i) falls erwünscht, eine Verbindung der Formel (I) worin R⁵ ein Cyanorest oder ein geschützter Tetrazolylrest ist und R¹, R², R⁴, R⁵, A, W und n die oben angegebenen Bedeutungen haben, in eine Verbindung der Formel (I) umwandelt, worin R⁵ ein Tetrazolylrest ist und R¹, R², R⁴, R⁵, A, W und n die oben angegebenen Bedeutungen haben, (ii) falls erwünscht, eine Verbindung der Formel (I), worin -R³ ein Niedrig(C₁-C₄)alkylcarbonyl- oder Halogencarbonylrest ist und R¹, R², R⁴, R⁵, R⁶, A und n die oben angegebenen Bedeutungen haben, in eine Verbindung der Formel (I) umwandelt, worin R³ ein Carboxylrest oder ein gegebenenfalls substituierter Carbamoylrest ist und R¹, R², R⁴, R⁵, R⁶, A und n die oben angegebenen Bedeutungen haben oder (iii) falls erwünscht, eine Verbindung der Formel (I), worin -R³ ein Carboxylrest ist und R¹, R², R⁴, R⁵, R⁶, A und n die oben angegebenen Bedeutungen haben, in eine Verbindung der Formel (I) umwandelt, worin R³ ein Halogencarbonylrest ist und R¹, R², R⁴, R⁵, R⁶, A und n die oben angegebenen Bedeutungen haben und, falls erwünscht, eine Verbindung der Formel (I) in ein pharmazeutisch annehmbares Salz davon umwandelt.

2. Verfahren nach Anspruch 1 zur Herstellung einer Verbindung der Formel (Ia): 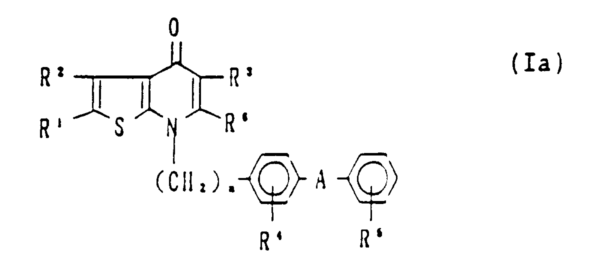 worin R¹ und R² wie in Anspruch 1 definiert sind;
R³ Wasserstoff, ein Formylrest, ein gegebenenfalls substituierter Alkyl- oder Alkenylrest, wie in Anspruch 1 definiert, ist oder -COD ist, worin D ein Alkoxy-, Hydroxy-, Halogen- oder gegebenenfalls substituierter Aminorest ist, wie in Anspruch 1 definiert;
R⁴ Wasserstoff, Halogen oder ein Nitrorest ist;
R⁵ wie in Anspruch 1 definiert ist;
R⁶, A und n wie in Anspruch 1 definiert sind und eines pharmazeutisch annehmbaren Salzes davon.

3. Verfahren nach Anspruch 1 zur Herstellung einer Verbindung der Formel (Ib): 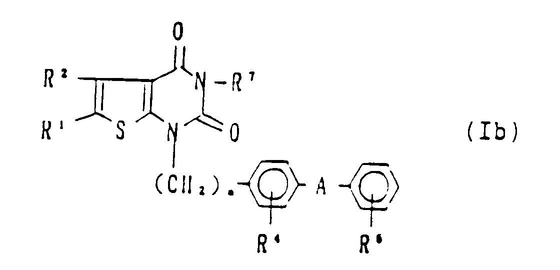 worin R¹ und R² wie in Anspruch 1 definiert sind;
R⁴ Wasserstoff, Halogen oder ein Nitrorest ist;
R⁵ wie in Anspruch 1 definiert ist;
R⁷, A und n wie in Anspruch 1 definiert sind und eines pharmazeutisch annehmbaren Salzes davon.

4. Verfahren nach Anspruch 1, worin ein wie für die Substituenten des Kohlenwasserstoffrestes in Anspruch 1 definierter gegebenenfalls substituierter Aminorest ein Amino-, Methylamino-, Dimethylamino-, Phenylamino-, Benzylamino-, Morpholino-, Piperidino-, Piperazino-, N-Phenylpiperazino- oder N-(m-Methoxy)-phenylpiperazinorest ist.

5. Verfahren nach Anspruch 1, worin D' ein Amino-, Methylamino-, Dimethylamino-, Phenylamino-, Benzylamino-, Morpholino-, Piperidino-, Piperazino- oder N-Phenylpiperazinorest ist.

6. Verfahren nach Anspruch 1, worin D ein Amino-, N-(C₁-C₄)Alkylamino-, N,N-Di(C₁-C₄)alkylamino-, Phenylamino-, Benzylamino-, Naphthylmethylamino-, Pyridylamino-, Pyridylmethylamino-, Morpholino-, Piperidino-, Piperazino-, Piperidylmethyl-, N-Phenylpiperazino- oder N-(p-Fluorphenyl)piperazinorest ist, worin die Alkyl-, Aryl- und Heteroarylgruppen gegebenenfalls mit Methyl-, Ethyl-, Propyl-, Isopropyl-, Butyl-, Isobutyl-, sek.-Butyl-, Hydroxyl-, Amino-, N-Niedrig(C₁-C₄)alkylamino-, N,N-Di-niedrig(C₁-C₄)alkylamino-, Morpholino-, Piperidino-, Piperazino-, N-Phenylpiperazino-, Halogen-, Nitro- oder Niedrig(C₁-C₄)alkoxyresten substituiert sein können.

7. Verfahren nach Anspruch 1, worin R⁵ ein Tetrazolylrest ist.

8. Verfahren nach Anspruch 1, worin R⁵ in ortho-Position ist.

9. Verfahren nach Anspruch 1, zur Herstellung einer Verbindung der Formel (Ia'): 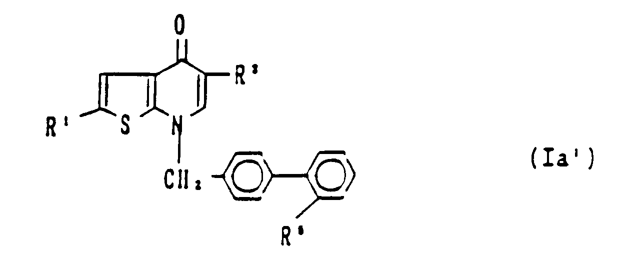 worin R¹ ein (C₁-C₈)-Alkylrest ist;
R³
(1) Wasserstoff,
(2) ein (C₁-C₈)-Alkylrest ist, der geradkettig oder verzweigt sein kann und gegebenenfalls mit Hydroxyl-, Amino-, N-Niedrig(C₁-C₄)alkylamino-, N,N-Diniedrig(C₁-C₄)alkylamino-, Halogen-, (C₁-C₄)-Alkoxyresten oder -COD'' substituiert sein kann, worin D'' ein (C₁-C₄)-Alkoxy-, Hydroxy-, Halogen-, Amino-, N-Niedrig(C₁-C₄)alkylamino-, N,N-Di-niedrig(C₁-C₄)alkylamino-, Phenylamino-, Naphthylamino-, Benzylamino-, Naphthylmethylamino-, Morpholino-, Piperidino-, Piperazino- oder N-Phenylpiperazinorest ist oder
(3) -COD ist, worin D Wasserstoff, ein (C₁-C₄)-Alkoxy-, Hydroxy-, Amino-, N-Niedrig(C₁-C₄)alkylamino-, N,N-Di-niedrig(C₁-C₄)alkylamino-, Phenylamino-, Benzylamino-, Naphthylmethylamino-, Pyridylamino-, Pyridylmethylamino-, Morpholino-, Piperidino-, Piperazino-, Piperidylmethyl-, N-(p-Fluorphenyl)piperazino- oder N-Phenylpiperazinorest ist und R⁵ ein Carboxyl- oder Tetrazolylrest ist oder
eines pharmazeutisch annehmbaren Salzes davon.

10. Verfahren nach Anspruch 1 zur Herstellung einer Verbindung der Formel (Ib'): 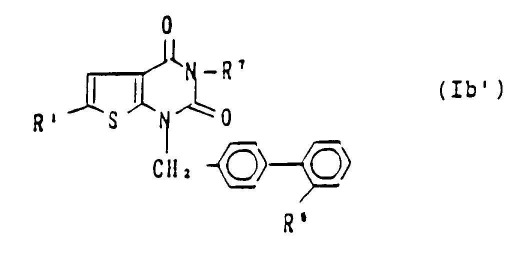 worin R¹ ein (C₁-C₈)-Alkylrest ist; R⁷ ein (C₁-C₈)-Alkylrest ist, der gegebenenfalls mit Phenylresten, die mit Halogen-, Nitro-, (C₁-C₄)-Alkoxy-, (C₁-C₄)-Alkylresten an freigewählter Position am Phenylring substituiert sein können, Amino-, N-Niedrig(C₁-C₄)alkylamino-, N,N-Diniedrig(C₁-C₄)alkylamino-, Phenylamino-, Naphthylamino-, Benzylamino-, Naphthylmethylamino-, Morpholino-, Piperidino-, Piperazino-, N-Phenylpiperazino-, N-(m-Methoxy)phenylpiperazinoresten oder -COD' substituiert sein kann, worin D' ein (C₁-C₄)-Alkoxy-, Hydroxy-, Amino-, N-Niedrig(C₁-C₄)alkylamino-, N,N-Di-niedrig(C₁-C₄)alkylamino-, Phenylamino-, Naphthylamino-, Benzylamino-, Naphthylmethylamino-, Morpholino-, Piperidino-, Piperazino- oder N-Phenylpiperazino-, (C₃-C₈)-Cycloalkyl- oder Phenyl- oder Naphthylrest ist, der mit Hydroxyl-, (C₁-C₄)-Alkoxy-, (C₁-C₄)-Alkyl-, Halogen-, Nitro-, Amino-, N-Niedrig(C₁-C4)alkylamino-, N,N-Diniedrig(C₁-C₄)-alkylamino-, Phenylamino-, Naphthylamino-, Benzylamino-, Naphthylmethylamino-, Morpholino-, Piperidino-, Piperazino-, N-Phenylpiperazino-, N-(m-Methoxy)phenylpiperazino-, (C₁-C₄)-Alkanoyloxy-, Benzoyloxy-, Phenylresten, die mit Halogen-, Nitro-, (C₁-C₄)-Alkoxy-, (C₁-C₄)-Alkylresten an freigewählter Position des Phenylrings substituiert sein können, oder einer Gruppe der Formel -COD' substituiert sein kann, worin D' ein Hydroxy-, (C₁-C₄)-Alkoxy-, Amino-, N-Niedrig(C₁-C₄)-alkylamino-, N,N-Di-niedrig(C₁-C₄)alkylamino-, Phenylamino-, Naphthylamino-, Benzylamino-, Naphthylmethylamino-, Morpholino-, Piperidino-, Piperazino- oder N-Phenylpiperazinorest ist; und R⁵ ein Carboxyl- oder Tetrazolylrest ist oder eines pharmazeutisch annehmbaren Salzes davon.

11. Verfahren nach Anspruch 1 zur Herstellung einer Verbindung, die ausgewählt ist aus der Gruppe bestehend aus Ethyl-2-ethyl-7-[[2'-(1H-tetrazol-5-yl)biphenyl-4-yl]-methyl]-4-oxo-4,7-Dihydrothieno[2,3-b]pyridin-5-carboxylat, 2-Ethyl-7-[[2'-(1H-tetrazol-5-yl)biphenyl-4-yl]-methyl]-4-oxo-4,7-Dihydrothieno[2,3-b]pyridin-5-carbonsäure, Methoxyethyl-2-ethyl-7-[[2'-(1H-tetrazol-5-yl)-biphenyl-4-yl]methyl]-4-oxo-4,7-Dihydrothieno[2,3-b]-pyridin-5-carboxylat, 2-Ethyl-5-(N-benzylcarbamoyl)-7-[[2'-(1H-tetrazol-5-yl)biphenyl-4-yl]methyl]-4-oxo-4,7-Dihydrothieno[2,3-b]pyridin-4(7H)-on, 2-Ethyl-5-(N-phenylcarbamoyl)-7-[[2'-(1H-tetrazol-5-yl)biphenyl-4-yl]methyl]-4-oxo-4,7-Dihydrothieno[2,3-b]pyridin-4(7H)-on und 2-Ethyl-5-(N-2-pyridylmethylcarbamoyl)-7-[[2'-(1H-tetrazol-5-yl)biphenyl-4-yl]methyl]-4-oxo-4,7-Dihy drothieno[2,3-b]pyridin-4(7H)-on, oder eines pharmazeutisch annehmbaren Salzes davon.

12. Verfahren nach Anspruch 10, worin R¹ ein Ethylrest ist, R⁵ ein 1H-Tetrazol-5-yl-rest ist und R⁷ ein Butyl-, Benzyl-, Ethyl-, p-Fluorphenyl-, 2,5-Dichlorphenyl-, Cyclohexyl-, Ethoxycarbonylmethyl- oder 2-[4-(o-Methoxyphenyl)-piperazino]ethylrest ist.

13. Verfahren zur Herstellung einer pharmazeutischen Zusammensetzung, um Angiotensin-II zu antagonisieren, das umfaßt, daß man eine therapeutisch wirksame Menge einer Verbindung nach Anspruch 1 oder ein pharmazeutisch annehmbares Salz davon mit einem pharmazeutisch annehmbaren Träger, Hilfsstoff oder Verdünnungsmittel vermischt.

14. Verwendung einer Verbindung nach Anspruch 1 oder eines pharmazeutisch annehmbaren Salzes davon zur Herstellung eines Arzneimittels, um Angiotensin-II zu antagonisieren.

## Revendications (Revendications pour l'(les) Etat(s) contractant(s) suivant(s): AT, BE, CH, DE, DK, FR, GB, IT, LI, LU, NL, SE)

1. Composé de formule 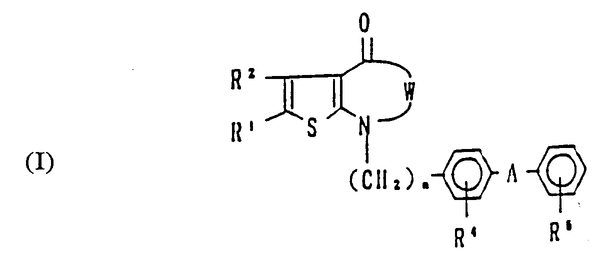 dans laquelle W est un groupe 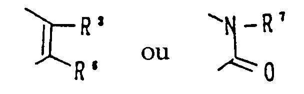 R¹ et R², identiques ou différents, représentent chacun indépendamment (1) un atome d'hydrogène, (2) un atome d'halogène, (3) un groupe cyano, (4) un groupe nitro, (5) un groupe de formule R⁸CO-NH-, où R⁸ représente un atome d'hydrogène ou un groupe alkyle en C₁₋₈ pouvant être substitué par un groupe hydroxyle, alcoxy en C₁₋₄, alkyle en C₁₋₄, un atome d'halogène, un groupe nitro, amino, méthylamino, diméthylamino, phénylamino, benzylamino, morpholino, pipéridino, pipérazino, N-phénylpipérazino, alcanoyloxy en C₁₋₄, benzoyloxy, phényle pouvant porter un substituant halogéno, nitro, alcoxy en C₁₋₄, alkyle en C₁₋₄ en une position choisie du noyau phényle, ou un groupe naphtyle, ou (6) un résidu hydrocarboné choisi dans le groupe formé par (i) un groupe alkyle en C₁₋₈, (ii) alcényle en C₂₋₈, (iii) alcynyle en C₂₋₈, (iv) cycloalkyle en C₃₋₈, (v) un résidu hydrocarboné aromatique choisi dans le groupe formé par les résidus phényle et naphtyle, ledit résidu hydrocarboné pouvant être substitué par un résidu hydroxyle, alcoxy en C₁₋₄, alkyle en C₁₋₄, halogéno, nitro, amino, N-(alkyle en C₁₋₄)-amino, N,N-di(alkyle en C₁₋₄)-amino, phénylamino, naphtylamino, benzylamino, naphtylméthylamino, morpholino, pipéridino, pipérazino, N-phénylpipérazino, N-(m-méthoxy)phénypipérazino, alcanoyloxy en C₁₋₄,benzoyloxy, phényle pouvant porter un substituant halogéno, nitro, alcoxy en C₁₋₄, alkyle en C₁₋₄ en une position choisie du noyau phényle, ou un groupe de formule -COD', où D' représente un groupe hydroxy, alcoxy en C₁₋₄, amino, N-(alkyle en C₁₋₄)-amino, N,N-di(alkyle en C₁₋₄)-amino, phénylamino, naphtylamino, benzylamino, naphtylméthylamino, morpholino, pipéridino, pipérazino ou N-phénylpipérazino;
R³ représente (1) un atome d'hydrogène, (2) un groupe alkyle en C₁₋₈ ou alcényle en C₂₋₈,linéaires ou ramifiés et qui peuvent être subsitués par un groupe hydroxyle, amino, N-(alkyle en C₁₋₄)-amino, N,N-di(alkyle en C₁₋₄)-amino, halogéno, alcoxy en C₁₋₄, ou un groupe de formule -COD'', où D'' représente un groupe alcoxy en C₁₋₄, hydroxy, halogéno, amino, N-(alkyle en C₁₋₄)-amino, N,N-di(alkyle en C₁₋₄)-amino, phénylamino, naphtylamino, benzylamino, naphtylméthylamino, morpholino, pipéridino, pipérazino ou N-phénylpipérazino; ou (3) un groupe -COD où D représente un atome d'hydrogène, un groupe alcoxy en C₁₋₄, hydroxy, halogéno, amino, N-(alkyle en C₁₋₄)-amino, N,N-di(alkyle en C₁₋₄)-amino, phénylamino, benzylamino, naphtylméthylamino, pyridylamino, pyridylméthylamino, morpholino, pipéridino, pipérazino, pipéridylméthyle, N-(p-fluorophényl)pipérazino ou N-phénylpipérazino, lesdits groupes alkyle, aryle et hétéroaryle pouvant être substitué par un groupe méthyle, éthyle, propyle, isopropyle, butyle, isobutyle, sec-butyle, hydroxyle, amino, N-(alkyle en C₁₋₄)-amino, N,N-di(alkyle en C₁₋₄)-amino, morpholino, pipéridino, pipérazino ou N-phénylpipérazino, halogéno, nitro ou alcoxy en C₁₋₄;
R⁴ est un atome d'hydrogène, d'halogène ou un groupe nitro, R⁵ est un groupe carboxyle, (alcoxy en C₁₋₄)-carbonyle, cyano, tétrazolyle, acide trifluorométhanesulfonique, acide phosphorique ou acide sulfonique,
R⁶ représente un atome d'hydrogène ou un groupe alkyle en C₁₋₈ ou alcényle en C₂₋₈ à chaîne linéaire ou ramifiée et pouvant être substitué par un groupe hydroxyle, amino, N-(alkyle en C₁₋₄)-amino, N,N-di(alkyle en C₁₋₄)-amino, halogéno, alcoxy inférieur en C₁₋₄ ou un groupe - COD'' où D'' représente un groupe alcoxy en C₁₋₄, hydroxy, halogéno, N-(alkyle en C₁₋₄)-amino, N,N-di(alkyle en C₁₋₄)-amino, phénylamino, naphtylamino, benzylamino, naphtylméthylamino, morpholino, pipéridino, pipérazino ou N-phénylpipérazino;
R⁷ représente un résidu hydrocarboné pouvant être substitué comme R¹ et R²; A est une liaison directe ou un bras de liaison long de 2 atomes ou moins entre le groupe phénylène et le groupe phényle, choisi dans le groupe formé par les résidus alkylène en C₁₋₄, -C(=O)-,-O-, -S-, -NH-, -C(=O)-NH-, -O-CH₂-, -S-CH₂- ou -CH=CH-,et n est un nombre entier égal a 1 ou 2;
et un sel pharmacologiquement acceptable d'un tel composé.

2. Composé conforme à la revendication 1 qui est un composé de formule (Ia) : 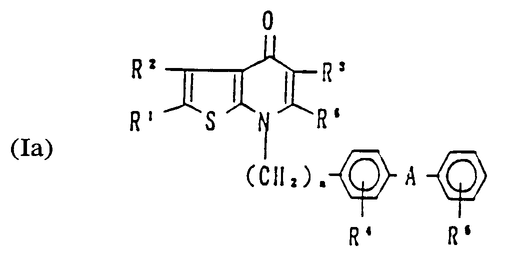 dans laquelle R¹ et R² sont définis comme dans la revendication 1,
R³ représente un atome d'hydrogène, un groupe formyle, un groupe alkyle ou alcényle éventuellement substitués, définis dans la revendication 1, ou un groupe-COD où D représente un groupe alcoxy, hydroxy, halogéno ou amino éventuellement substitué de la manière définie dans la revendication 1;
R⁴ représente un atome d'hydrogène ou d'halogène ou un groupe nitro;
R⁵ est défini comme dans la revendication 1;
R⁶, A et n sont définis comme dans la revendication 1; et un sel pharmacologiquement acceptable d'un tel composé.

3. Composé conforme à la revendication 1 qui est un composé de formule (Ib) 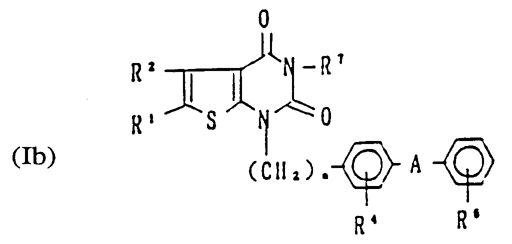 dans laquelle R¹ et R² sont définis comme dans la revendication 1;
R⁴ représente un atome d'hydrogène ou d'halogène ou un groupe nitro; R⁵ est défini comme dans la revendication 1;
R⁷, A et n sont définis comme dans la revendication 1;
et un sel pharmacologiquement acceptable d'un tel composé.

4. Composé conforme à la revendication 1 dans lequel un groupe amino éventuellement substitué défini dans la revendication 1 pour les substituants dudit résidu hydrocarboné est un groupe amino, méthylamino, diméthylamino, phénylamino, benzylamino, morpholino, pipéridino, pipérazino, N-phénylpipérazino ou N-(m-méthoxy)phénylpipérazino.

5. Composé conforme à la revendication 1 dans lequel ledit résidu D' est un résidu amino, méthylamino, diméthylamino, phénylamino, benzylamino, morpholino, pipéridino, pipérazino ou N-phénylpipérazino.

6. Composé conforme à la revendication 1 dans lequel ledit résidu D est un groupe amino, N-(alkyle en C₁₋₄)-amino, N,N-di(alkyle en C₁₋₄)-amino, phénylamino, benzylamino, naphtylméthylamino, pyridylamino, pyridylméthylamino, morpholino, pipéridino, pipérazino, pipéridylméthyle, N-phénylpipérazino ou N-(p-fluorophényl)pipérazino où lesdits groupes alkyle, aryle et hétéroaryle peuvent être substitués par un groupe méthyle, éthyle, propyle, isopropyle, butyle, isobutyle, sec-butyle, hydroxyle, amino, N-(alkyle en C₁₋₄)-amino, N-N-di(alkyle en C₁₋₄)-amino, morpholino, pipéridino, pipérazino, N-phénylpipérazino, halogéno, nitro ou alcoxy en C₁₋₄.

7. Composé conforme à la revendication 1 dans lequel ledit groupe R⁵ est un résidu tétrazolyle.

8. Composé conforme à la revendication 1 dans lequel ledit résidu R⁵ est en position ortho.

9. Composé conforme à la revendication 1 qui est un composé de formule (Ia') : 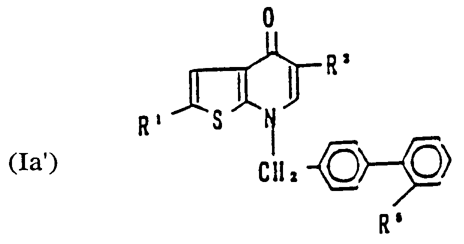 dans laquelle R¹ est un groupe alkyle en C₁₋₈, R³ est (1) un atome d'hydrogène, (2) un groupe alkyle en C₁₋₈, linéaire ou ramifié, pouvant être substitué par un groupe hydroxyle, amino, N-(alkyle en C₁₋₄)-amino, N,N-di(alkyle en C₁₋₄)-amino, halogéno, alcoxy en C₁₋₄, ou un groupe -COD'' où D'' représente un groupe alcoxy en C₁₋₄, hydroxy, halogéno, amino, N-(alkyle en C₁₋₄)-amino, N,N-di(alkyle en C₁₋₄)-amino, phénylamino, naphtylamino, benzylamino, naphtylméthylamino, morpholino, pipéridino, pipérazino ou N-phénylpipérazino; ou (3) un groupe -COD où D représente un atome d'hydrogène, un groupe alcoxy en C₁₋₄, hydroxy, amino, N-(alkyle en C₁₋₄)-amino, N,N-di(alkyle en C₁₋₄)-amino, phénylamino, benzylamino, naphtylméthylamino, pyridylamino, pyridylméthylamino, morpholino, pipéridino, pipérazino, pipéridylméthyle, N-(p-fluorophényl)pipérazino ou N-phénylpipérazino, et
R⁵ est un groupe carboxyle ou tétrazolyle,
ou un sel pharmaceutiquement acceptable d'un tel composé.

10. Composé conforme à la revendication 1 qui est un composé de formule (Ib') : 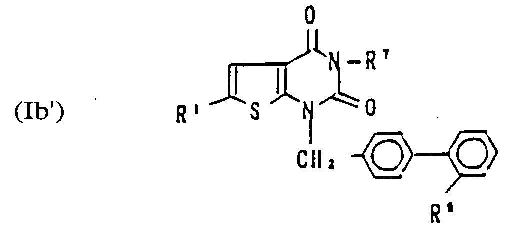 dans laquelle R¹ est un groupe alkyle en C₁₋₈, R⁷ est un groupe alkyle en C₁₋₈ pouvant être substitué par un groupe phényle qui, à son tour peut être substitué par un atome d'halogène, un groupe nitro, alcoxy en C₁₋₄, alkyle en C₁₋₄ en une position choisie du noyau phényle, amino, N-(alkyle en C₁₋₄)-amino, N,N-di(alkyle en C₁₋₄)-amino, phénylamino, naphtylamino, benzylamino, naphtylméthylamino, morpholino, pipéridino, pipérazino, N-(m-méthoxy)phénylpipérazino, ou un groupe -COD' où D' représente un groupe alcoxy en C₁₋₄, hydroxy, amino, N-(alkyle en C₁₋₄)-amino, N,N-di(alkyle en C₁₋₄)-amino, phénylamino, naphtylamino, benzylamino, naphtylméthylamino, morpholino, pipéridino, pipérazino ou N-phénylpipérazino, cycloalkyle en C₃₋₈, ou phényle ou naphtyle pouvant être substitué par un groupe hydroxyle, alcoxy en C₁₋₄, alkyle en C₁₋₄, halogéno, nitro, amino, N-(alkyle en C₁₋₄)-amino, N,N-di(alkyle en C₁₋₄)-amino, phénylamino, naphtylamino, benzylamino, naphtylméthylamino, morpholino, pipéridino, pipérazino ou N-phénylpipérazino, N-(m-méthoxy)phénylpipérazino, alcanoyloxy en C₁₋₄, benzoyloxy, phényle pouvant être substitué par un atome d'halogène, un groupe nitro, alcoxy en C₁₋₄, alkyle en C₁₋₄ en une position choisie du noyau phényle, ou un groupe de formule -COD' où D' représente un groupe alcoxy en C₁₋₄, hydroxy, amino, N-(alkyle en C₁₋₄)-amino, N,N-di(alkyle en C₁₋₄)-amino, phénylamino, naphtylamino, benzylamino, naphtylméthylamino, morpholino, pipéridino, pipérazino ou N-phénylpipérazino;
R⁵ est un groupe carboxyle ou tétrazolyle
ou un sel pharmacologiquement acceptable d'un tel composé.

11. Composé conforme à la revendication 1 ou un sel pharmaceutiquement acceptable d'un tel composé, qui est choisi dans le groupe formé par
le 2-éthyl-7-{[2'-(1H-tétrazol-5-yl)biphényl-4-yl]méthyl]-4-oxo-4,7-dihydrothiéno[2,3-b]pyridine-5-carboxylate d'éthyle,
l'acide 2-éthyl-7-{[2'-(1H-tétrazol-5-yl)biphényl-4-yl]méthyl]-4-oxo-4,7-dihydrothiéno[2,3-b]pyridine-5-carboxylique,
le 2-éthyl-7-{[2'-(1H-tétrazol-5-yl)biphényl-4-yl]méthyl]-4-oxo-4,7-dihydrothiéno[2,3-b]pyridine-5-carboxylate de méthoxyéthyle, la 2-éthyl-5-(N-benzylcarbamoyl)-7-{[2'-(1H-tétrazol-5-yl)biphényl-4-yl]méthyl]-4-oxo-4,7-dihydrothiéno[2,3-b]pyridin-4(7H)-one,
la 2-éthyl-5-(N-phénylcarbamoyl)-7-{[2'-(1H-tétrazol-5-yl)biphényl-4-yl]méthyl]-4-oxo-4,7-dihydrothiéno[2,3-b]pyridine-4(7H)-one, et
la 2-éthyl-5-(N-2-pyridylméthylcarbamoyl)-7-{[2'-(1H-tétrazol-5-yl)biphényl-4-yl]méthyl]-4-oxo-4,7-dihydrothiéno[2,3-b]-pyridin4(7H)-one.

12. Composé conforme à la revendication 10 dans lequel R¹ est un groupe éthyle, R⁵ un groupe 1H-tétrazol-5-yle et R⁷ est un groupe butyle, benzyle, éthyle, p-fluorophényle, 2,5-dichlorophényle, cyclohexyle, éthoxycarbonylméthyle ou 2-[4-(o-méthoxyphényl)-pipérazino]éthyle.

13. Composition pharmaceutique à effet antagoniste de l'angiotensine II comprenant une quantité thérapeutiquement efficace d'un composé conforme à la revendication 1 ou d'un sel pharmaceutiquement acceptable d'un tel composé mélangé avec un véhicule, excipient ou diluant pharmaceutiquement acceptables.

14. Utilisation d'un composé conforme à la revendication 1 ou d'un sel pharmaceutiquement acceptable d'un tel composé pour la fabrication d'un médicament à effet antagoniste de l'angiotensine II.

15. Procédé de préparation d'un composé de formule (I) 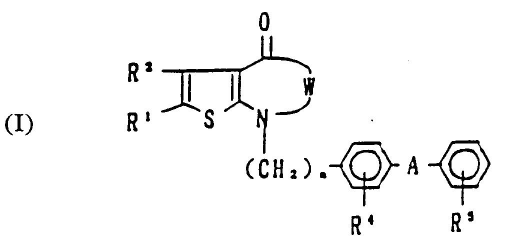 dans laquelle W, R¹, R², R³, R⁴, R⁵, R⁶, R⁷, A et n ont la signification indiquée dans la revendication 1, ou d'un sel pharmaceutiquement acceptable d'un tel composé,
lequel procédé consiste à faire réagir un composé de formule (II) 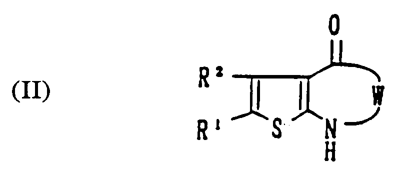 dans laquelle R¹, R² et W ont les significations indiquées ci-dessus, avec un composé de formule (III) 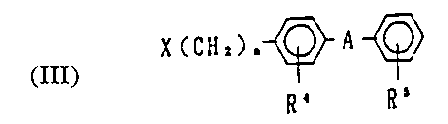 dans laquelle R⁴, R⁵, A et n ont les significations indiquées ci-dessus, et X est un atome d'halogène, et, (i) si on le souhaite, à convenir un composé de formule (I) où R⁵ est un groupe cyano ou un groupe tétrazolyle protégé, et R¹, R², R⁴, R⁵, A, W et n ont la signification indiquée ci-dessus, en un composé de formule (I) où R⁵ représente un groupe tétrazolyle et R¹, R², R⁴, R⁵, A, W et n ont la signification indiquée ci-dessus, (ii) si on le souhaite, à convertir un composé de formule (I) où -R³ est un groupe alcoxycarbonyle en C₁₋₄ ou halogénocarbonyle, et R¹, R², R⁴, R⁵, R⁶, A et n ont la signification indiquée ci-dessus, en un composé de formule (I) où -R³ est un groupe carboxyle ou carbamoyle éventuellement substitué, et R¹, R², R³, R⁴, R⁵, R⁶, R⁷, A et n ont la signification ci-dessus, ou (iii) si on le souhaite, à convertir d'un composé de formule (I) où R³ est un groupe carboxyle et R¹, R², R⁴, R⁵, R⁶, A et n ont la signification indiquée ci-dessus, en un composé de formule (I) dans laquelle R³ est un résidu halogénocarbonyle et R¹, R², R⁴, R⁵, R⁶, A et n ont la signification indiquée ci-dessus, et si on le souhaite, à convertir un composé de formule (I) en un sel pharmaceutiquement acceptable.

## Revendications (Revendications pour l'(les) Etat(s) contractant(s) suivant(s): ES, GR)

1. Procédé de préparation d'un composé de formule 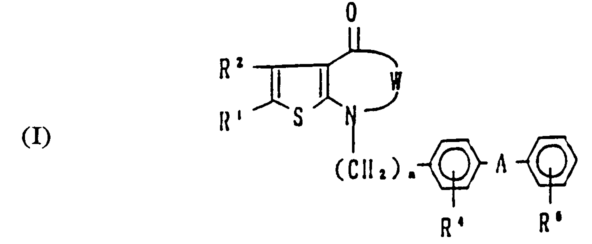 dans laquelle W est un groupe 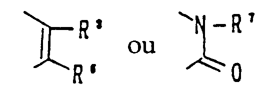 R¹ et R², identiques ou différents, représentent chacun indépendamment (1) un atome d'hydrogène, (2) un atome d'halogène, (3) un groupe cyano, (4) un groupe nitro, (5) un groupe de formule R⁸CO-NH-, où R⁸ représente un atome d'hydrogène ou un groupe alkyle en C₁₋₈ pouvant être substitué par un groupe hydroxyle, alcoxy en C₁₋₄, alkyle en C₁₋₄, un atome d'halogène, un groupe nitro, amino, méthylamino, diméthylamino, phénylamino, benzylamino, morpholino, pipéridino, pipérazino, N-phénylpipérazino, alcanoyloxy en C₁₋₄, benzoyloxy, phényle pouvant porter un substituant halogéno, nitro, alcoxy en C₁₋₄, alkyle en C₁₋₄ en une position choisie du noyau phényle, ou un groupe naphtyle, ou (6) un résidu hydrocarboné choisi dans le groupe formé par (i) un groupe alkyle en C₁₋₈, (ii) alcényle en C₂₋₈, (iii) alcynyle en C₂₋₈, (iv) cycloalkyle en C₃₋₈, (v) un résidu hydrocarboné aromatique choisi dans le groupe formé par les résidus phényle et naphtyle, ledit résidu hydrocarboné pouvant être substitué par un résidu hydroxyle, alcoxy en C₁₋₄, alkyle en C₁₋₄, halogéno, nitro, amino, N-(alkyle en C₁₋₄)-amino, N,N-di(alkyle en C₁₋₄)-amino, phénylamino, naphtylamino, benzylamino, naphtylméthylamino, morpholino, pipéridino, pipérazino, N-phénylpipérazino, N-(m-méthoxy)phénypipérazino, alcanoyloxy en C₁₋₄, benzoyloxy, phényle pouvant porter un substituant halogéno, nitro, alcoxy en C₁₋₄, alkyle en C₁₋₄ en une position choisie du noyau phényle, ou un groupe de formule -COD', où D' représente un groupe hydroxy, alcoxy en C₁₋₄, amino, N-(alkyle en C₁₋₄)-amino, N,N-di(alkyle en C₁₋₄)-amino, phénylamino, naphtylamino, benzylamino, naphtylméthylamino, morpholino, pipéridino, pipérazino ou N-phénylpipérazino;
R³ représente (1) un atome d'hydrogène, (2) un groupe alkyle en C₁₋₈ ou alcényle en C₂₋₈, linéaires ou ramifiés et qui peuvent être substitués par un groupe hydroxyle, amino, N-(alkyle en C₁₋₄)-amino, N,N-di(alkyle en C₁₋₄)-amino, halogéno, alcoxy en C₁₋₄, ou un groupe de formule -COD'', où D'' représente un groupe alcoxy en C₁₋₄, hydroxy, halogéno, amino, N-(alkyle en C₁₋₄)-amino, N,N-di(alkyle en C₁₋₄)-amino, phénylamino, naphtylamino, benzylamino, naphtylméthylamino, morpholino, pipéridino, pipérazino ou N-phénylpipérazino; ou (3) un groupe -COD où D représente un atome d'hydrogène, un groupe alcoxy en C₁₋₄, hydroxy, halogéno, amino, N-(alkyle en C₁₋₄)-amino, N,N-di(alkyle en C₁₋₄)-amino, phénylamino, benzylamino, naphtylméthylamino, pyridylamino, pyridylméthylamino, morpholino, pipéridino, pipérazino, pipéridylméthyle, N-(p-fluorophényl)pipérazino ou N-phénylpipérazino, lesdits groupes alkyle, aryle et hétéroaryle pouvant être substitué par un groupe méthyle, éthyle, propyle, isopropyle, butyle, isobutyle, sec-butyle, hydroxyle, amino, N-(alkyle en C₁₋₄)-amino, N,N-di(alkyle en C₁₋₄)-amino, morpholino, pipéridino, pipérazino ou N-phénylpipérazino, halogéno, nitro ou alcoxy en C₁₋₄;
R⁴ est un atome d'hydrogène, d'halogène ou un groupe nitro,
R⁵ est un groupe carboxyle, (alcoxy en C₁₋₄)-carbonyle, cyano, tétrazolyle, acide trifluorométhanesulfonique, acide phosphorique ou acide sulfonique,
R⁶ représente un atome d'hydrogène ou un groupe alkyle en C₁₋₈ ou alcényle en C₂₋₈ à chaîne linéaire ou ramifiée et pouvant être substitué par un groupe hydroxyle, amino, N-(alkyle en C₁₋₄)-amino, N,N-di(alkyle en C₁₋₄)-amino, halogéno, alcoxy inférieur en C₁₋₄ ou un groupe - COD'' où D'' représente un groupe alcoxy en C₁₋₄, hydroxy, halogéno, N-(alkyle en C₁₋₄)-amino, N,N-di(alkyle en C₁₋₄)amino, phénylamino, naphtylamino, benzylamino, naphtylméthylamino, morpholino, pipéridino, pipérazino ou N-phénylpipérazino;
R⁷ représente un résidu hydrocarboné pouvant être substitué comme R¹ et R²; A est une liaison directe ou un bras de liaison long de 2 atomes ou moins entre le groupe phénylène et le groupe phényle, choisi dans le groupe formé par les résidus alkylène en C₁₋₄, -C(=O)-, -O-, -S-, -NH-, -C(=O)-NH-, -O-CH₂-, -S-CH₂- ou -CH=CH-, et n est un nombre entier égal a 1 ou 2;
et un sel pharmacologiquement acceptable d'un tel composé,
lequel procédé consiste à faire réagir un composé de formule (II) 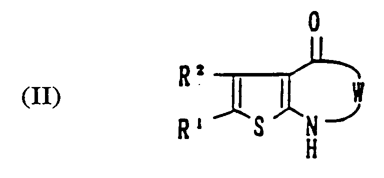 dans laquelle R¹, R² et W ont les significations indiquées ci-dessus, avec un composé de formule (III) 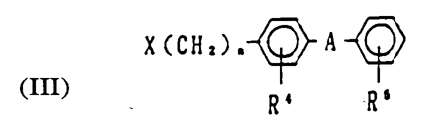 dans laquelle R⁴, R⁵, A et n ont les significations indiquées ci-dessus, et X est un atome d'halogène, et, (i) si on le souhaite, à convertir un composé de formule (I) où R⁵ est un groupe cyano ou un groupe tétrazolyle protégé, et R¹, R², R⁴, R⁵, A, W et n ont la signification indiquée ci-dessus, en un composé de formule (I) où R⁵ représente un groupe tétrazolyle et R¹, R², R⁴, R⁵, A, W et n ont la signification indiquée ci-dessus, (ii) si on le souhaite, à convertir un composé de formule (I) où -R³ est un groupe alcoxycarbonyle en C₁₋₄ ou halogénocarbonyle, et R¹, R², R⁴, R⁵, R⁶, A et n ont la signification indiquée ci-dessus, en un composé de formule (I) où -R³ est un groupe carboxyle ou carbamoyle éventuellement substitué, et R¹, R², R³, R⁴, R⁵, R⁶, R⁷, A et n ont la signification ci-dessus, ou (iii) si on le souhaite, à convertir d'un composé de formule (I) où R³ est un groupe carboxyle et R¹, R², R⁴, R⁵, R⁶, A et n ont la signification indiquée ci-dessus, en un composé de formule (I) dans laquelle R³ est un résidu halogénocarbonyle et R¹, R², R⁴, R⁵, R⁶, A et n ont la signification indiquée ci-dessus, et si on le souhaite, à convertir un composé de formule (I) en un sel pharmaceutiquement acceptable.

2. Procédé conforme à la revendication 1 pour la préparation d'un composé de formule (Ia) : 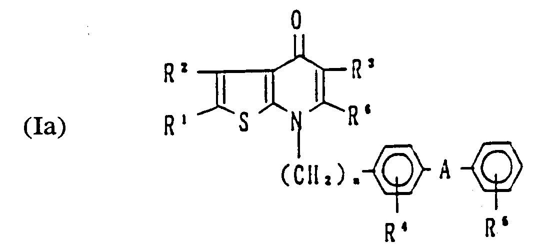 dans laquelle R¹ et R² sont définis comme dans la revendication 1,
R³ représente un atome d'hydrogène, un groupe formyle, un groupe alkyle ou alcényle éventuellement substitués, définis dans la revendication 1, ou un groupe-COD où D représente un groupe alcoxy, hydroxy, halogéno ou amino éventuellement substitué de la manière définie dans la revendication 1;
R⁴ représente un atome d'hydrogène ou d'halogène ou un groupe nitro;
R⁵ est défini comme dans la revendication 1;
R⁶, A et n sont définis comme dans la revendication 1;
et d'un sel pharmacologiquement acceptable d'un tel composé.

3. Procédé conforme à la revendication 1 pour la préparation d'un composé de formule (Ib) 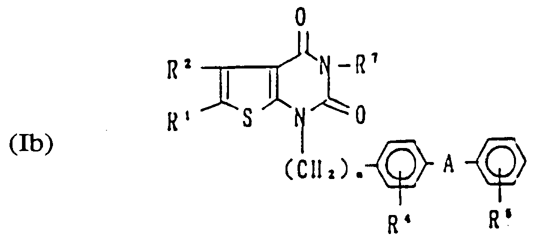 dans laquelle R¹ et R² sont définis comme dans la revendication 1; R⁴ représente un atome d'hydrogène ou d'halogène ou un groupe nitro; R⁵ est défini comme dans la revendication 1;
R⁷, A et n sont définis comme dans la revendication 1;
et d'un sel pharmacologiquement acceptable d'un tel composé.

4. Procédé conforme à la revendication 1 dans lequel un groupe amino éventuellement substitué défini dans la revendication 1 pour les substituants dudit résidu hydrocarboné est un groupe amino, méthylamino, diméthylamino, phénylamino, benzylamino, morpholino, pipéridino, pipérazino, N-phénylpipérazino ou N-(m-méthoxy)phénylpipérazino.

5. Procédé conforme à la revendication 1 dans lequel ledit résidu D' est un résidu amino, méthylamino, diméthylamino, phénylamino, benzylamino, morpholino, pipéridino, pipérazino ou N-phénylpipérazino.

6. Procédé conforme à la revendication 1 dans lequel ledit résidu D est un groupe amino, N-(alkyle en C₁₋₄)-amino, N,N-di(alkyle en C₁₋₄)-amino, phénylamino, benzylamino, naphtylméthylamino, pyridylamino, pyridylméthylamino, morpholino, pipéridino, pipérazino, pipéridylméthyle, N-phénylpipérazino ou N-(p-fluorophényl)pipérazino où lesdits groupes alkyle, aryle et hétéroaryle peuvent être substitués par un groupe méthyle, éthyle, propyle, isopropyle, butyle, isobutyle, sec-butyle, hydroxyle, amino, N-(alkyle en C₁₋₄)-amino, N-N-di(alkyle en C₁₋₄)-amino, morpholino, pipéridino, pipérazino, N-phénylpipérazino, halogéno, nitro ou alcoxy en C₁₋₄.

7. Procédé conforme à la revendication 1 dans lequel ledit groupe R⁵ est un résidu tétrazolyle.

8. Procédé conforme à la revendication 1 dans lequel ledit résidu R⁵ est en position ortho.

9. Procédé conforme à la revendication 1 pour la préparation d'un composé de formule (Ia') : 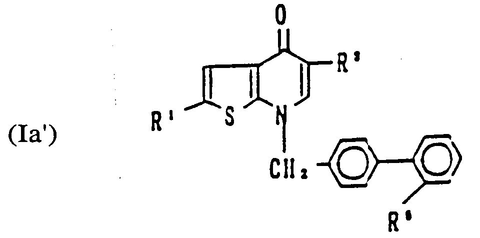 dans laquelle R¹ est un groupe alkyle en C₁₋₈,
R³ est (1) un atome d'hydrogène, (2) un groupe alkyle en C₁₋₈, linéaire ou ramifié, pouvant être substitué par un groupe hydroxyle, N-(alkyle en C₁₋₄)-amino, N,N-di(alkyle en C₁₋₄)-amino, halogéno, alcoxy en C₁₋₄, ou un groupe -COD'' où D'' représente un groupe alcoxy en C₁₋₄, hydroxy, halogéno, amino, N-(alkyle en C₁₋₄)-amino, N,N-di(alkyle en C₁₋₄)-amino, phénylamino, naphtylamino, benzylamino, naphtylméthylamino, morpholino, pipéridino, pipérazino ou N-phénylpipérazino; ou
(3) un groupe -COD où D représente un atome d'hydrogène, un groupe alcoxy en C₁₋₄, hydroxy, amino, N-(alkyle en C₁₋₄)-amino, N,N-di(alkyle en C₁₋₄)-amino, phénylamino, benzylamino, naphtylméthylamino, pyridylamino, pyridylméthylamino, morpholino, pipéridino, pipérazino, pipéridylméthyle, N-(p-fluorophényl)pipérazino ou N-phénylpipérazino, et
R⁵ est un groupe carboxyle ou tétrazolyle, ou d'un sel pharmaceutiquement acceptable d'un tel composé.

10. Procédé conforme à la revendication 1 pour la préparation d'un composé de formule (Ib') : 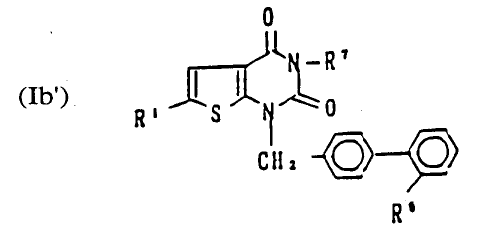 dans laquelle R¹ est un groupe alkyle en C₁₋₈, R⁷ est un groupe alkyle en C₁₋₈ pouvant être substitué par un groupe phényle qui, à son tour peut être substitué par un atome d'halogène, un groupe nitro, alcoxy en C₁₋₄, alkyle en C₁₋₄ en une position choisie du noyau phényle, amino, N-(alkyle en C₁₋₄)-amino, N,N-di(alkyle en C₁₋₄)amino, phénylamino, naphtylamino, benzylamino, naphtylméthylamino, morpholino, pipéridino, pipérazino, N(m-méthoxy)phénylpipérazino, ou un groupe -COD' où D' représente un groupe alcoxy en C₁₋₄, hydroxy, amino, N-(alkyle en C₁₋₄)-amino, N,N-di(alkyle en C₁₋₄)-amino, phénylamino, naphtylamino, benzylamino, naphtylméthylamino, morpholino, pipéridino, pipérazino ou N-phénylpipérazino, cycloalkyle en C₃₋₈, ou phényle ou naphtyle pouvant être substitué par un groupe hydroxyle, alcoxy en C₁₋₄, alkyle en C₁₋₄, halogéno, nitro, amino, N-(alkyle en C₁₋₄)-amino, N,N-di(alkyle en C₁₋₄)-amino, phénylamino, naphtylamino, benzylamino, naphtylméthylamino, morpholino, pipéridino, pipérazino ou N-phénylpipérazino, N-(m-méthoxy)phénylpipérazino, alcanoyloxy en C₁₋₄, benzoyloxy, phényle pouvant être substitué par un atome d'halogène, un groupe nitro, alcoxy en C₁₋₄, alkyle en C₁₋₄ en une position choisie du noyau phényle, ou un groupe de formule -COD' où D' représente un groupe alcoxy en C₁₋₄, hydroxy, amino, N-(alkyle en C₁₋₄)-amino, N,N-di(alkyle en C₁₋₄)-amino, phénylamino, naphtylamino, benzylamino, naphtylméthylamino, morpholino, pipéridino, pipérazino ou N-phénylpipérazino;
R⁵ est un groupe carboxyle ou tétrazolyle
ou d'un sel pharmacologiquement acceptable d'un tel composé.

11. Procédé conforme à la revendication 1 pour la préparation d'un composé choisi dans le groupe formé par
le 2-éthyl-7-{[2'-(1H-tétrazol-5-yl)biphényl-4-yl]méthyl]-4-oxo-4,7-dihydrothiéno[2,3-b]pyridine-5-carboxylate d'éthyle,
l'acide 2-éthyl-7-{[2'-(1H-tétrazol-5-yl)biphényl-4-yl]méthyl]-4-oxo-4,7-dihydrothiéno[2,3-b]pyridine-5-carboxylique,
le 2-éthyl-7-{[2'-(1H-tétrazol-5-yl)biphényl-4-yl]méthyl]-4-oxo-4,7-dihydrothiéno[2,3-b]pyridine-5-carboxylate de méthoxyéthyle,
la 2-éthyl-5-(N-benzylcarbamoyl)-7-{[2'-(1H-tétrazol-5-yl)biphényl-4-yl]méthyl]-4-oxo-4,7-dihydrothiéno[2,3-b]pyridin-4(7H)-one,
la 2-éthyl-5-(N-phénylcarbamoyl)-7-{[2'-(1H-tétrazol-5-yl)biphényl-4-yl]méthyl]-4-oxo-4,7-dihydrothiéno[2,3-b]pyridine-4(7H)-one, et
la 2-éthyl-5-(N-2-pyridylméthylcarbamoyl)-7-{[2'-(1H-tétrazol-5-yl)biphényl-4-yl]méthyl]-4-oxo-4,7-dihydrothiéno[2,3-b]-pyridin4(7H)-one.

12. Procédé conforme à la revendication 10 dans lequel R¹ est un groupe éthyle, R⁵ un groupe 1H-tétrazol-5-yle et R⁷ est un groupe butyle, benzyle, éthyle, p-fluorophényle, 2,5-dichlorophényle, cyclohexyle, éthoxycarbonylméthyle ou 2-[4-(o-méthoxyphényl)-pipérazino]éthyle.

13. Procédé de préparation d'une composition pharmaceutique à effet antagoniste de l'angiotensine II comprenant la mélangeage d'une quantité thérapeutiquement efficace d'un composé conforme à la revendication 1 ou d'un sel pharmaceutiquement acceptable d'un tel composé avec un véhicule, excipient ou diluant pharmaceutiquement acceptables.

14. Utilisation d'un composé conforme à la revendication 1 ou d'un sel pharmaceutiquement acceptable d'un tel composé pour la fabrication d'un médicament à effet antagoniste de l'angiotensine II.
